# EUROPEAN PATENT SPECIFICATION

(11) **EP 1 740 201 B1**
(45) Date of publication and mention of the grant of the patent: **05.11.2008**
(21) Application number: 05734803.9
(22) Date of filing: 09.03.2005
(51) Int. Cl.: A61K 38/21, A61P 43/00

(54) **PHARMACEUTICAL COMPOSITIONS COMPRISING INTERFERON-TAU**
PHARMAZEUTISCHE ZUSAMMENSETZUNGEN MIT INTERFERON-TAU
PROCEDES DE TRAITEMENT AU MOYEN D'INTERFERON TAU

(30) Priority: 10.03.2004 US 552279 P; 14.04.2004 US 824710; 14.04.2004 US 825068; 14.04.2004 US 825382; 14.04.2004 US 825457; 02.07.2004 US 884741; 21.01.2005 US 40706
(43) Date of publication of application: 10.01.2007
(73) Proprietor: Pepgen Corporation, Alameda, CA 94502 (US)
(72) Inventor: LIU, Chih-Ping, San Francisco, CA 94109 (US); VILLARETE, Lorelie, H., Alameda, CA 94501 (US)
(74) Representative: Vossius & Partner
(86) International application number: PCT/US2005/007887
(87) International publication number: WO 2005/087254

(56) References cited:
- WO-A-02/06343
- WO-A-97/33607
- WO-A-03/061728

### SEQUENCE LISTING

<110> Pepgen Corporation
   Liu, Chih-Ping
   Villarete, Lorelie H.
<120> Methods of Treatment Using Interferon-Tau
<130> 556008014WO0
<140> Not Yet Assigned
   <141> Filed Herewith
<150> US 60/552,279
   <151> 2004-03-10
<150> US 10/824,710
   <151> 2004-04-14
<150> US 10/825,068
   <151> 2004-04-14
<150> US 10/825,382
   <151> 2004-04-14
<150> US 10/825,457
   <151> 2004-04-14
<150> US 10/884,741
   <151> 2004-07-02
<150> US 11/040,706
   <151> 2005-01-21
<160> 6
<170> PatentIn version 3.3
<210> 1
   <211> 516
   <212> DNA
   <213> Ovis aries
<400> 1
   tgctacctgt cgcgaaaact gatgctggac gctcgagaaa atttaaaact gctggaccgt     60
   atgaatcgat tgtctccgca cagctgcctg caagaccgga aagacttcgg tctgccgcag    120
   gaaatggttg aaggtgacca actgcaaaaa gaccaagctt tcccggtact gtatgaaatg    180
   ctgcagcagt ctttcaacct gttctacact gaacattctt cggccgcttg ggacactact    240
   cttctagaac aactgtgcac tggtctgcaa cagcaactgg accatctgga cacttgccgt    300
   ggccaggtta tgggtgaaga agactctgaa ctgggtaaca tggatccgat cgttactgtt    360
   aaaaaatatt tccagggtat ctacgactac ctgcaggaaa aaggttactc tgactgcgct    420
   tgggaaatcg tacgcgttga aatgatgcgg gccctgactg tgtcgactac tctgcaaaaa    480
   cggttaacta aaatgggtgg tgacctgaat tctccg                              516
<210> 2
   <211> 172
   <212> PRT
   <213> Ovis aries
<400> 2
   Cys Tyr Leu Ser Arg Lys Leu Met Leu Asp Ala Arg Glu Asn Leu Lys
   1               5                   10                  15
   Leu Leu Asp Arg Met Asn Arg Leu Ser Pro His Ser Cys Leu Gln Asp
               20                  25                  30
   Arg Lys Asp Phe Gly Leu Pro Gln Glu Met Val Glu Gly Asp Gln Leu
           35                  40                  45
   Gln Lys Asp Gln Ala Phe Pro Val Leu Tyr Glu Met Leu Gln Gln Ser
       50                  55                  60
   Phe Asn Leu Phe Tyr Thr Glu His Ser Ser Ala Ala Trp Asp Thr Thr
   65                  70                  75                  80
   Leu Leu Glu Gln Leu Cys Thr Gly Leu Gln Gln Gln Leu Asp His Leu
                   85                  90                  95
   Asp Thr Cys Arg Gly Gln Val Met Gly Glu Glu Asp Ser Glu Leu Gly
               100                 105                 110
   Asn Met Asp Pro Ile Val Thr Val Lys Lys Tyr Phe Gln Gly Ile Tyr
           115                 120                 125
   Asp Tyr Leu Gln Glu Lys Gly Tyr Ser Asp Cys Ala Trp Glu Ile Val
       130                 135                 140
   Arg Val Glu Met Met Arg Ala Leu Thr Val Ser Thr Thr Leu Gln Lys
   145                 150                 155                 160
   Arg Leu Thr Lys Met Gly Gly Asp Leu Asn Ser Pro
                   165                 170
<210> 3
   <211> 172
   <212> PRT
   <213> Artificial
<220>
   <223> Recombinant IFN-Tau Based on Ovis aries Sequence
<400> 3
   Cys Tyr Leu Ser Glu Arg Leu Met Leu Asp Ala Arg Glu Asn Leu Lys
   1               5                   10                  15
   Leu Leu Asp Arg Met Asn Arg Leu Ser Pro His Ser Cys Leu Gln Asp
               20                  25                  30
   Arg Lys Asp Phe Gly Leu Pro Gln Glu Met Val Glu Gly Asp Gln Leu
           35                  40                  45
   Gln Lys Asp Gln Ala Phe Pro Val Leu Tyr Glu Met Leu Gln Gln Ser
       50                  55                  60
   Phe Asn Leu Phe Tyr Thr Glu His Ser Ser Ala Ala Trp Asp Thr Thr
   65                  70                  75                  80
   Leu Leu Glu Gln Leu Cys Thr Gly Leu Gln Gln Gln Leu Asp His Leu
                   85                  90                  95
   Asp Thr Cys Arg Gly Gln Val Met Gly Glu Glu Asp Ser Glu Leu Gly
               100                 105                 110
   Asn Met Asp Pro Ile Val Thr Val Lys Lys Tyr Phe Gln Gly Ile Tyr
           115                 120                 125
   Asp Tyr Leu Gln Glu Lys Gly Tyr Ser Asp Cys Ala Trp Glu Ile Val
       130                 135                 140
   Arg Val Glu Met Met Arg Ala Leu Thr Val Ser Thr Thr Leu Gln Lys
   145                 150                 155                 160
   Arg Leu Thr Lys Met Gly Gly Asp Leu Asn Ser Pro
                   165                 170
<210> 4
   <211> 516
   <212> DNA
   <213> Artificial
<220>
   <223> Recombinant IFN-Tau Based on Ovis aries Sequence
<400> 4
   tgctacctgt cggagcgact gatgctggac gctcgagaaa atttaaaact gctggaccgt     60
   atgaatcgat tgtctccgca cagctgcctg caagaccgga aagacttcgg tctgccgcag    120
   gaaatggttg aaggtgacca actgcaaaaa gaccaagctt tcccggtact gtatgaaatg    180
   ctgcagcagt ctttcaacct gttctacact gaacattctt cggccgcttg ggacactact    240
   cttctagaac aactgtgcac tggtctgcaa cagcaactgg accatctgga cacttgccgt    300
   ggccaagtta tgggtgaaga agactctgaa ctgggtaaca tggatccgat cgttactgtt    360
   aaaaaatatt tccagggtat ctacgactac ctgcaggaaa aaggttactc tgactgcgct    420
   tgggaaatcg tacgcgttga aatgatgcgg gccctgactg tgtcgactac tctgcaaaaa    480
   cggttaacta aaatgggtgg tgacctgaat tctccg                              516
<210> 5
   <211> 295
   <212> PRT
   <213> Homo sapiens
<400> 5
<210> 6
   <211> 186
   <212> PRT
   <213> Homo sapiens
<400> 6

## Description

The present invention relates to pharmaceutical compositions containing interferon-tau (IFNτ) and uses thereof. More particularly, the invention relates to compositions for treating diseases or conditions responsive to interleukin-10 (IL-10) therapy in a mammal by administering IFNτ, either alone or in combination with one or more therapeutic agents. The invention also relates to the use of the compositions for modulating blood levels of IL-10, and/or interleukin-12 (IL-12), by stimulating production of IL-10 and/or effecting a decrease in IL-12 production. The invention also relates to the use of the compositions for preventing an increase in the blood level of interferon-gamma (IFN-γ). The invention also relates to combined treatment therapies using interferon-tau and one or more additional agents.

Interferon-tau (hereinafter "IFNτ" or "interferon-τ") was discovered originally as a pregnancy recognition hormone produced by the trophectoderm of ruminant conceptuses (Imakawa, K et al, Nature, 330:377-379, (1987); Bazer, F.W. and Johnson, H.M., Am. J. Repro. Immunol., 26:19-22, (1991)). The distribution of the IFNτ gene is restricted to ruminants, including cattle, sheep, and goats, (Alexenko, A.P. et al., J. Interferon and Cytokine Res., 19: 1335-1341, (1999)) but has been shown to have activity in cells belonging to other species including humans and mice (Pontzer, C.H. et al., Cancer Res., 51:5304-5307, (1991); Alexenko, A.P. et al., J. Interferon and Cytokine Res., 20:817-822, (2000)). For example, IFNτ has been demonstrated to possess antiviral, (Pontzer, C.H. et al., Biochem. Biophys. Res. Commun., 152:801-807, (1988)), antiproliferative, (Pontzer, C.H. et al., Cancer Res., 51:5304-5307, (1991)) and immunoregulatory activities (Assal-Meliani, A., Am. J. Repro. Immunol., 33:267-275 (1995)).

While IFNτ displays many of the activities classically associated with type I interferons, such as interferon-α and interferon-β, considerable differences exist between IFNτ and the other type I interferons. The most prominent difference is the role of IFNτ in pregnancy in ruminant species. The other interferons have no similar activity in pregnancy recognition. Also different is viral induction. All type I interferons, except IFNτ, are induced readily by virus and dsRNA (Roberts, et al., Endocrine Reviews, 13:432 (1992)). Induced IFN-α and IFN-β expression is transient, lasting approximately a few hours. In contrast, IFNτ synthesis, once induced, is maintained over a period of days (Godkin, et al., J. Reprod. Fert., 65:141 (1982)). On a per-cell basis, 300-fold more IFN-τ is produced than other type I interferons (Cross, J.C. et al., Proc. Natl. Acad. Sci. USA 88:3817-3821 (1991)).

Another difference lies in the amino acid sequences of IFNτ and other type I interferons. The percent amino acid sequence similarity between the interferons α_{2b}, β₁, ω₁, γ, and τ are summarized in the table below.

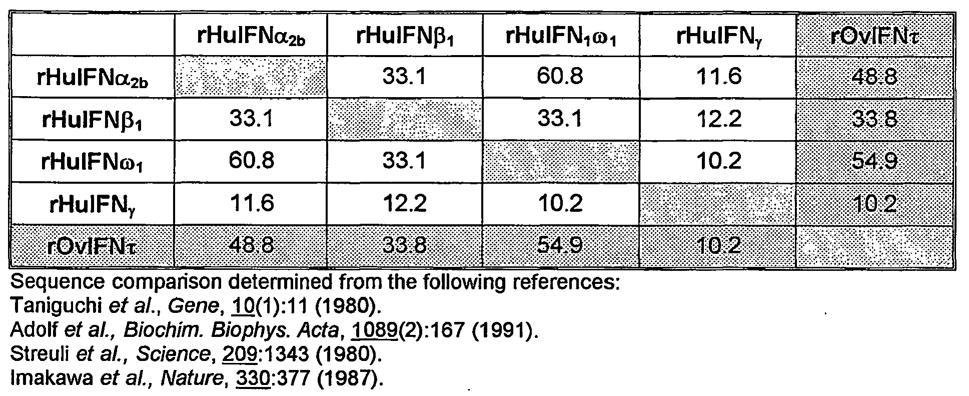

Recombinant ovine IFNτ is 48.8 percent homologous to IFNα_{2b} and 33.8 percent homologous to IFNβ₁. Because of this limited homology between IFNτ and IFNα and between IFNτ and IFNβ, it cannot be predicted whether or not IFNτ would behave in the same manner as IFNα or IFNβ when administered to a human subject. IFNτ is also reported to have a low receptor binding affinity for type I receptors on human cells (Brod, S., J. Interferon and Cytokine Res., 18:841 (1999); Alexenko, A. et al., J. Interferon and Cytokine Res., 17:769 (1997)). Additionally, the fact that IFNτ is a non-endogeneous human protein generates the potential for systemic neutralizing antibody formation when IFNτ is injected into the human body (Brod, S., J. Interferon and Cytokine Res., 18:841 (1999)). These differences between IFNτ and the other interferons make it difficult to predict whether IFNτ will provide a therapeutic benefit when administered to a human.

One limiting factor in the use of IFNτ, as well as proteins and polypeptides in general, is related to biodistribution, as affected by protein interaction with plasma proteins and blood cells, when given parenterally: The oral route of administration is even more problematic due to proteolysis in the stomach, where the acidic conditions can destroy the molecule before reaching its intended target. For example, polypeptides and protein fragments, produced by action of gastric and pancreatic enzymes, are cleaved by exo- and endopeptidases in the intestinal brush border membrane to yield di- and tri-peptides. If proteolysis by pancreatic enzymes is avoided, polypeptides are subject to degradation by brush border peptidases. Polypeptides or proteins that might survive passage through the stomach are subject to metabolism in the intestinal mucosa where a penetration barrier prevents entry into cells. For this reason, effort has focused primarily on delivering proteins to the oral-pharyngeal region in the form of a lozenge or solution held in the oral cavity for a period of time.

Cytokines are secreted regulatory proteins that control the survival, growth, differentiation and effector function of immune cells. Cytokines encompass those families of regulators variously known as growth factors, colony-stimulating factors, interleukins, lymphokines, monokines, and interferons. They are molecules found in the extracellular medium that interact with specific target cells to communicate information regarding the status of the animal and they result in an appropriate biological response in the target tissue.

Cytokines are produced by various cells, including T helper (Th) cells. Th cells can be divided into two functional subsets, Th1 and Th2, which can be distinguished by the patterns of cytokines they secrete. Th1 cells secrete interleukin-2, interferon-gamma, TNF alpha and beta, and lymphotoxin, among others, and are responsible for the development of the cell-mediated immune response. This response is critical for the successful removal of intracellular pathogens, such as certain bacteria and viruses. Th2 cells secrete IL-4, IL-5, IL-10, and IL-13 and are responsible for the development of high levels of IgG1, IgA, and IgE production by B cells, and for the activation of effector cells such as eosinophils.

There often exists a relatively large population of activated T cells that represent uncommitted precursors which can become either Th1 or Th2 cells. The commitment to a Th1 or Th2 phenotype appears to occur shortly after antigenic stimulation, and is characterized by changes in the expression of particular cell surface markers and cell signaling components. Cytokines appear to play a role in Th1 and Th2 differentiation, where the cytokine milieu present can influence Th1 or Th2 development. Accordingly, methods for modulating the Th1 or Th2 cell populations, and in particular for decreasing Th1 cells to decrease Th1 produced cytokines and increasing Th2 cells to increase Th2 produced cytokines are sought.

Such a method for generating a polarized or biased Th2 response has significance since the presence of Th2 cell produced cytokines have been shown to correlate with disease onset and severity. For example, it has been shown or suggested that the following diseases may be benefited by IL-10 therapy or otherwise show some linkage between IL-10 and the particular disease or condition: liver fibrosis (Nelson, D.R. et al. Hepatology 38(4):859-868 (2003); Louis, H., Acta Gastr. Belg. 66(1):7-14 (2003)); pulmonary fibrosis (Aral, T., et al., Am. J. Physiol. Lung. Cell. Mol. Physiol. 278:L914-L922 (2000)); Alzheimer's disease (De Luigi, A. et al., Mech. Age. Dev. (16):1985-1995 (2001); Remarzue, E.J., and Bollen, E.L., Exp. Gerontol. 36(1):171-176 (2001); Town, T. et al., J. Neuroimmunol. 132(1-2):49-59 (2002)); stroke (Frenkel, D. et al., J. Immunol. 171 (12):6549-6555 (2003)); anti-phospholipid syndrome (Krause, I, et al. Eur. J. Immunol. 32(12):3414-3424 (2002)); atherosclerosis (Ohashi R. et al., Med Sci Monit 10(11): RA255-60 (2004); Zimmerman MA, et al., J Surg Res 121(2):206-13 (2004); Fichtlscherer S, et al., J Am Coll Cardiol 44(1):44-9 (2004); Potteauz S, et al., Arterioscler Thromb Vasc Biol 24(8):1474-8 (2004)); rejection from organ transplantation (Zheng HX, et al., J Heart Lung Transplant 23:541-6 (2004); Fischer S, et al., J Thora Cardiovas Surg 126:1174-80 (2003); Sembeil R, et al., Transpl Immunol 13(1):1-8 (2004)); autism (Jyonouchi, H., et al., J. Neuroimmun. 120:170-179 (2001)); chronic obstructive pulmonary disease (Takanashi, S., et al., Eur. Respir. J. 14:309-314 (1999); various autoimmune disorders, including type I diabetes mellitus (Slavin AJ, et al., Int Immunol 13(6):825-33 (20010; Zhang ZL, et al., Acta Pharmacol Sin 24(8):751-6 (2003)); arthritis, including rheumatoid arthritis (Tanaka Y, et al., Inflamm Res 45(6):283-8 (1996); Detanico T, et al., Clin Exp Immunol 135(2):336-42 (2004); Driessler F, et al., Clin Exp Immunol 135(1):64-73 (2004)), psoriasis (Asadullah K, et al., Pharmacol Rev 55(2):241-69 (2003); Asadullah K, et al., Curr Drug Targets Inflamm Allergy 3(2):185-92 (2004)) multiple sclerosis (Soos et al., J Neuroimmunol 75:43-50 (1997)); uveitis (Kezuka, T., et al., J. Immunol. 173(2):1454-1462 (2004), Sun, B., et al., Exp. Eye Res. 70:493-502 (2000); allergies (Zuany-Amorim, C. et al., J. Clin. Invest. 95:2644-2651 (1995), Borish, L., et al., J. Allergy Clin. Immunol 97:1288-1296 (1996)); inflammatory bowel disease (Li MC and He SH, World J Gastroenterol 10(5):620-5 (2004); Braat H, et al., Expert Opin Biol Ther 3(5):725-31 (2003)), and optic neuritis (Navikas, V., et al., Scand. J. Immunol. 41(2):171-178 (1995)).

It has also been reported that down-regulation of interleukin-12 (IL-12) may be beneficial in treating patients with multiple sclerosis (Tuohy, V. et al., J. Neuroimmunol., 111(1-2):55 (2000)). A link between interferon-gamma and multiple sclerosis is also reported in the literature (Moldovan, I.R. et al., J. Neuroimmunol., 141(1-2):55 (2000)).

Although many of these diseases or conditions may be improved or otherwise treated with various methods and compositions, many of such methods and compositions have drawbacks such that there is a continuing need for safe and effective methods and compositions to treat such diseases or conditions. The present invention addresses this need.

Interleukin 10 has been found to be associated with a wide variety of disease states. Accordingly, compositions and their use in treating a disease or condition responsive to IL-10 therapy in a mammal are provided.

More generally, uses for biasing the immune system toward production of Th2 cells in an animal for enhanced production of Th2-cell produced cytokines, including IL-10.

in one aspect, a composition for treating a disease or condition responsive to IL-10 therapy in a mammal comprises an orally administrable formulation of IFNτ at a daily dosage of greater than 1 x 10⁹ Units of IFNτ.

In another aspect, the use for treating an autoimmune condition in a subject comprises modulating the subject's serum cytokine levels in such a way to alleviate symptoms, inhibit progression of the condition, and/or facilitate resolution of the condition.

A patient suffering from or at risk of continued progression of a disease or condition that responds to an up-regulation of blood IL-10 is provided a dose of IFNτ sufficient to modulate selected serum cytokine levels, relative to baseline pre-treatment serum cytokine levels of that patient or of a model patient population.

In another aspect, a composition for use in the preparation of a medicament is provided, for treating a condition responsive to an increase in blood interleukin-10 (IL-10) level, and optionally a decrease in blood interleukin-12 (IL-12) level, the composition being comprised of an orally-administrable formulation of IFNτ. The medicament is preferably provided at a daily dosage of greater than 1 x 10⁹ Units.

In another aspect, the use of the composition of the invention for up-regulating the blood IL-10 level in a human subject comprises orally administering IFNτ to the subject at a daily dosage of greater than 1 x 10⁹ Units to produce an initial measurable increase in the subject's blood IL-10 level, relative to the blood IL-10 level in the subject in the absence of IFNτ administration. Oral administration of IFNτ to the subject continues on a regular basis of at least several times per week, independent of changes in the subject's blood IL-10 level, until a desired clinical endpoint is achieved.

In another aspect, the use of the composition of the invention for increasing IL-10/IFN-γ ratio in subjects suffering from an autoimmune condition comprises orally administering IFNτ to the subject at a daily dosage of greater than 1 x 10⁹ Units to produce an initial measurable increase in the subject's blood IL-10 level, relative to the blood IL-10 level in the subject in the absence of IFNτ administration, with (i) no substantial change in the subject's blood IFN-γ level relative to the IFN-γ level in the absence of IFNτ administration, or (ii) a decrease in the subject's blood IFN-γ level relative to the IFN-γ level in the absence of IFNτ administration, and continuing to orally administer IFNτ to the subject on a regular basis of at least several times per week, independent of changes in the subject's blood IL-10 level, until a desired clinical endpoint is achieved.

In another aspect, the use of the composition of the invention for preventing an increase in the blood level of IFN-γ in a subject at risk of an elevated IFN-γ blood level due to (i) administration of a therapeutic agent or (ii) a disease condition is provided. The use comprises orally administering IFNτ to the subject at a dosage of greater than 1 x 10⁹ Units, to decrease the subject's IFN-γ blood level relative to the IFN-γ blood level in the absence of IFNτ administration. The use, when applied to a subject having an elevated IFN-γ level due to an autoimmune condition, involves orally administering IFNτ during the period of the subject's symptoms. The use, when applied for treating a subject having an elevated IFN-γ level due to treatment with IFN-α or IFN-β, involves administering IFNτ during the period of the subject's symptoms.

In another aspect, the use of the composition of the invention for increasing the IL-10/IL-12 blood ratio in subjects suffering from an autoimmune disorder comprises orally administering IFNτ to the subject at a daily dosage of greater than 1 x 10⁹ Units to produce an initial measurable increase in the subject's blood IL-10 level, relative to the blood IL-10 level in the subject in the absence of IFNτ administration, and a decrease in the subject's IL-12 blood level, relative to the IL-12 level in the absence of IFNτ administration, and continuing to orally administer IFNτ to the subject on a regular basis of at least several times per week, independent of changes in the subject's blood IL-10 level, until a desired clinical endpoint is achieved.

In one embodiment, the IFNτ is ovine IFNτ or bovine IFNτ. Exemplary ovine IFNτ sequences are identified as SEQ ID NO:2 or SEQ ID NO:3.

In another embodiment, IFNτ is orally administered to the intestinal tract of the subject.

When a subject suffering from an autoimmune condition is treated in one embodiment, the desired clinical endpoint is alleviation of the subject's symptoms. Exemplary autoimmune conditions include multiple sclerosis, Type I diabetes mellitus, rheumatoid arthritis, lupus erythematosus, psoriasis, Myasthenia Gravis, Graves' disease, Hashimoto's thyroiditis, Sjogren's syndrome, ankylosing spondylitis, and inflammatory bowel disease.

In another embodiment, administration of IFNτ is combined with administration of a second therapeutic agent, simultaneously or sequentially. Exemplary second therapeutic agents incude anti-viral agents; anti-cancer agents, and agents suitable for treatment of autoimmune disorders.

In another aspect, the use in slowing the progression of multiple sclerosis in a subject comprises orally administering IFNτ to the subject at a daily dosage of greater than 1 x 10⁹ Units to produce an initial measurable increase in the subject's blood IL-10 level, relative to the blood IL-10 level in the subject in the absence of IFNτ administration, and continuing to orally administer IFNτ to the subject on a regular basis of at least several times per week, independent of changes in the subject's blood IL-10 level.

In yet another aspect, the use in reducing the risk of relapse in a subject suffering from multiple sclerosis comprises orally administering IFNτ to the subject at a daily dosage of greater than 1 x 10⁹ Units to produce an initial measurable increase in the subject's blood IL-10 level, relative to the blood IL-10 level in the subject in the absence of IFNτ administration, and continuing to orally administer IFNτ to the subject on a regular basis of at least several times per week, independent of changes in the subject's blood IL-10 level.

In yet another aspect, the use in treating a patient suffering from psoriasis comprises orally administering a dose of IFNτ on a regular basis to achieve an increased serum IL-10 level, relative to the blood IL-10 level in the subject in the absence of IFNτ administration. Treatment continues until a reduction in a clinically suitable assessment score is achieved. Preferably the reduction is of at least about 50%, more preferably at least about 70%, still more preferably of at least about 80%. For example, a reduction of at least about 70% in the Physician's Static Global Assessment score is achieved; alternatively a reduction of at least about 50% in a scaling score, a plaque score, or an erythema score is obtained. Methods of reducing a psoriasis assessment score in a person afflicted with psoriasis and of reducing the time between psoriatic flare-ups are contemplate.

In still another aspect, the composition of the invention is for use in treating an autoimmune condition in a subject. The use comprises administering to the subject IFNτ in an amount sufficient to produce an initial measurable increase in the subjects's blood IL-10 level, relative to the blood IL-10 level in the subject in the absence of IFNτ administration; ceasing administration of IFNτ for a selected period of time during which the subject's blood IL-10 level remains increased relative to the blood IL-10 level in the subject in the absence of IFNτ administration; and resuming administration of IFNτ.

These and other objects and features of the invention will be more fully appreciated when the following detailed description of the invention is read in conjunction with the accompanying drawings.

### Brief Description of the Drawings

Figs. 1A-1C are graphs showing the IL-10 serum level, in pg/mL, in human patients suffering from multiple sclerosis and treated orally with IFNτ, as a function of time, in days, for patient groups I, II, and III treated daily with 0.2 mg IFNτ (Fig. 1A), 0.6 mg IFNτ (Fig. 1B), and 1.8mg IFNτ (Fig. 1C) from days 1-29.

Fig. 1D is a graph showing the mean IL-10 serum level, in pg/mL, for the human patients in each of the test Groups I, II, and III treated daily with 0.2 mg IFNτ (diamonds, Group I), 0.6 mg IFNτ (squares, Group II), and 1.8 mg IFNτ (triangles, Group III) from days 1-29.

Figs. 2A-2C are graphs showing the IFN-γ serum level, in pg/mL, in human patients suffering from multiple sclerosis and treated orally with IFNτ, as a function of time, in days, for patient groups I, II, and III treated daily with 0.2 mg IFNτ (Fig. 2A), 0.6 mg IFNτ (Fig. 2B), and 1.8 mg IFNτ (Fig. 2C) from days 1-29.

Fig. 2D is a graph showing the mean IFN-γ serum level, in pg/mL, for the human patients in each of the test Groups I, II, and III treated daily with 0.2 mg IFNτ (diamonds, Group I), 0.6 mg IFNτ (squares, Group II), and 1.8 mg IFNτ (triangles, Group III) from days 1-29.

Figs. 3A-3E show IL-10 (diamonds) and IFN-γ (squares) serum concentrations, both in pg/mL, for selected individual patients from the treatment Groups I, II, and III discussed with respect to Figs. 1-2.

Figs. 4A-4C are graphs showing the IL-10 serum level, in pg/mL, in human patients suffering from hepatitis C and treated orally with IFNτ, as a function of time, in days, for the six patients in Test Group I treated daily with 0.33 mg IFNτ three times daily (Fig. 4A), for the six patients in Test Group II treated daily with 1.0 mg IFNτ three times daily (Fig. 4B); and for the six patients in Test Group IIII treated daily with 3 mg IFNτ three times daily (Fig. 4C).

Fig. 4D is a summary plot for the test Groups I, II, and III in Figs. 4A-4C, showing the percent increase in serum IL-10 levels as a function of time for test Group I (diamonds, 0.33 mg three times daily), Group II (squares, 1 mg three times daily), and Group III (triangles, 3 mg three times daily).

Figs. 5A-5C are graphs showing the IFN-γ serum level, in pg/mL, in human patients suffering from hepatitis C and treated orally with IFNτ, as a function of time, in days, for the six patients in Test Group I treated daily with 0.33 mg IFNτ three times daily (Fig. 5A), for the six patients in Test Group II treated daily with 1.0 mg IFNτ three times daily (Fig. 5B); and for the six patients in Test Group IIII treated daily with 3 mg IFNτ three times daily (Fig. 5C).

Fig. 5D is a summary plot for the test Groups I, II, and III in Figs. 5A-5C, showing the mean serum IFN-γ levels as a function of time for test Group I (diamonds, 0.33 mg three times daily), Group II (circles, 1 mg three times daily), and Group III (triangles, 3 mg three times daily).

Figs. 6A-6F show IL-10 (diamonds) and IFN-γ (squares) serum concentrations, both in pg/mL, for selected individual patients from the treatment Groups I, II, and III discussed with respect to Figs. 4-5.

Figs. 7A-7B are graphs showing the IL-10 serum level (Fig. 7A) and the IFN-γ serum level (Fig. 7B), in pg/mL, in human patients suffering from hepatitis C and treated orally with IFNτ, as a function of time, in days, where a 7.5 mg dose of IFNτ was given twice a day on an empty stomach.

Figs. 8A-8D show the IL-10 (diamonds), IFN-γ (squares), and IL-12 (triangles) serum levels, in pg/mL, for the patients treated as described with respect to Figs. 7A-7B, where the IL-12 values are presented as 0.1 times the measured IL-12 serum level.

### Brief Description of the Sequences

SEQ ID NO: is the nucleotide sequence of a synthetic gene encoding ovine interferon-T (IFNτ).

SEQ ID NO:2 corresponds to an amino acid sequence of mature ovine interferon-τ (IFNτ; oTP-1; GenBank Accession No. Y00287; PID g1358).

SEQ ID NO:3 corresponds to an amino acid sequence of mature ovine IFNτ, where the amino acid residues at positions 5 and 6 of the sequence are modified relative to the sequence of SEQ ID NO:2.

SEQ ID NO:4 is a synthetic nucleotide sequence encoding the protein of SEQ ID NO:3.

SEQ ID NO:5 corresponds to an amino acid sequence of human myelin oligodendrocyte protein (GenBank Accession No. BC035938).

SEQ ID NO:6 corresponds to an amino acid sequence of human myelin basic protein (GenBank Accession No. NM_002385).

### Detailed Description of the Invention

### I. Definitions

*Interferon-tau*, abbreviated as IFNτ or interferon-τ, refers to any one of a family of interferon proteins having at least one characteristic from each of the following two groups of characteristics: (i) (a) anti-luteolytic properties, (b) anti-viral properties, or (c) anti-cellular proliferation properties; and (ii) about 45 to 68% amino acid homology with an α-interferon or greater than about 70% amino acid homology to known IFNτ sequences (e.g., Ott, et al., J. Interferon Res., 11:357 (1991); Helmer, et al., J. Reprod. Fert., 79:83 (1987); Imakawa, et al., Mol. Endocrinol, 3:127 (1989); Whaley, et al., J. Biol. Chem., 269:10846 (1994); Bazer, et al., WO 94/10313 (1994)). Amino acid homology or sequence "identity" is determined by comparing the amino acid sequences of the proteins when aligned so as to maximize overlap and identity while minimizing sequence gaps. The percent identity of two amino acid or two nucleic acid sequences can be determined by visual inspection and/or mathematical calculation, or more preferably, the comparison is done by comparing sequence information using one of many available computer programs. IFNτ sequences have been identified in various ruminant species, including but not limited to, cow (*Bovine sp.*, Helmer S.D., J. Reprod. Fert., 79:83 (1987); Imakawa, K., Mol. Endocrinol., 119:532 (1988)), sheep (*Ovine sp.*), musk ox (*Ovibos sp.*), giraffe (*Giraffa sp.*, GenBank Accession no. U55050), horse (*Equus caballus*), zebra (*Equus burchelli*, GenBank Accession no. NC005027), hippopotamus (*Hippopotamus sp.*), elephant (*Loxodonta sp.*), Ilama (*Llama glama*), goat (*Capra sp.*, GenBank Accession nos. AY357336, AY357335, AY347334, AY357333, AY357332, AY357331, AY357330, AY357329, AY357328, AY357327), and deer (*Cervidae sp.*). The nucleotide sequences of IFNτ for many of these species are reported in public databases and/or in the literature (see, for example, Roberts, R.M. et al., J. Interferon and Cytokine Res., 18:805 (1998), Leaman D.W. et al., J. Interferon Res., 12:1 (1993), Ryan, A.M. et al., Anim. Genet., 34:9 (1996)). IFNτ intends to encompass the IFNτ protein from any ruminant species, exemplified by those recited above, that has at least one characteristic from each of the two groups of characteristics listed above.

*Ovine* IFNτ (*OvIFNτ*) refers to a protein having the amino acid sequence as identified herein as SEQ ID NO:2, and to proteins having amino acid substitutions and alterations such as neutral amino acid substitutions that do not significantly affect the activity of the protein, such as the IFNτ protein identified herein as SEQ ID NO:3. More generally, an ovine IFNτ protein is one having about 80%, more preferably 90%, sequence homology to the sequence identified as SEQ ID NO:2. Sequence homology is determined, for example, by a strict amino acid comparison or using one of the many programs commercially available.

*Treating* a condition refers to administering a therapeutic substance effective to reduce the symptoms of the condition and/or lessen the severity of the condition.

*Oral* refers to any route that involves administration by the mouth or direct administration into the stomach or intestines, including gastric administration.

*Intestine* refers to the portion of the digestive tract that extends from the lower opening of the stomach to the anus, composed of the small intestine (duodenum, jejunum, and ileum) and the large intestine (ascending colon, transverse colon, descending colon, sigmoid colon, and rectum).

"*Measurable increase in blood IL-10 level*" refers to a statistically meaningful increase in blood (serum and/or blood-cell) levels of IL-10, typically at least a 20% increase, more preferably at least a 25% increase, over pre-treatment levels measured under identical conditions. Methods for measuring IL-10 levels in the blood are described herein using a commercially-available enzyme-linked immunosorbent assay (ELISA) kit. A fold-increase is determined by dividing the value at timepoint x by the screening or baseline value. A percent increase is determined by finding the difference between the value at timepoint x and the screening or baseline value; dividing this difference by the screening or baseline value; and multiplying the quotient by 100.

*"Measurable decrease in blood IL-92 level*" refers to a statistically meaningful increase in blood (serum and/or blood-cell) levels of interleukin-12, typically at least a 20% increase, more preferably at least a 25% increase, over pre-treatment levels measured under identical conditions. Methods for measuring IL-12 levels in the blood are described herein using a commercially-available enzyme-linked immunosorbent assay (ELISA) kit. A fold-increase is determined by dividing the value at timepoint x by the screening or baseline value. A percent increase is determined by finding the difference between the value at timepoint x and the screening or baseline value; dividing this difference by the screening or baseline value, and multiplying the quotient by 100.

"*Maintaining interferon-gamma blood level*" or "*no substantial decrease in interferon-gamma blood level*" refers to no statistically meaningful change in blood (serum and/or blood-cell) level of interferon-gamma. Methods for measuring interferon-gamma levels in the blood are described herein using a commercially-available enzyme-linked immunosorbent assay (ELISA) kit.

*A* "*daily dosage of greater than 5 x 10⁸ Units*" refers to an amount of IFNτ sufficient to provide more than about 5x10⁸ antiviral Units of protein, where the antiviral activity of IFNτ is measured using a standard cytopathic effect inhibition assay, such as that described in the Methods section below. It will be appreciated that the amount (i.e., mg) of protein to provide a daily dosage of greater than 5x10⁸ Units will vary according to the specific antiviral activity of the protein.

### II. Interferon-τ Compositions and Method of Treatment

### A. Interferon-τ

The first IFNτ to be identified was ovine IFNτ (IFNτ), as a 18-19 kDa protein. Several isoforms were identified in conceptus (the embryo and surrounding membranes) homogenates (Martal, J., et al., J. Reprod. Fertil. 56:63-73 (1979)). Subsequently, a low molecular weight protein released into conceptus culture medium was purified and shown to be both heat labile and susceptible to proteases (Godkin, J.D., et al., J. Reprod. Fertil. 65:141-150 (1982)). IFNτ was originally called ovine trophoblast protein-one (oTP-1) because it was the primary secretory protein initially produced by trophectoderm of the sheep conceptus during the critical period of maternal recognition in sheep. Subsequent experiments have determined that IFNτ is a pregnancy recognition hormone essential for establishment of the physiological response to pregnancy in ruminants, such as sheep and cows (Bazer, F.W., and Johnson, H.M., Am. J. Reprod. Immunol. 26:19-22 (1991)).

An IFNτ cDNA obtained by probing a sheep blastocyst library with a synthetic oligonucleotide representing the N-terminal amino acid sequence (Imakawa, K. et al, Nature, 330:377-379, (1987)) has a predicted amino acid sequence that is 45-55% homologous with IFN-αs from human, mouse, rat, and pig and 70% homologous with bovine IFN-αII, now referred to as IFN-Ω. Several cDNA sequences have been reported which may represent different isoforms (Stewart, H.J., et al, Mol. Endocrinol. 2:65 (1989); Klemann, S.W., et al., Nuc. Acids Res. 18:6724 (1990); and Charlier, M., et al., Mol. Cell Endocrinol. 76:161-171 (1991)). All are approximately 1 kb with a 585 base open reading frame that codes for a 23 amino acid leader sequence and a 172 amino acid mature protein. The predicted structure of IFNτ as a four helical bundle with the amino and carboxyl-termini in apposition further supports its classification as a type I IFN (Jarpe, M.A., et al., Protein Engineering 7:863-867 (1994)).

| **Overview of the Interferons** | | | | |
|---|---|---|---|---|
| **Aspects** | **Type I** | **Type I** | **Type I** | **Type II** |
| Types | α&ω | β | τ | γ |
| Produced by: | leukocyte | fibroblast | trophoblast | lymphocyte |
| Antiviral | + | + | + | + |
| Antiproliferative | + | + | + | + |
| Pregnancy Signaling | - | - | + | - |

While IFNτ displays some of the activities classically associated with type I interferons (see Table, above), considerable differences exist between it and the other type I interferons as described above. These differences include biological activity, induction by virus, period of expression after induction, homology with other type I interferons, etc. Other differences may exist in the regulatory regions of the IFNτ gene. For example, transfection of the human trophoblast cell line JAR with the gene for bovine IFNτ resulted in antiviral activity while transfection with the bovine IFN-Ω gene did not. This implies unique transacting factors involved in IFNτ gene expression. Consistent with this is the observation that while the proximal promoter region (from 126 to the transcriptional start site) of IFNτ is highly homologous to that of IFN-α and IFN-β; the region from -126 to -450 is not homologous and enhances only IFNτ expression (Cross, J.C. et al.. , Proc. Natl. Acad. Sci. USA, 88:3817-3821 (1991)). Thus, different regulatory factors appear to be involved in IFNτ expression as compared with the other type I interferons.

The 172 amino acid sequence of ovine-IFNτ is set forth, for example, in U.S. Patent No. 5,958,402, and its homologous bovine-IFNτ sequence is described, for example, in Helmer et al., J. Reprod. Fert., 79:83-91 (1987) and Imakawa, K. et al., Mol. Endocrinol., 3:127 (1989). The sequences of ovine-IFNτ and bovine-IFNτ from these references are hereby incorporated by reference. Exemplary amino acid sequences of ovine IFNτ are shown herein as SEQ ID NO:2 and SEQ ID NO:3.

### 1. Isolation of IFNτ

IFNτ may be isolated from conceptuses collected from pregnant sheep and cultured *in vitro* in a modified minimum essential medium as described by Godkin, J.D., et al., J. Reprod. Fertil. 65:141-150 (1982) and Vallet, J.L., et al., Biol. Reprod. 37:1307 (1987). The IFNτ may be purified from the conceptus cultures by ion exchange chromotography and gel filtration. The homogeneity of isolated IFNτ may be assessed by sodium dodecyl sulfate polyacrylamide gel electrophoresis (Maniatis, T., et a/., in MOLECULAR CLONING: A LABORATORY MANUAL, Cold Spring Harbor Laboratory, Cold Spring Harbor, NY (1982); Ausubel, F.M., et al., in CURRENT PROTOCOLS IN MOLECULAR BIOLOGY, John Wiley & Sons, Inc., Media, PA (1988)), and determination of protein concentration in purified IFNτ samples may be performed using the bicinchoninic (BCA) assay (Pierce Chemical Co., Rockford, IL; Smith, P.K., et al., Anal. Biochem. 150:76 (1985)).

### 2. Recombinant Production of IFNτ

Recombinant IFNτ protein may be produced from any selected IFNτ polynucleotide fragment using a suitable expression system, such as bacterial or yeast cells. The isolation of IFNτ nucleotide and polypeptide sequences is described in PCT publication WO/94/10313, which is incorporated by reference herein.

To make an IFNτ expression vector, an IFNτ coding sequence (*e.g*, SEQ ID NOS:1 or 4) is placed in an expression vector, *e.g*., a bacterial expression vector, and expressed according to standard methods. Examples of suitable vectors include lambda gt11 (Promega, Madison WI); pGEX (Smith, P.K. et al., Anal. Biochem. 150:76 (1985)); pGEMEX (Promega); and pBS (Strategene, La Jolla CA) vectors. Other bacterial expression vectors containing suitable promoters, such as the T7 RNA polymerase promoter or the tac promoter, may also be used. Cloning of the IFNτ synthetic polynucleotide into a modified pIN III omp-A expression vector is described in the Methods section below.

For the studies described herein, the IFNτ coding sequence present in SEQ ID NO:4 was cloned into a vector, suitable for transformation of yeast cells, containing the methanol-regulated alcohol oxidase (AOX) promoter and a Pho1 signal sequence. The vector was used to transform *P. pastoris* host cells and transformed cells were used to express the protein according to the manufacturer's instructions (Invitrogen, San Diego, CA).

Other yeast vectors suitable for expressing IFNτ for use with methods of the present invention include 2 micron plasmid vectors (Ludwig, D.L. et al., Gene, 132:33 (1993)), yeast integrating plasmids (Shaw, K.J. et al., DNA, 7:117 (1988)), YEP vectors (Shen, L.P. et al., Sci. Sin., 29:856 (1986)), yeast centromere plasmids (YCps), and other vectors with regulatable expression (Hitzeman, R.A. et al., U.S. Patent No. 4,775,622; Rutter, W.J. et al., U.S. Patent No. 4,769, 238; Oeda, K. et al., U.S. Patent No. 4,766,068). Preferably, the vectors include an expression cassette containing an effective yeast promoter, such as the MFα1 promoter (Bayne, M.L. et al., Gene 66:235-244 (1988), GADPH promoter (glyceraldehyde-3-phosphate-dehydrogenase; Wu, D.A. et al., DNA, 10:201 (1991)) or the galactose-inducible GAL10 promoter (Ludwig, D.L. et al., Gene, 132:33 (1993); Feher, Z. et al., Curr. Genet., 16:461 (1989)); Shen, L.P. et al., Sci. Sin., 29:856 (1986)). The yeast transformation host is typically *Saccharomyces cerevisiae,* however other yeast suitable for transformation can be used as well (*e.g., Schizosaccharomyces pombe, Pichia pastoris,* and the like).

Further, a DNA encoding an IFNτ polypeptide can be cloned into any number of commercially available vectors to generate expression of the polypeptide in the appropriate host system. These systems include the above described bacterial and yeast expression systems as well as the following: baculovirus expression (Reilly, P.R. et al., BACULOVIRUS EXPRESSION VECTORS: A LABORATORY MANUAL, (1992); Beames et al., Biotechniques, 11:378 (1991); Clontech, Palo Alto CA); plant cell expression, transgenic plant expression, and expression in mammalian cells (Clontech, Palo Alto CA; Gibco-BRL, Gaithersburg MD). The recombinant polypeptides can be expressed as fusion proteins or as native proteins. A number of features can be engineered into the expression vectors, such as leader sequences which promote the secretion of the expressed sequences into culture medium. The recombinantly produced polypeptides are typically isolated from lysed cells or culture media. Purification can be carried out by methods known in the art including salt fractionation, ion exchange chromatography, and affinity chromatography. Immunoaffinity chromatography can be employed, as described above, using antibodies generated based on the IFNτ polypeptides.

In addition to recombinant methods, IFNτ proteins or polypeptides can be isolated from selected cells by affinity-based methods, such as by using appropriate antibodies. Further, IFNτ peptides (*e.g.* SEQ ID NOS:2 or 3) may be chemically synthesized using methods known to those skilled in the art.

### B. Administration of IFNτ

In studies performed in support of the invention, IFNτ was administered to patients suffering from conditions that respond to IL-10 therapy. Specifically, in one study patients diagnosed with multiple sclerosis were treated with IFNτ. In another study, patients suffering from a viral infection were treated with IFNτ. During the studies, the blood serum levels of the cytokines IL-10, IFN-γ, and IL-12 were monitored in each patient. These studies will now be described.

### 1. Administration of IFNτ to Humans Suffering from Multiple Sclerosis

Humans suffering from multiple sclerosis were enrolled in a trial for treatment with IFNτ. As described in Example 1, 15 patients were randomized into three treatment groups, summarized in Table 1.

**Table 1**

| | **Group I** | **Group II** | **Group III** |
|---|---|---|---|
| | **(n=5)** | **(n=5)** | **(n=5)** |
| IFNτ Oral Dose¹ | 0.2mg/day | 0.6mg/day | 1.8 mg/day |
| | (2 × 10⁷ U) | (6 × 10⁷ U) | (1.8 × 10⁸ U) |
| Average Weight | 67.2 kg | 58.9 kg | 90.0 kg |
| Average Age | 30 | 34.5 | 47 |

| | | | |
|---|---|---|---|
| ¹1 mg IFNτ = 1 × 10⁸ Units | | | |

Prior to treatment, blood samples were taken from each subject to determine a baseline serum cytokine concentration. After the blood draw on Day 1, each patient began treatment by taking the IFNτ orally in the appropriate dose. Treatment continued for 28 days and blood samples were taken from each patient on days 1, 4, 8, 15, 29, and 57 of the study. The samples were analyzed for IFN-γ and IL-10 concentrations.

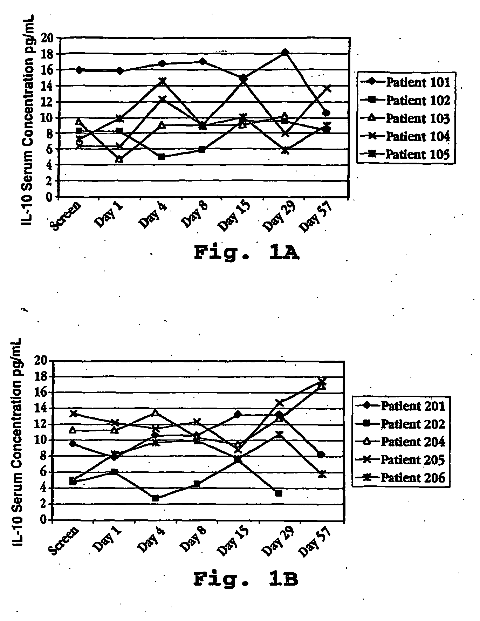

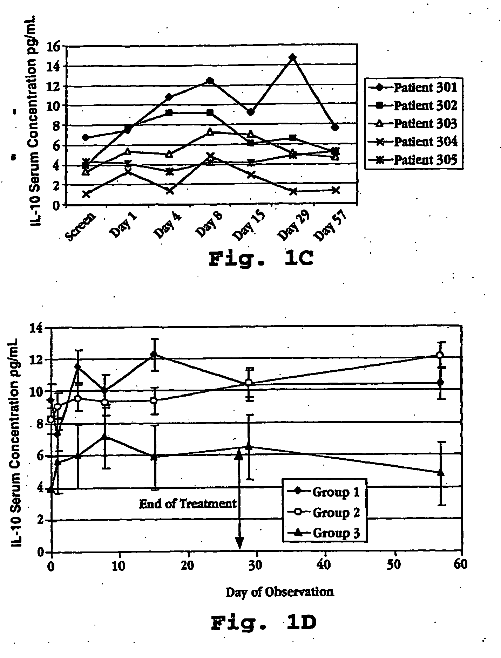

The IL-10 levels for the patients in Groups I, II, and III are shown in Figs. 1A-1C, respectively. Fig. 1A shows serum IL-10 levels, in pm/mL, for the five patients in Group I. Three of the patients, patient numbers 103, 104, and 105, showed an increase in IL-10 level at Day 4, however the IL-10 levels decreased on the Day 8 reading in these patients. The IL-10 levels at Days 8 and 15 in Patient nos. 103 and 104 were not significantly changed from the level at Day 4. Figs. 1 B and 1C show the results for the patients in test Groups II and III, respectively. There is a suggestion of a slight increase in serum IL-10 levels after administration of IFNτ, particularly in the Group III patients.

Fig. 1D shows the mean IL-10 serum levels, in pg/mL, for Groups I, II, and III. A slight upregulation of IL-10 in the test groups during the period of IFNτ dosing, between Days 2 and 28, however, the slight upregulation was not statistically significant, based on the statistical analysis set forth in Example 1. The small increase in IL-10 blood level continued in Groups I and II for a period of time after dosing with IFNτ was stopped on Day 28. The IL-10 serum levels at Day 57, which is 34 days after the last dose of IFNτ, remained above the baseline levels measured on Day 0 and Day 1. Thus, the invention contemplates a method of treating an autoimmune condition in a subject, where IFNτ is administered in an amount sufficient to produce an initial measurable increase in the subject's blood IL-10 level, relative to the blood IL-10 level in the subject in the absence of IFNτ administration. Then, administration of IFNτ is terminated for a selected period of time during which the subject's blood IL-10 level remains increased relative to the blood IL-10 level in the subject in the absence of IFNτ administration. Administration of IFNτ may then resume as desired.

In this study, the blood levels of IFN-γ were also monitored. IFN-γ is a pro-inflammatory cytokine, and up-regulation of IFN-γ is correlated with increased discomfort in patients suffering from autoimmune conditions, such as multiple sclerosis and arthritis. During treatment of multiple sclerosis with interferon-beta (IFN-β), it has been reported that the frequency of IFN-γ-secreting cells increases during the first two months of IFN-β treatment, and this increase of IFN-γ serum levels possibly contributes to the prominent "flu-like" symptoms that patients experience during treatment with IFN-β. Thus, a method of treating autoimmune conditions where IL-10 levels are favorably up-regulated with no accompanying up-regulation of IFN-γ would be beneficial.

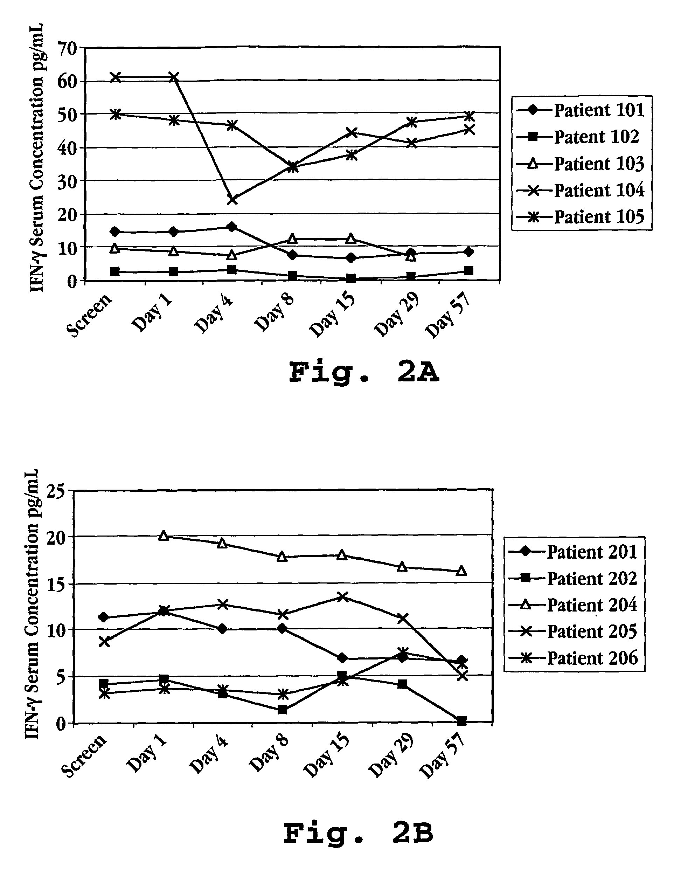

Figs. 2A-2D show the IFN-γ blood levels, in pg/mL, for the patients in Groups I, II, and III, suffering from multiple sclerosis and treated orally with IFNτ. Fig. 2A shows the serum levels for the patients in Group I, treated with 0.2 mg IFNτ. Patient nos. 101, 102, 104, 105 each had a reduction in IFN-γ blood level during the course of treatment. The serum levels increased upon cessation of treatment at Day 28. The IFN-γ serum level in patient no. 103 did not increase, but remained essentially unchanged.

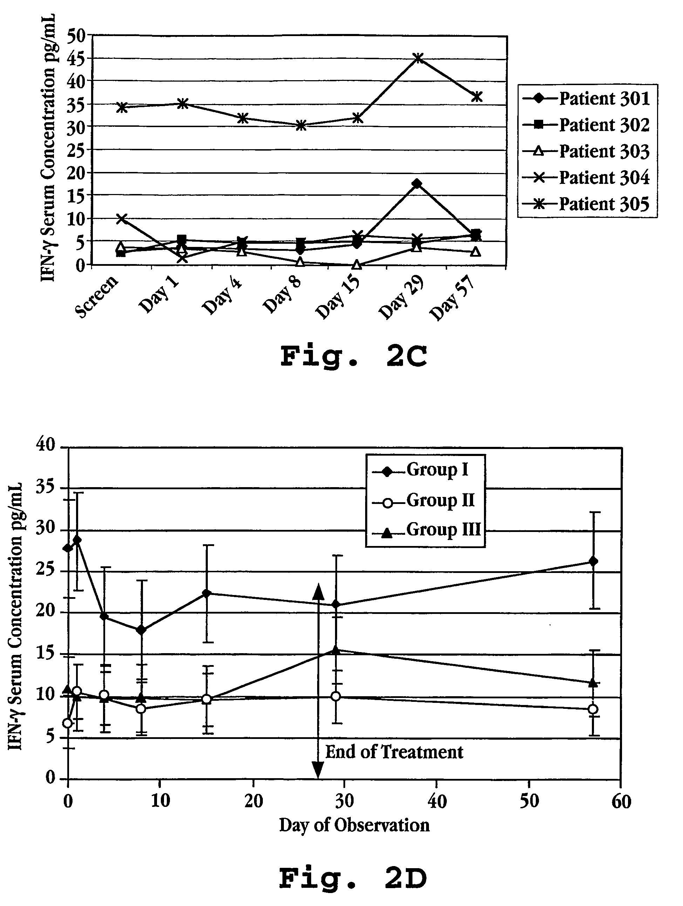

Fig. 2B shows the IFN-γ blood levels, in pg/mL, for the patients in Group II, and treated with 0.6 mg IFNτ daily. Fig. 2C shows the IFN-γ blood levels, in pg/mL, for the patients in Group III, and treated with 1.8 mg IFNτ daily. As noted above, the first dose of IFN-τ was taken after the blood draw on Day 1 and the final dose was taken on Day 28. Thus, the data points at Day 1 and "screen" are baseline levels for the individual patients. All patients in Groups II and III experienced either a reduction in IFN-γ serum levels or no meaningful change in IFN-γ serum level during treatment with IFN-γ.

Fig. 2D summarizes the mean blood level of IFN-γ, in pg/mL, for the patients in each of the test Groups I, II, and III. The decreasing trend of the IFN-γ blood levels is apparent, particularly when the higher doses of IFN-τ are administered (Group III).

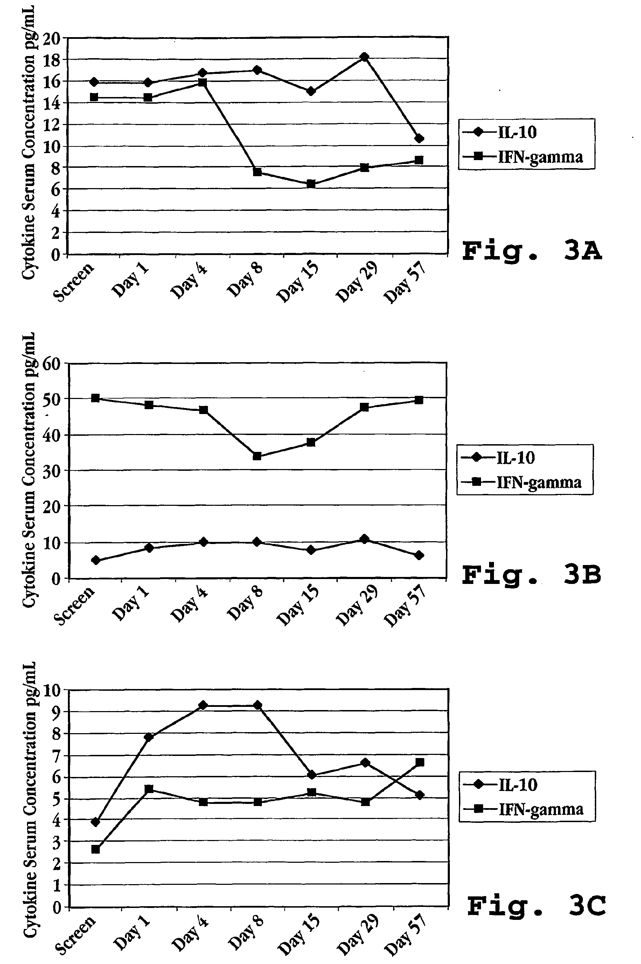

Figs. 3A-3E show IL-10 (diamonds) and IFN-γ (squares) serum concentrations, both in pg/mL, for selected individual patients from the treatment Groups I, II, and III. Fig. 3A shows the cytokine production kinetics for patient number 101, in treatment Group I. The blood IL-10 level (diamonds) does not increase statistically during the treatment period. The IFN-γ blood level decreases during treatment with orally administered IFN-τ. The baseline levels of IL-10 and IFN-γ were 15.8 pg/mL and 14.5 pg/mL, respectively, to give an initial IL-10/IFN-γ ratio of 1.1. During treatment with IFN-τ, the IL-10/IFN-γ ratio increased to about 2.2, due to the decreasing IFN-γ blood level. The IL-10/IFN-γ ratio returned to the baseline ratio of about 1.1 at Day 57, about a month after treatment ended. Thus, during the period of treatment with IFN-τ, the IL-10/IFN-γ ratio was increased by about 100%.

Fig. 3B shows the cytokine production kinetics for patient number 105, in treatment Group I. The baseline levels of IL-10 and IFN-γ were on average of 6.6 pg/mL and 49.2 pg/mL, respectively, to give an initial IL-10/IFN-γ ratio of 0.13. During treatment with IFN-τ, the IL-10/IFN-γ ratio increased to about 0.2-0.3, due to a decrease in IFN-γ blood level. The IL-10/IFN-γ ratio returned to the baseline ratio of about 0.12 at Day 57, about a month after treatment ended. Thus, treatment with IFNτ was effective to modulate the IL-10/IFN-γ ratio, increasing the ratio by more than 50%, more preferably by more than 80%.

Fig. 3C shows the cytokine production kinetics for patient number 302, in treatment Group III. The baseline levels (taken as an average of Screen and Day 1) of IL-10 and IFN-γ were 5.8 pg/mL and 4.0 pg/mL, respectively, to give an initial IL-10/IFN-γ ratio of 1.45. During treatment with IFN-τ, the average IL-10 blood level (average of lL-10 levels on Days 4, 8, 15) was 7.7 pg/mL, which was not statistically different than the baseline IL-10 level (average of IL-10 blood levels at Screen and Day 1). The IFN-γ level remained substantially unchanged over the treatment period. The IL-10 /IFN-γ ratio for this patient remained essentially unchanged.

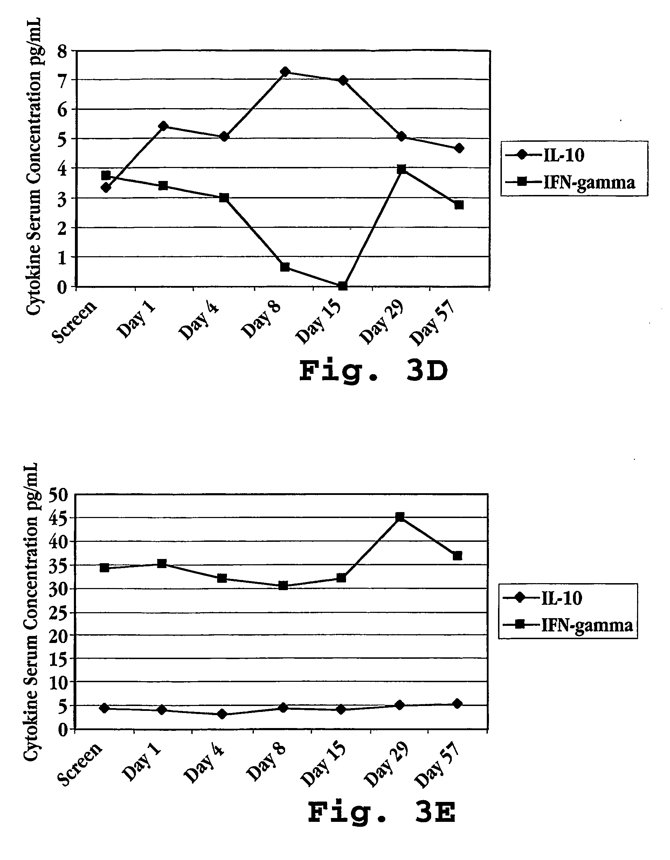

Fig. 3D shows the cytokine production kinetics for patient number 303, in treatment Group III. The baseline levels (taken as an average of Screen and Day 1) of IL-10 and IFN-γ were 4.4 pg/mL and 3.6 pg/mL, respectively, to give an initial IL-10/IFN-γ ratio of 1.2. During treatment with IFN-τ, due to a decrease in IFN-γ blood level, the IL-10 / IFN-γ ratio increased to about 11 on Day 8, with a return to the baseline ratio at Day 29.

Fig. 3E shows the cytokine production kinetics for patient number 305 in treatment Group III. The baseline level (taken as an average of Screen and Day 1) of IL-10 and IFN-γ were 4.3 pg/mL and 34.8 pg/mL, respectively, to give an initial IL-10/IFN-γ ratio of 0.1. During treatment with IFN-τ, the IL-10 blood level was essentially constant; the IFN-γ blood level decreased slightly, to give an IL-10/IFN-γ ratio increase by about 14%, to 0.14, on Day 8.

Thus, in one aspect, a method of increasing IL-10/IFN-γ ratio in subjects suffering from an autoimmune condition or a viral infection. The method comprises administering IFNτ to the subject in an amount effective to produce an initial measurable increase in the subject's blood IL-10 level, relative to the blood IL-10 level in the subject in the absence of IFNτ administration, with (i) no substantial change in the subject's blood IFN-γ level relative to the IFN-γ level in the absence of IFNτ administration or (ii) a decrease in the subject's blood IFN-γ level relative to the IFN-γ level in the absence of IFNτ administration. The IL-10/IFN-γ ratio is increased by at least about 10%, preferably by about 25%, more preferably by about 40%, still more preferably by at least about 50%. In one embodiment, the IFNτ is ovine or bovine IFNτ. In another embodiment, the IFNτ is administered at a dose of greater than about 5 x 10⁸ antiviral Units (U), more preferably, at a dose of 0.5 x 10⁹ U or more, still more preferably at a dose of 1 x 10⁹ U or more.

### 2. Administration to Humans Suffering from a Viral Infection

In another study, human patients infected with a virus, hepatitis C, were recruited. The patients were divided into four test groups for treatment with oral IFNτ (SEQ ID NO:3). As described in Example 2, each subject in the test groups self-administered three times daily a controlled volume of a 1 mg/mL solution of IFNτ. Patients in Test Groups I, II, and III received a total daily dose of 1 mg IFNτ, 3 mg IFNτ, or 9 mg IFNτ, respectively (1 mg IFNτ is approximately 1 x 10⁸ antiviral Units). The treatment period lasted for 84 days, with the patients returning to the test clinic at defined intervals to provide a blood sample for analysis of the levels of IL-10 and IFN-γ. Monitoring continued for 169 days, 85 days after the end of treatment with IFNτ.

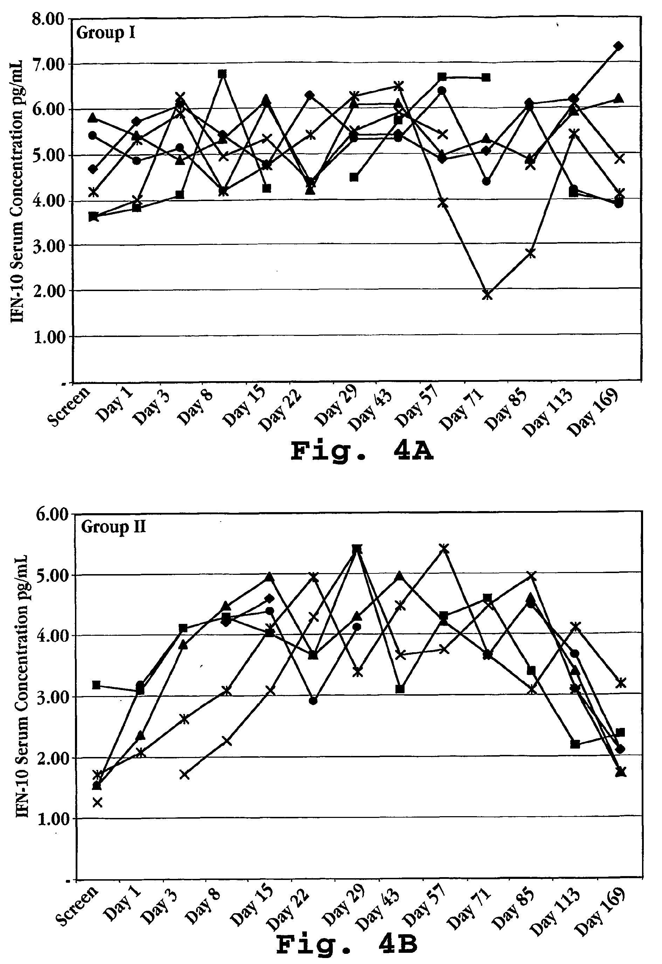

Figs. 4A-4C are graphs showing the IL-10 serum level, in pg/mL, in the six patients in each of the test Groups I, II, and III. Fig. 4A shows the IL-10 levels for the six patients in Test Group I treated daily with 0.33 mg IFNτ three times daily, for a total daily dose of 1 mg (1 x 10⁸ U). The data for all patients shows a slight, though not statistically significant, trend toward increasing IL-10 levels.

Fig. 4B shows the data for the six patients in Test Group II, each treated daily with 1.0 mg IFNτ three times daily (3 x 10⁸ U/day) until Day 84. The data for all patients shows a more definite, yet not statistically significant, trend toward increasing IL-10 levels over the treatment period (Days 1-84). Upon cessation of IFNτ dosing, the IL-10 blood levels slowly approached baseline levels over the period of continued monitoring from Day 85-169.

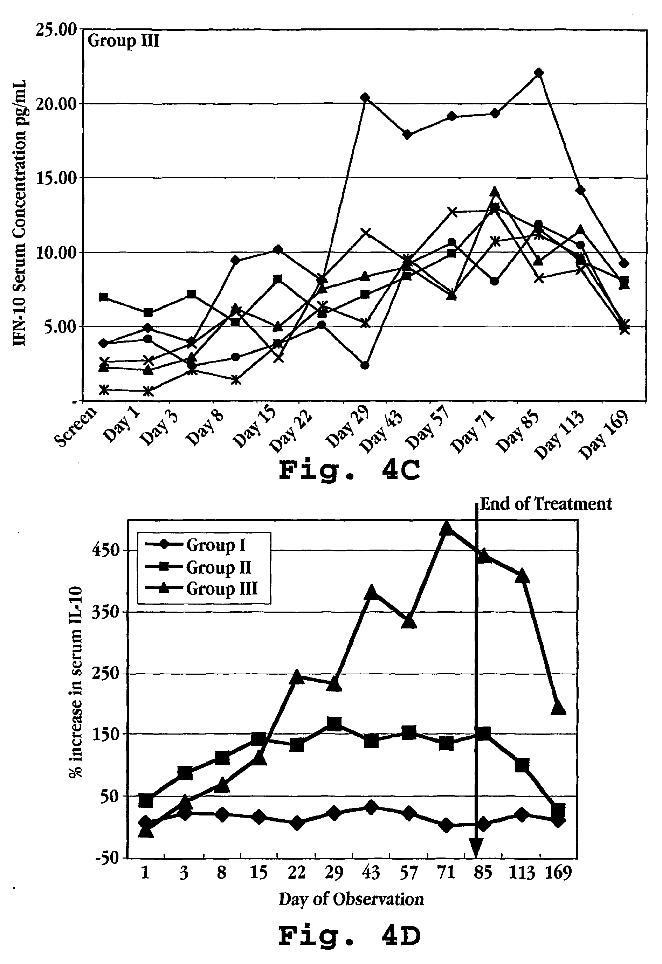

Fig. 4C shows the IL-10 serum levels for the six patients in test Group III, treated daily with 3 mg IFNτ three times daily (9 x 10⁸ U/day) from Day 1 to Day 84. All patients had a statistically increased serum IL-10 level in response to dosing with IFNτ. Upon termination of IFNτ dosing, the IL-10 blood levels remained elevated for nearly 3 months.

Fig. 4D is a summary plot of the IL-10 serum levels for the test Groups I, II, and III in Figs. 4A-4C. Fig. 4D shows the percent increase in serum IL-10 levels as a function of time for test Group I (diamonds, 0.33 mg three times daily), Group II (squares, 1 mg three times daily), and Group III (triangles, 3 mg three times daily). The percent increase in serum IL-10 level as a function of dose is evident from the drawing, with the highest dose of 9 mg (3 mg three times daily; (9 x 10⁸ U/day) inducing an up-regulation of IL-10 of more than 100% within the first 15 days of treatment. A daily dose of 3 mg (test Group II, squares) stimulated IL-10 production to cause about a 150% increase by test Day 15. The 3 mg daily dose was sufficient to maintain the 150% increase for the 84 day test period.

Fig. 4D also illustrates the continued elevation in IL-10 levels, relative to baseline, pretreatment levels, during the period of days 85-169 when dosing of IFNτ had ceased. In test Group III (9 mg IFNτ daily), the IL-10 level had not returned to baseline levels by day 169. Thus, a method of treating an autoimmune condition, particularly multiple sclerosis, psoriasis, rheumatoid arthritis and allergies, by administering to the subject IFNτ in an amount sufficient to produce an initial measurable increase in the subject's blood IL-10 level, relative to the blood IL-10 level in the subject in the absence of IFNτ administration; ceasing administration of IFNτ for a selected period of time during which the subject's blood IL-10 level remains increased relative to the blood IL-10 level in the subject in the absence of IFNτ administration; and resuming administration of IFNτ when desired, such as when symptoms worsen, is contemplated. The amount of IFNτ sufficient to produce an initial measurable increase in the blood IL-10 level is greater than about 5 x 10⁸ U/day, more preferably 0.5 x 10⁹ U/day or more, still more preferably 1 x 10⁹ U/day or more. The time period during which administration of IFNτ is ceased can vary according to the disease condition, but is readily determined from studies where the IL-10 levels of patients suffering from that disease condition are monitored during treatment with IFNτ and after termination of treatment with IFNτ. Results from such a study can be applied generally to other patients and provide recommended dosing patterns. Alternatively, the time period during which administration of IFNτ is ceased can be tracked for individual patients, by actual monitoring of IL-10 blood levels on a regular basis, e.g., weekly or twice weekly, during a period of non-treatment to determine when treatment should resume, or by a subjective indication of patient perception of symptoms. Treatment resumes when the IL-10 level approaches pre-treatment levels for that particular patient or for a model patient population, or when symptoms worsen for a particular patient being treated.

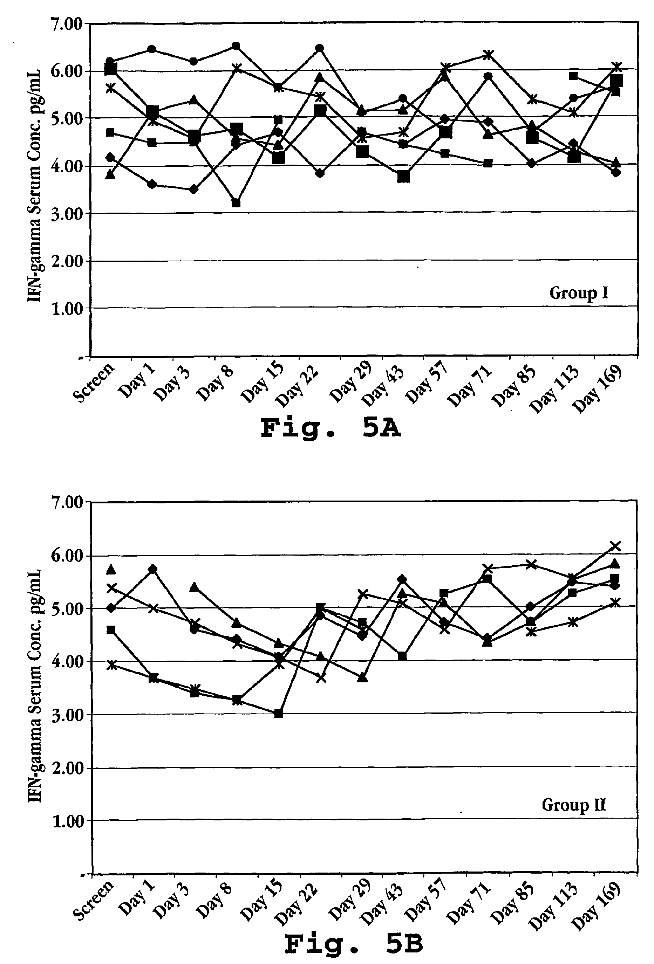

Figs. 5A-5C are graphs showing the IFN-γ serum level, in pg/mL, for the patients in this study. Fig. 5A shows the IFN-γ levels for the six patients in Test Group I treated daily with 0.33 mg IFNτ three times daily. An overall trend of maintaining IFN-γ levels at the baseline level and toward slightly decreasing IFN-γ levels is apparent.

Fig. 5B shows the IFN-γ serum levels for the six patients in Test Group II treated daily with 1.0 mg IFNτ three times daily. A decrease in IFN-γ levels at the early phase of treatment, from about days 3 to 15 is apparent. The levels then returned to baseline and were maintained at about pre-dosing levels for the remainder of the test period.

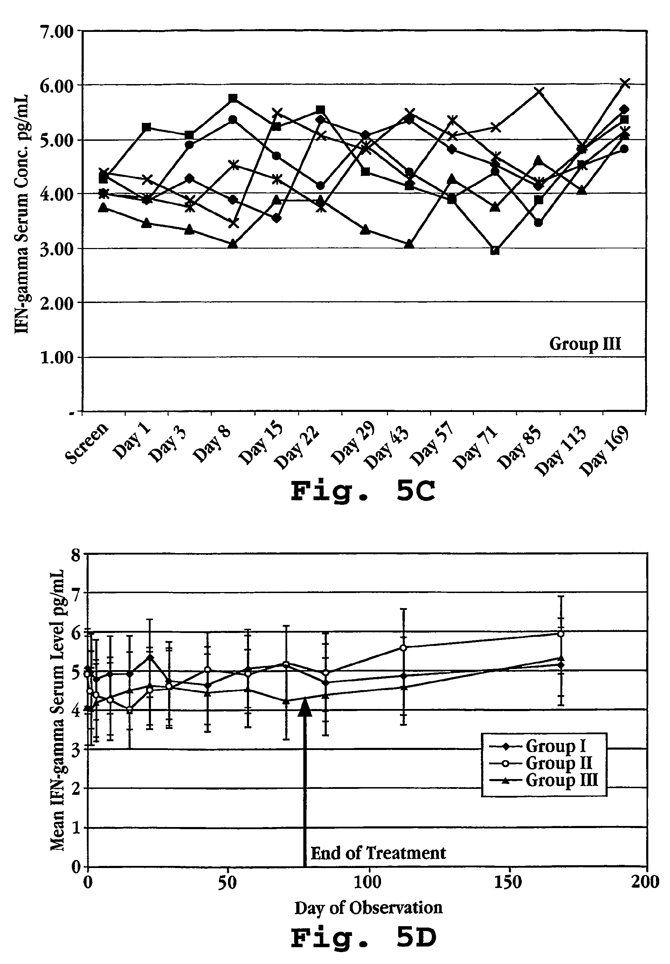

Fig. 5C shows the IFN-γ serum levels for the six patients in Test Group IIII treated daily with 3 mg IFNτ three times daily. While some patients experienced a defined decrease in the IFN-γ level, overall the treatment group appeared to have little change in the level over the treatment period. An increase in the IFN-γ levels upon cessation of dosing is seen, from days 85-169. This suggests that a reduction in levels to some degree was achieved by administration of IFNτ.

Fig. 5D is a summary plot for the test Groups I, II, and III in Figs. 5A-5C, showing the mean serum IFN-γ levels as a function of time for test Group I (diamonds, 0.33 mg three times daily), Group II (circles, 1 mg three times daily), and Group III (triangles, 3 mg three times daily) as a function of time. It is clear that administration of IFNτ either (1) caused no significant change in IFN-γ levels, with the level remaining essentially at the screen, pre-dosing level, or (2) caused a reduction in IFN-γ level from the baseline, pre-dosing level.

Thus, in another aspect, a method of reducing the blood level of IFN-γ in a subject comprises administering IFNτ to the subject in an amount effective to decrease the subject's IFN-γ blood level relative to the IFN-γ blood level in the absence of IFNτ administration. This method finds use particularly for patients taking an agent that causes an elevated IFN-γ level or for patients suffering from a condition that elevates their IFN-γ levels. Thus, also contemplated is a method of preventing an increase in the blood level of IFN-γ in a subject at risk of an elevated IFN-γ blood level due to (i) administration of a therapeutic agent or (ii) a disease condition, by administering IFNτ to the subject in an amount effective to decrease the subject's IFN-γ blood level relative to the IFN-γ blood level in the absence of IFNτ administration. As noted above, treatment of multiple sclerosis with IFN-β causes an increase level of IFN-γ in patients. Co-administration (simultaneous or sequential administration) of IFNτ will assist in maintaining the IFN-γ level at the level prior to treatment. Typically, the amount of IFNτ sufficient to produce such a decrease in subject's IFN-γ blood level is greater than about 5 x 10⁸ U/day, more preferably 0.5 x 10⁹ U/day or more, still more preferably 1 x 10⁹ U/day or more.

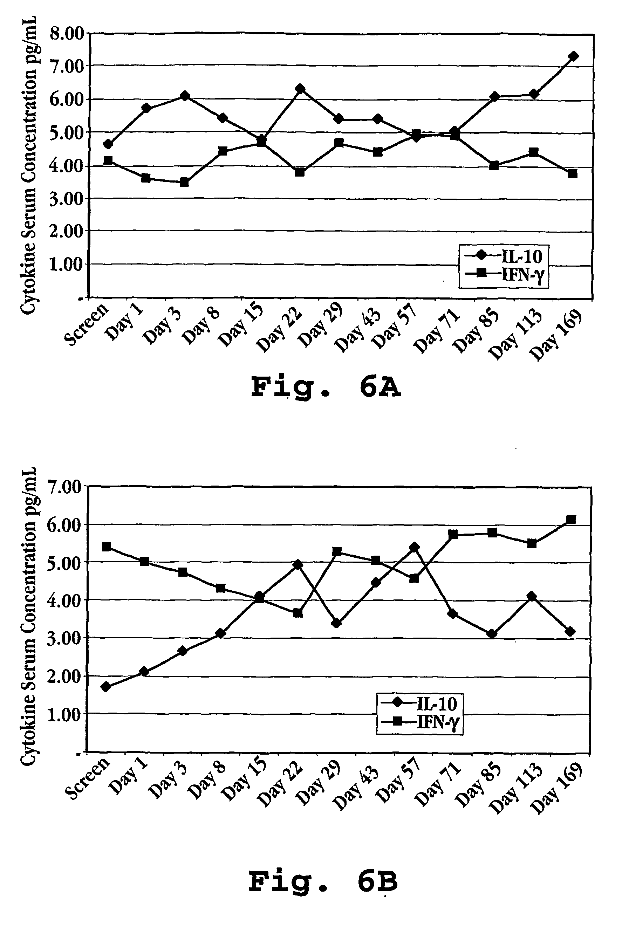

Figs. 6A-6F show IL-10 (diamonds) and IFN-γ (squares) serum concentrations, both in pg/mL, for selected patients from the treatment Groups and III discussed with respect to Figs. 4-5.

Fig. 6A shows the IL-10 (diamonds) and IFN-γ (squares) serum concentrations for patient no. 101 in test group I, treated with 0.33 mg IFNτ three times daily, for a daily dose of 1 mg IFN-τ. The baseline levels of IL-10 and IFN-γ were on average 5.2 pg/mL and 3.9 pg/mL, respectively (averages of values at Screen and at Day 1), to give an initial IL-10/IFN-γ ratio of 1.3. During treatment with IFNτ, the IL-10 / IFN-γ ratio increased to 1.6 at Day 22, with a return to the baseline ratio thereafter, until cessation of dosing at Day 84.

Fig. 6B shows the IL-10 (diamonds) and IFN-γ (squares) serum concentrations for patient no. 205 in test Group II, treated with 1.0 mg IFNτ three times daily, for a daily dose of 3 mg IFNτ. The baseline levels of IL-10 and IFN-γ were on average 3.8 pg/mL and 5.2 pg/mL, respectively (averages of values at Screen and at Day 1), to give an initial IL-1 O/IFN-γ ratio of 0.73. During treatment with IFNτ, the IL-10/IFN-γ ratio approached and reached 1 at Day 15. Thus, treatment with IFNτ resulted in modulation of the IL-10/IFN-γ ratio by increasing the ratio about 25%.

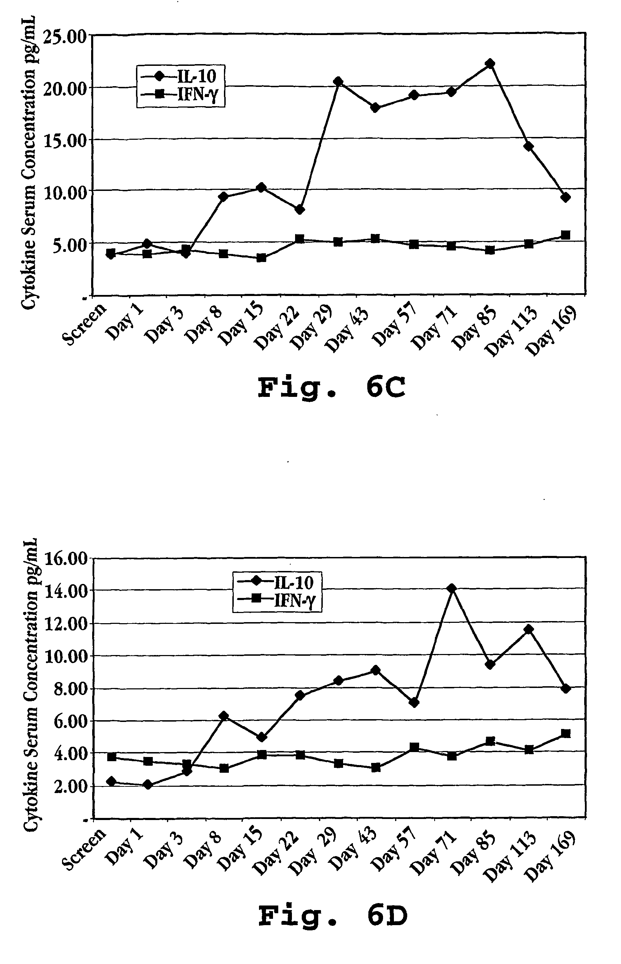

Fig. 6C shows the IL-10 (diamonds) and IFN-γ (squares) serum concentrations for patient no. 301 in test Group III, treated with 3.0 mg IFNτ three times daily, for a daily dose of 9 mg (9 x 10⁸ U) IFNτ. The baseline levels of IL-10 and IFN-γ were on average 4.4 pg/mL and 3.9 pg/mL, respectively (averages of values at Screen and at Day 1), to give an initial IL-10/IFN-γ ratio of about 1.0. During treatment with IFNτ, the IL-10 level increased 4-5 fold, a substantial increase, while the IFN-γ level was maintained at around the initial level of 4-5 pg/mL. Thus, the IL-10/IFN-γ ratio increased upon dosing with IFNτ from about 1.0 to around 4.0, a four-fold increase.

Figs. 6D-6F show the IL-10 (diamonds) and IFN-γ (squares) serum concentrations for patient nos. 303, 304, and 305 in test Group III, treated with 3.0 mg IFNτ three times daily, for a daily dose of 9 mg IFNτ. An analysis of the IL-10/IFN-γ ratios is similar to that for patient no. 301, discussed in Fig. 6C. Specifically, Fig. 6D shows the data for patient no. 303. In this patient, the IL-10 blood concentration increased by about four-fold from baseline level by test Day 43 and increased by more than six-fold by test Day 71. The IFN-γ blood level remained substantially constant. Thus, the IL-10/IFN-γ blood ratio increased from a baseline value of 0.6 to greater than 3, a five-fold increase (500% increase).

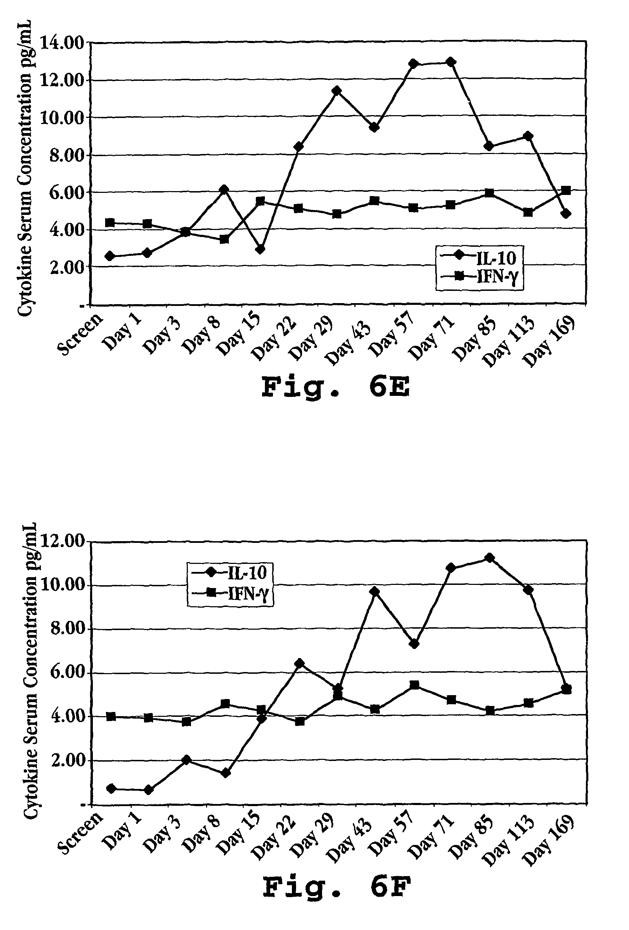

Fig. 6E shows the data for patient no. 304 in Group III. The patient's IL-10 blood level increased 4-5 fold during treatment with IFNτ, whereas the IFN-γ level remained essentially unchanged. Thus, the IL-10/IFN-γ ratio increased from its initial value of 0.6 to 2.6 at Day 71, an increase of more than 400%.

Fig. 6F shows the data for patient no. 305 in Group III. The increasing IL-10 blood level during the treatment period is evident, with an increase from 0.7 pg/mL to more than 9 pg/mL by Day 43. The IFN-γ level remained essentially unchanged, resulting in an IL-10/IFN-γ ratio increase of more than 10 fold.

In summary, the data presented for the patients in Group III show that administration of IFNτ was effective to increase the IL-10/IFN-γ ratio. In particular, the IL-10 blood levels were measurably increased by oral administration of IFNτ, as evidenced by the statistical increase in IL-10 blood concentrations. The IL-10 blood levels were increased by more than 25%, and in this patient population, the increase in IL-10 blood concentrations was considerably greater.

In another study, five patients suffering from a viral infection, hepatitis C, were recruited for treatment with IFNτ. In this study, described in Example 3, the patients were treated with 7.5 mg IFNτ twice daily, for a daily dose of 15 mg IFNτ (1.5 x 10⁹ antiviral units). The first dose was taken in the morning, before breakfast, and the second dose was taken at least three hours after an evening meal. Blood samples were taken at defined intervals over a 113 day test period; dosing of IFN-τ was terminated at test Day 84. The samples were analyzed for IL-10, IL-12, and IFN-γ levels in the serum using commercially available methods.

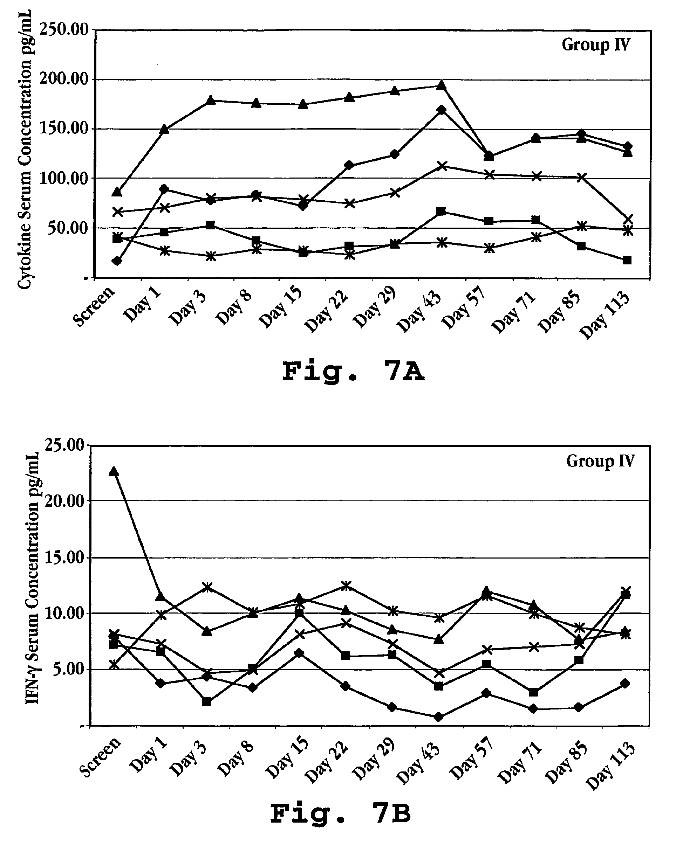

Figs. 7A-7B are graphs showing the IL-10 serum level (Fig. 7A) and the IFN-γ serum level (Fig. 7B), in pg/mL, in the five patients, as a function of time, in days. As seen in Fig. 7A, three of the patients (patients represented by triangles, diamonds, and x's) showed an increased IL-10 level over the period of IFNτ dosing, from Day 1 to Day 84. Fig. 7B shows that all five patients had a reduction in IFN-γ blood levels over the dosing period from Day 1 to Day 84. At the end of dosing, the IFN-γ levels increase, as seen during the period from Day 85 to Day 113.

The blood samples drawn from the patients in this study were also analyzed for IL-12 levels. IL-12 is a pro-inflammatory cytokine and contributes to the pathogenesis of multiple sclerosis. The literature reports that (1) increased production of IL-12 is a key mechanism in the pathogenesis of multiple sclerosis (Filson et al., Clin. Immunol., 106(2):127 (2003); (2) MS patients typically display decreased IL-10 and increased IL-12 levels, and the levels of these cytokines correlate with the disease stage (van Boxel-Dezaire et al., Ann. Neurol., 45:695 (1999)). With respect to viral infections, a high IL-12 level has also been shown to exacerbate bacterial colonization of *B*. *pertussis* (Carter et al., Clin. Exp. Immunol., 135(2):233 (2004)). Thus, it was desirable to monitor the IL-12 levels in the patients enrolled in this study.

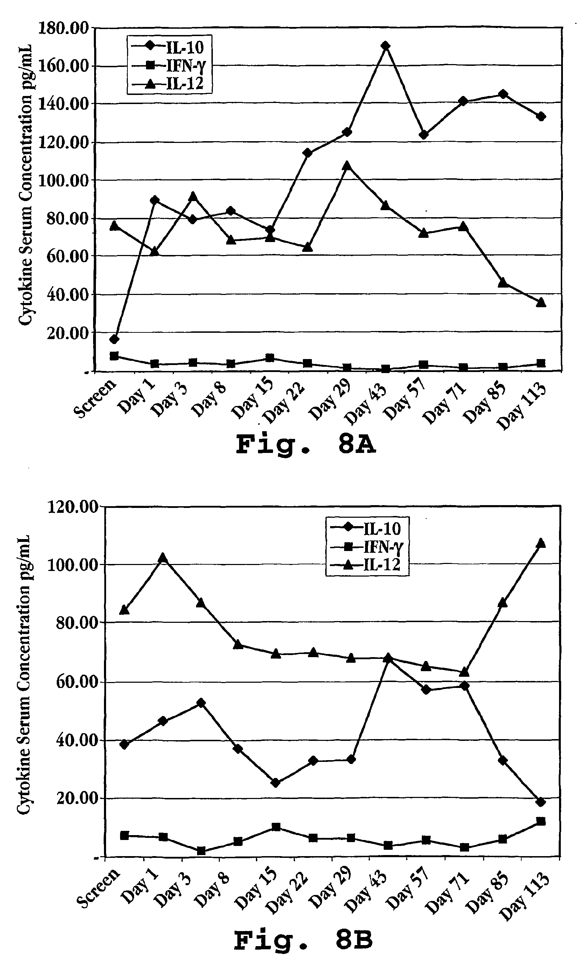

Figs. 8A-8D show the IL-10 (diamonds), IFN-γ (squares), and IL-12 (triangles) serum levels, in pg/mL, for the six patients in this study (Example 3). The actual IL-12 concentrations are 10 times the value shown in Figs. 8A-8D (actual values were divided by 10 to show all data on a single graph).

Fig. 8A shows the data for patient no. 401. As seen, the IL-10 level increased over the treatment period when IFNτ was administered, IFN-γ was unchanged or decreased slightly, and IL-12 fluctuated initially and then was downregulated after about Day 29. The initial IL-10 level was 53.1 pg/mL and the initial, baseline IL-12 was 696 pg/mL, for an IL-10/IL-12 ratio of 0.08. During the treatment period, this ratio increased to between about 0.12-0.18, a 570-1200% increase. The IL-10 level in this patient increased from a baseline value of 53.1 pg/mL to greater than 140 pg/mL, an increase of more than 160% (2.6 fold).

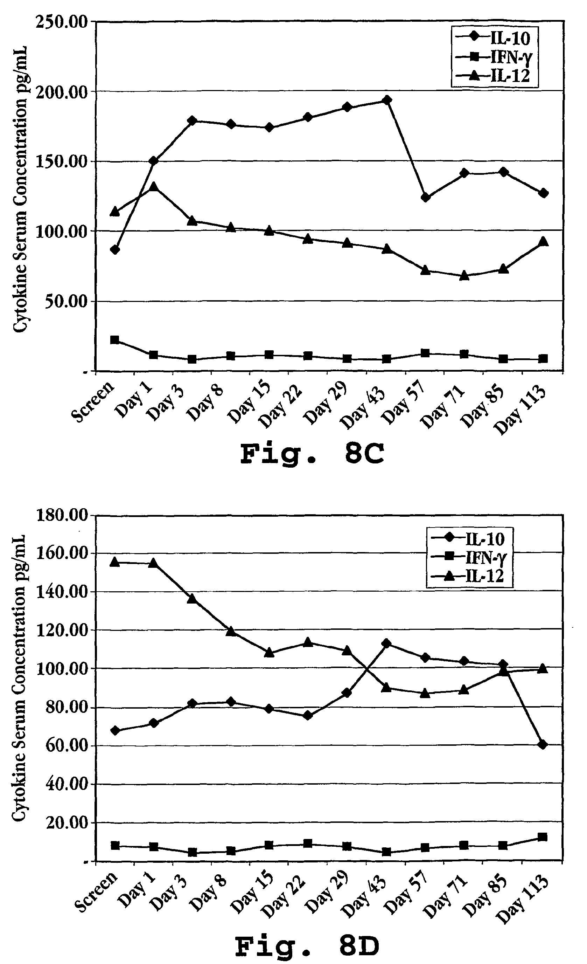

Fig. 8B shows the data for patient no. 402 and Fig. 8C shows the data for patient no. 403. Patient No. 402 had an initial, baseline IL-10 blood level of 42.7 pg/mL (average blood concentrations of Screen and Day 1). The IL-10 blood level peaked at Day 43, when the concentration reached 67 pg/mL, a 56% increase. The IFN-γ blood concentration fluctuated around the baseline level. The IL-12 blood level prior to treatment was 934 pg/mL, for an initial IL-10/IL-12 ratio of 0.046. At Day 43, the IL-10/IL-12 ratio was 0.088, a 90% increase from the baseline ratio.

In Fig. 8C the patient's initial IL-10/IL-12 ratio was 0.10 (IL-10 = 118.5 pg/mL; IL-12 = 1227 pg/mL). This ratio increased over the treatment period, with a ratio value of 0.22 at Day 43, a 2.2 fold increase in IL-10/IL-12 ratio. The patient's IL-10 blood level peaked on Day 43 at a value 63% higher than the baseline level.

Fig. 8D shows the data for patient no. 404. This patient had an initial IL-10 blood level of 69.6 pg/mL and an initial IL-12 level of 1552 pg/mL for an initial lL-1 011L-1 2 ratio of 0.045. During treatment with IFNτ at a dosage of 1.5 x 10⁹ U per day the IL-10 blood level rose to 113 pg/mL on Day 43, an approximately 60% increase. The IL-12 at Day 43 had decreased to 900 pg/mL, providing an II-10/IL-12 ratio at Day 43 of 0.12.

Patient no. 405 in this study had an initial IL-10 blood concentration of 34.9 pg/mL and an initial IL-12 blood concentration of 976 pg/mL (IL-10/IL-12 ratio 0.036) (data not shown). Administration of IFNτ at a dosage of 1.5 x 10⁹ U per day was effective to increase the IL-10/IL-12 ratio to 0.058 at Day 71 of the treatment period, a 60% increase. The IL-10 blood concentration increased 20% from the initial pre-treatment level to the level at Day 71.

Accordingly, a method of increasing the IL-10/IL-12 blood ratio in subjects suffering from an autoimmune disorder comprises administering IFNτ to the subject in an amount effective to produce an initial measurable increase in the subject's blood IL-10 level, relative to the blood IL-10 level in the subject in the absence of IFNτ administration, and a decrease in the subject's IL-12 blood level, relative to the IL-12 level in the absence of IFNτ administration. Also contemplated is a method of inhibiting progression of an autoimmune condition in a subject, by administering IFNτ to the subject in an amount effective to produce an initial measurable increase in the subject's blood IL-10 level, relative to the blood IL-10 level in the subject in the absence of IFNτ administration, and a decrease in the subject's IL-12 blood level, relative to the IL-12 level in the absence of IFNτ administration. In particular, the patients treated with greater than about 5 x 10⁸ U of IFNτ had increased IL-10 blood levels of more than 25%, and in many cases of more than 50%. In the same patients, the IFN-γ blood concentrations were essentially unchanged or were decreased and the IL-12 levels generally decreased.

In summary, the invention contemplates administration of IFNτ orally to a patient in need of treatment, where the initial dose(s) of IFNτ is selected to achieve an increased blood IL-10 level for that particular patient, and/or a decreased or unchanged IFN-γ level, and/or a decreased IL-12 level. The IFNτ is preferably administered in a form that targets the intestinal tract of the patient, rather than the oral cavity. Dosage selection can be made or confirmed, for example, by monitoring blood IL-10 levels e.g, prior to treatment and following initiation of treatment. Alternatively, an effective dose may be predetermined from model patient responses to given doses under different disease conditions. For example, a patient within a given age range and having a specified condition, e.g., a viral infection or an autoimmune condition, may be monitored for changes in blood IL-10 in response to different initial IFNτ levels, to predetermine suitable doses for patients with that age/disease profile, and such dosing guidelines may be supplied to the treating physician. One aspect of the present invention includes an IFNτ therapy kit that includes IFNτ in an oral delivery form suitable for targeting the protein to the intestinal tract, e.g., an enteric coated form of IFN-tau, and product literature or insert that provides guidelines for effective doses, under different patient condition; that is, doses effective to produce a measurable increase in IL-10 blood levels. Preferably, the insert provides a range of doses and predicted initial changes in IL-10 response.

Following the initial administration, or when a dose is reached that produces a measurable increase in blood IL-10 levels (an effective dose), the administration of an effective dose IFNτ is continued, preferably on a daily or several-time-weekly basis, for an extended treatment period. The effective dose that is administered on an extended basis is one effective to produce an initial measurable increase in blood IL-10, independent of the behavior of actual blood IL-10 levels over the extended treatment period, whether or not the continuing effective dose is the same or different from the initial effective dose. Thus, during the treatment period, blood IL-10 levels may remain constant at an elevated level, continue to increase, or even decrease (for example, in response to decreasing levels of infecting virus), even though the patient is continuing to receive an IFNτ dose effective to produce an initial measurable increase in blood IL-10 levels. This effective dose is typically in the range of greater than about 5 x 10⁸ Units per day and up to about 10¹² Units per day; more specifically, the dose is greater than about 5 x 10⁸ Units per day, more preferably about 0.5 x 10⁹ Units or more per day, still more preferably about 1 x 10⁹ Units or more per day. The dose can be adjusted to achieve a desired initial increase in blood IL-10, e.g., between 1.5 and 4 fold normal, untreated levels.

It will be appreciated that for some patients and for some conditions, administration of IFNτ in combination with another therapeutic agent is contemplated, and is described in more detail below. For example, combination of IFNτ with an anti-viral agent may be beneficial in some patients. Similarly, combination of IFNτ with agents used to treat autoimmune conditions will be beneficial in treating the condition. Combination of IFNτ with chemotherapeutic agents in patients suffering from cellular proliferation is also contemplated. More generally, combination of IFNτ with any known pharmaceutical agent is contemplated and exemplary combination treatment regimens are given below. It will be appreciated that "combination" of IFNτ with a second agent intends sequential or simultaneous administration of the two agents, where the sequential administration can be immediate or non-immediate.

### III. Methods of Use

As illustrated by the data described above, administration of IFNτ provides a method for treating in a human subject a disease or condition that responds to an up-regulation of IL-10. A disease or condition "responsive to IL-10 therapy" is one in which the existence, progression, or symptoms of the disease or condition is altered upon administration of IL-10 or an agent that results in an increase in blood IL-10 level. The data above also illustrates that administration of IFNτ finds use in treating subjects having a disease or condition that is responsive to interferon therapy. A condition "responsive to interferon therapy" is one in which the existence, progression, or symptoms of the condition is altered upon administration of an interferon, in particular a type-I interferon, and more particularly, IFNτ. Conditions responsive to treatment with IFNα or IFNβ may also respond to treatment with IFNτ. The methods described herein encompass providing IFNτ, preferably in an orally-administrable dosage form for administration to the stomach and/or intestines, in an amount effective for therapy, as evidenced by, for example, an increase in blood IL-10 level determined from studies on similarly situated patients or on the particular individual patient being treated. The dose of IFNτ sufficient to increase blood IL-10 level can also be effective to cause a reduction in IL-12 blood level, with a reduction or no change in IFN-γ level.

The disease or condition to be treated is typically one that is responsive to cytokine therapy, such as IL-10 therapy. A wide variety of diseases or conditions may be responsive to IL-10 therapy and are therefore amenable to treatment with IFNτ, either alone or in combination with another therapeutic agent. For example, the methods of the invention may be advantageously used to treat neurological disorders, including Alzheimer's disease and autism; fibrotic diseases, including pulmonary fibrosis and liver fibrosis; autoimmune diseases, including anti-phospholipid syndrome, arthritis, allergies, diabetes, such as type I diabetes mellitus; inflammatory bowel disease, including Crohn's disease and ulcerative colitis; psoriasis; multiple sclerosis, uveitis; chronic obstructive pulmonary disease, including chronic bronchitis and emphysema; diseases or conditions characterized by cellular, tissue or organ damage or death due to decreased blood flow and/or oxygen to the cell, tissue or organ, including stroke; and atherosclerosis; and rejection from organ transplant.

### A. Treatment of Immune System Disorders

The method of the present invention is advantageous for treating conditions relating to immune system hypersensitivity. There are four types of immune system hypersensitivity (Clayman, C.B., Ed., AMERICAN MEDICAL ASSOCIATION ENCYCLOPEDIA OF MEDICINE, Random House, New York, N.Y., (1991)). Type I, or immediate/anaphylactic hypersensitivity, is due to mast cell degranulation in response to an allergen (*e.g.*, pollen), and includes asthma, allergic rhinitis (hay fever), urticaria (hives), anaphylactic shock, and other illnesses of an allergic nature. Type II, or autoimmune hypersensitivity, is due to antibodies that are directed against perceived "antigens" on the body's own cells. Type III hypersensitivity is due to the formation of antigen/antibody immune complexes which lodge in various tissues and activate further immune responses, and is responsible for conditions such as serum sickness, allergic alveolitis, and the large swellings that sometimes form after booster vaccinations. Type IV hypersensitivity is due to the release of lymphokines from sensitized T-cells, which results in an inflammatory reaction. Examples include contact dermatitis, the rash of measles, and "allergic" reactions to certain drugs.

The mechanisms by which certain conditions may result in hypersensitivity in some individuals are generally not well understood, but may involve both genetic and extrinsic factors. For example, bacteria, viruses or drugs may play a role in triggering an autoimmune response in an individual who already has a genetic predisposition to the autoimmune disorder. It has been suggested that the incidence of some types of hypersensitivity may be correlated with others. For example, it has been proposed that individuals with certain common allergies are more susceptible to autoimmune disorders.

Autoimmune disorders may be loosely grouped into those primarily restricted to specific organs or tissues and those that affect the entire body. Examples of organ-specific disorders (with the organ affected) include multiple sclerosis (myelin coating on nerve processes), type I diabetes mellitus (pancreas), Hashimotos thyroiditis (thyroid gland), pernicious anemia (stomach), Addison's disease (adrenal glands), myasthenia gravis (acetylcholine receptors at neuromuscular junction), rheumatoid arthritis (joint lining), uveitis (eye), psoriasis (skin), Guillain-Barré Syndrome (nerve cells) and Grave's disease (thyroid). Systemic autoimmune diseases include systemic lupus erythematosus and dermatomyositis. Another autoimmune disorder is Sjogren's syndrome, where white blood cells attack the moisture-producing glands. The hallmark symptoms of Sjogren's syndrome are dry eyes and dry mouth, but it is a systemic disease, affecting many organs.

Other examples of hypersensitivity disorders include asthma, eczema, atopical dermatitis, contact dermatitis, other eczematous dermatitides, seborrheic dermatitis, rhinitis, Lichen planus, Pemplugus, bullous Pemphigoid, Epidermolysis bullosa, uritcaris, angioedemas, vasculitides, erythemas, cutaneous eosinophilias, Alopecia areata, atherosclerosis, primary biliary cirrhosis and nephrotic syndrome. Related diseases include intestinal inflammations, such as Coeliac disease, proctitis, eosinophilia gastroenteritis, mastocytosis, inflammatory bowel disease, Crohn's disease and ulcerative colitis, as well as food-related allergies. Ankylosing spondylitis is another example of an autoimmune, inflammatory disease, where some or all of the joints and bones of the spine fuse together.

Autoimmune diseases particularly amenable for treatment using the methods of the present invention include multiple sclerosis, type I (insulin dependent) diabetes mellitus, lupus erythematosus, amyotrophic lateral sclerosis, Crohn's disease, rheumatoid arthritis, stomatitis, asthma, uveitis, allergies, psoriasis, Ankylosing spondylitis, Myasthenia Gravis, Grave's disease, Hashimoto's thyroiditis, Sjogren's syndrome, and inflammatory bowel disease.

The method of the present invention is used to therapeutically treat and thereby alleviate autoimmune disorders, such as those discussed above. Treatment of an autoimmune disorder is exemplified herein with respect to the treatment of EAE, an animal model for multiple sclerosis. When used to treat an autoimmune disorder, IFNτ is administered at a dose sufficient to achieve the measurable increase in IL-10 during the initial phase(s) of IFNτ administration. Once a desired effective dose is achieved, the patient is treated over an extended period with an effective IFNτ dose, independent of further changes in IL-10 blood levels. The treatment period extends at least over the period of time when the patient is symptomatic. Upon cessation of symptoms associated with the autoimmune condition, the dosage may be adjusted downward or treatment may cease. The patient may be co-treated during the treatment period of IFNτ treatment with another agent, such as a known anti-inflammatory or immune-suppressive agent.

In one embodiment, a method of treating a patient suffering from psoriasis by administering IFNτ is provided. There are two main types of psoriasis: psoriasis vulgaris (plaque psoriasis) and psoriasis pustulosa (pustular psoriasis). The different types of psoriasis can be divided into subgroups according to severity, duration, location on the body and appearance of the lesions. Administration of IFNτ to persons afflicted with plaque psoriasis is described in Example 4 and results in an increase in IL-10 serum levels, resulting in a reduction in the severity of the patches of thickened skin. The severity of skin lesions can be assessed using a variety of scoring tests described in the literature, for example the Physician's Static Global Assessment score, a scaling score, a plaque score, or an erythema score. Treatment with IFNτ continues until a reduction in a clinically suitable assessment score is achieved. Preferably the reduction is of at least about 50%, more preferably at least about 70%, still more preferably of at least about 80%. For example, once a reduction of at least about 70% in the Physician's Static Global Assessment score, relative to the score prior to treatment, is achieved the patient ceases treatment with IFNτ or continues treatment with a different, typically lower, dose of IFNτ. For example, a dose of greater than about 5 x 10⁸ Units/day can be given until the desired assessment score reduction is achieved, and then a maintenance dose of less 5 x 10⁸ Units/day is given, such as 2-3 x 10⁸ Units/day or lower. Thus, the invention provides a method of reducing a psoriasis assessment score in a person afflicted with psoriasis.

The treatment method can also be used to prolong the time between attacks of psoriasis. Psoriasis tends to recur in attacks of varying severity, the attacks being triggered by a number of factors, such as emotional stress, skin damage, and physical illness. It is contemplated that treatment of a psoriasis attack by orally administering IFNτ will improve the number and severity of skin lesions, and also prolong the period of time before recurrence of a subsequent attack. In particular, for patients treated with a maintenance dose of IFNτ between outbreaks and that have an elevated IL-10 blood level during the maintenance period.

Also contemplated is a method of preventing progression of an autoimmune condition, by administering IFNτ in a dose that elevates the IL-10 level in a subject. Also contemplated is a method of inhibiting onset of an autoimmune condition, by administering IFNτ in a dose effective to increase IL-10 serum levels, preferably with no change or a reduction in the IFN-γ level. Also contemplated is a method of treating an autoimmune condition by administering IFNτ in a dose effective to increase the IL-10/IL-12 serum ratio. As discussed above, the dose of IFNτ is preferably provided in an oral form and is typically greater than about 5 × 10⁸ Units/day.

### B. Treatment of Viral Infections

The method of the invention is also used to treat conditions associated with viral infection. The antiviral activity of IFNτ has broad therapeutic applications without the toxic effects that are usually associated with IFNαs, and IFNτ exerts its therapeutic activity without adverse effects on the cells. The relative lack of cytotoxicity of IFN-τ makes it extremely valuable as an *in vivo* therapeutic agent and sets IFNτ apart from most other known antiviral agents and all other known interferons.

Formulations containing IFNτ can be orally-administered to inhibit viral replication. For use in treating a viral infection, the protein is administered at a dose sufficient to achieve a measurable increase in blood IL-10 in the patient. Thereafter, treatment is continued at an effective dose, independent of further changes in blood IL-10 levels, for example, a fall in IL-10 blood levels due to reduction in viral load. Administration of IFNτ is continued until the level of viral infection, as measured for example from a blood viral titer or from clinical observations of symptoms associated with the viral infection, abates.

The viral infection can be due to a RNA virus or a DNA virus. Examples of specific viral diseases which may be treated by orally-administered IFNτ include, but are not limited to, hepatitis A, hepatitis B, non-A, non-B, non-C hepatitis, Epstein-Barr viral infection, HIV infection, herpes virus (EB, CML, herpes simplex), papilloma, poxvirus, picorna virus, adeno virus, rhino virus, HTLV I, HTLV II, and human rotavirus. The patient may be co-treated during the IFNτ treatment period with a second antiviral agent and exemplary agents are given below.

### C. Method for Treating Conditions of Cellular Proliferation

In another embodiment, treatment of conditions characterized by hyperproliferation are provided. IFNτ exhibits potent anti-cellular proliferation activity (Pontzer, C.H. et al., Cancer Res., 51:5304-5307, (1991), and thus acts to inhibit, prevent, or slow uncontrolled cell growth when orally administered.

Examples of cell proliferation disorders in humans which may be treated by orally-administered IFNτ include, but are not limited to, lung large cell carcinoma, colon adenocarcinoma, skin cancer (basal cell carcinoma and malignant melanoma), renal adenocarcinoma, promyelocytic leukemia, T cell lymphoma, cutaneous T cell lymphoma, breast adenocarcinoma, steroid sensitive tumors, hairy cell leukemia, Kaposi's Sarcoma, chronic myelogenous leukemia, multiple myeloma, superficial bladder cancer, ovarian cancer, and glioma.

For use in treating a cell-proliferation condition, IFNτ is administered at a dose sufficient to achieve an initial measurable increase in blood IL-10 in the patient. Thereafter, treatment is continued at an effective dose, independent of further changes in blood IL-10 levels, for example, a fall in IL-10 blood levels due to a reduction in cancer cells in the body. Administration of IFNτ at an effective dose is continued until a desired level of regression is observed, as measured for example, by tumor size or extent of cancer cells in particular tissues.

The patient may be co-treated during the IFNτ treatment period with a second anticancer agent, e.g., cis-platin, doxorubicin, or taxol and the other agents given below.

### D. Combination Treatment Regimens

Combination of IFNτ with one or more other agents is also contemplated. Various combination treatment regimens are contemplated, including oral administration of IFNτ in combination with (i) a second therapeutic agent, (ii) an agent effective to stabilize or protect IFNτ from loss of activity after oral administration; and (iii) a second therapeutic agent and a stabilizing agent. These various treatment methods are discussed below.

In one embodiment, oral administration of IFNτ is combined with administration of a second therapeutic agent. In this embodiment, IFNτ is administered at a daily dose of greater than about 5 x 10⁸ U and the second agent is administered as prescribed by the attending medical caregiver, usually in accord with the recommended dosing schedule for the agent. The second agent, which is selected based on the particular disease or condition, can be administered by any suitable route of administration, prior to, concurrent with, or subsequent to oral administration of IFNτ.

Treatment of autoimmune conditions is a preferred treatment method, and an exemplary combination regimen is includes oral administration of IFNτ in combination with administration of an antigen against which an autoimmune response is directed. Examples include co-administration of myelin basic protein and IFNτ to treat multiple sclerosis; collagen and IFNτ to treat rheumatoid arthritis, and acetylcholine receptor polypeptides and IFNτ to treat myasthenia gravis.

Furthermore, IFNτ may be orally administered with known immunosuppressants, such as steroids, to treat autoimmune diseases such as multiple sclerosis. The immunosuppressants may act synergistically with IFNτ and result in a more effective treatment that could be obtained with an equivalent dose of IFNτ or the immunosuppressant alone. More generally, IFNτ administered in combination with drugs, i.e., therapeutic agents, for treatment of autoimmune conditions is contemplated, where representative drugs include, but are not limited to azathioprine, cyclophosphamide, corticosteroids (prednisone, prednisolone, others), cyclosporine, mycophenolate mofetil, antithymocyte globulin, muromonab-CD3 monoclonal antibody, mercaptopurine, mitoxantrone, glatiramer acetate (Copaxone), interferon-beta (Avonex^{™}, Betaseron^{™}, Ribif^{™}), daclizumab, methotrexate, sirolimus, tacrolimus, and others.

In one embodiment, IFNτ and a selective adhesion molecule inhibitor, particularly, an integrin antagonist, are administered in combination for treatment of, for example, an autoimmune condition such as Crohn's disease or multiple sclerosis. The integrins are a family of cell-surface adhesion molecues that mediate cell-cell and cell-extracellular matrix interactions. They consist of transmembrane α and 13 subunits that associate as heterodimers to form functional molecules. There are currently 18 α and eight β subunits identified that form more than 24 different integrin receptors (Sandborn, W.J. et al., Am. J. Gastroenterol., 98(11):2372 (2003)). Alpha-4 integrin (α4 integrin) is expressed on lymphocytes, monocytes, eosinophils, and basophils and at low levels on neutrophils. α4 integrin can pair with either of two β subunits, β1 and β7. α4β1 integrin is also referred to as very late antigen 4 (VLA-4) and binds to either the vascular cell adhesion molecule-1 (VCAM-1) present on blood-brain barrier endothelium or the connecting segment-1 (CS-1) containing fibronectin. α4β1 integrin is an important mediator of cell adhesion and transendothelial migration, as well as a regulator of immune-cell activation within inflamed tissue. α4β7, also known as lamina propria-associated molecule-1 (LPAM-1) binds to the mucosal vascular addressin cell adhesion molecule-1 expressed on gut-associated lymphoid tissue.

Natalizumab (Antegren^{®}, Elan Pharmaceuticals and Biogen) is a humanized monoclonal antibody with activity as an α4β1 integrin antagonist (Elices, M.J., Curr. Opin. Investig. Drugs, 4(11):1354 (2003); WO 95/19790). The antibody attaches to α4 integrin subunits on the cell surfaces and inhibits α4-integrin-mediated adhesion and migration of leukocytes. Natalizumab has been evaluated in clinical trials involving patients with inflammatory bowel disease and multiple sclerosis (Sandborn, W.J. et al., Am. J. Gastroenterol., 98(11):2372 (2003); Miller, D. H. et al., New Engl. J. Med., 348(1):15 (2003); Elices, M.J., Curr. Opin. Investig. Drugs, 4(11):1354 (2003)). Short-term treatment with natalizumab in patients with relapsing-remitting and secondary progressive multiple sclerosis reduced the number of new active lesions, as assessed by magnetic resonance imaging (Sandborn *et al., supra).* Fewer inflammatory brain lesions and fewer relapses were observed over a six-month period in patients with relapsing multiple sclerosis (Miller *et al., supra*)*.*

Accordingly, a combined treatment regimen comprising administering IFNτ orally to a patient suffering from an autoimmune disorder and additionally administering natalizumab is contemplated, as exemplified in Example 5. The dosing regimen will vary according to the patient, the condition and its severity, along with other factors. Exemplary dosing regimens include daily oral administration of IFNτ at a daily dose of greater than about 5 x 10⁸ U and once-per-month (every 28 days) intravenous administration of natalizumab at a dose of about 3 mg/kg. The different mechanism of actions of IFNτ and natalizumab in treating autoimmune conditions would be expected to provide enhanced therapeutic results.

In another embodiment, a treatment regiment for psoriasis is provided, where IFNτ and a second agent useful for treating psoriasis are administered. Therapeutic agents that may be combined with IFNτ treatment of psoriasis may include topical or systemic agents. Suitable therapeutic agents include immunosuppressants, including monoclonal antibody against TNF-alpha factor, the statins and glatiramer acetate previously described herein, collagen, retinoids, anthralin, calpotriene, coal tar, salicylic acid, clobetasol propionate, alefacept, hydroxyurea, and etanercept.

In another embodiment, IFNτ and a statin are administered in combination for treatment of, for example, an autoimmune condition, such as multiple sclerosis. Statins are a group of pharmacologic agents which are 3-hydroxy-3-methylglutaryl enzyme-CoA reductase inhibitors (also referred to as Hmg-CoA reductase inhibitors, HRI's, and mevalonic acid biosynthesis inhibitors). Statins alter serum lipid (cholesterol) levels by blocking the enzyme in the liver that produces cholesterol. Inflammation in the walls of arteries also plays a role in atherosclerosis, and statins also play a role in reducing inflammation.

There are several statins available for prescription, including lovastatin (Mevacor^{®}), simvastatin (Zocor^{®}; velostatin), pravastatin (Pravachol^{®}), fluvastatin (Lescol^{®}), atorvastatin (Lipitor^{®}), rosuvastatin (Crestor^{®}), and cerivastatin (Bayco^{®}). The drugs are typically given orally in a daily dose of between about 5-40 mg. Other statins include itavastatin, mevastatin, etc.

A combined treatment regimen comprising administering IFNτ orally to a patient suffering from a condition caused by inflammation, such as rheumatoid arthritis or multiple sclerosis, and additionally administering a statin is contemplated. The dosing regimen will vary according to the patient, the condition and its severity, along with other factors. Exemplary dosing regimens include daily oral administration of IFNτ at a daily dose of greater than about 5 x 10⁸ U and a daily dose, generally given orally, of a statin of about 5-50 mg.

In another embodiment, IFNτ and the immunosuppressive agent mycophenolate mofetil (Cellcept^{®}, Hoffman-La Roche AG, Grenzach, Germany) are administered in combination to reduce the risk of and/or prevent organ rejection. This embodiment is exemplified in Example 6. Mycophenolate mofetil is an inosine monophosphate dehydrognase inhibitor and inhibits purine synthesis, especially in T cells and B cells (Groetzner J., et al., Transplantation, 77(4):568 (2004)). The compound has been shown to prolong survival of allogeneic transplants in animal models (kidney, heart, liver, intestine, limb, small bowel, pancreatic islets, bone marrow, etc.). The drug has also been shown to reverse ongoing acute rejection in the canine renal and rat cardiac allograft models, and to inhibit proliferative arteriopathy in aortic and cardiac allografts in rats and in primate cardiac xenografts. Mycophenolate mofetil is absorbed following oral administration and hydrolized to mycophenolic acid, its active metabolite. Mycophenolic acid is a potent, selective, uncompetitive, and reversible inhibitor of inosine monophosphate dehydrogenase and inibits the de novo pathway of guanosine nucleotide synthesis without incorporation into DNA. It has a potent cytostatic effect on T- and B-lymphocytes, which are dependent for proliferation on de novo synthesis of purines.

Mycophenolate mofetil is available for oral administration in the form of capsules, tablets, and as a power for oral suspension or for intravenous infusion. A dose of 1 gram administered orally or intravenously twice a day (daily dose of 2 g) is recommended for use in renal transplant patients. For cardiac transplant patients, a dose of 1.5 grams twice daily intravenously or orally is recommended.

A combined treatment regimen is contemplated, comprised of orally administering IFNτ and administering mycophenolate mofetil to an organ transplant patient, to reduce the risk of organ rejection and/or prevent organ rejection. It will be appreciated that the mycophenolate mofetil can be a free base or salt form. The dosing regimen will vary according to the patient, the type of organ transplantation, along with other factors. Exemplary dosing regimens include daily oral administration of IFNτ at a daily dose of greater than about 5 x 10⁸ U and a daily dose, given orally or intravenously, of mycophenolate mofetil at a dose of up to 3 grams per day.

In treating a cancer or viral disease other than hepatitis C, IFNτ may be administered in conjunction with, e.g., a therapeutically effective amount of one or more chemotherapy agents. Exemplary types of agents for treatment of cellular proliferative conditions include, but are not limited to, nitrogen mustards, ethylenimines, methylmelamines, alkyl-sulfonates, nitrosoureas, triazenes, folic acid anlogs, pyrimidine analogs, purine analogs, vinca alkaloids, epipodphyllotoxins, antibiotics, enzymes, biological response modifiers (e.g., cytokines), platinum coordination complexes, anthracenedione, substituted ureas, methylhydrazine derivatives, adrenocortical suppressants, progestins, estrogens, antiestrogens, androgens, antiandrogens, and gonadotropin releasing hormone anlogs. Representative drugs include, but are not limited to mechlorethamine, cyclophosphamide, ifosfamide, melphalan, chlorambucil, hexamethylmelamine, thiotepa, busulfan, carmustine, lomustine, semustine, streptozocin, dacarbazine, methotrexate, fluorouracil, floxuridine, cytarabine, mercaptopurine, thioguanine, pentostatin, vinblastine, vincristine, etoposide, teniposide, dactinomycin, daunorubicin, doxorubicin, bleomycin, plicamycin, mitomycin, asparaginase, interferon-alpha, cisplatin, carboplatin, mitoxantrone, hydroxyurea, procarbazine, mitotane, aminoglyethimide, prednisone, hydroxyprogesterone caproate, medroxyprogesterone acetate, megestrol acetate, diethylstilbestrol, ethinyl estradiol, tamoxifen, testosterone propionate, fluoxymesterone, flutamide, leuprolide, zidovudine (AZT), leucovorin, melphalan, cyclophosphamide, dacarbazine, dipyridamole, and others.

Exemplary agents for co-administration with IFNτ for treatment of a viral infection include, but are not limited to, antiherpesvirus agents, antiretroviral agents, and antiviral agents. Representative drugs include acyclovir, famciclovir, foscarnet, ganciclovir, idoxuridine, sorivudine, trifluridine, valacyclovir, vidarabine, didanosine, stavudine, zalcitabine, zidovudine, amantadine, interferon-alpha, ribavirin, rimantadine, lamivudine, protease inhibitors, acyclic nucleoside phosphonates, and others.

Alzheimer's disease may be benefited by administration of IFNτ in combination with amyloid beta, neurotransmission enhancing drugs, including anticholinesterase inhibitors such as, for example, tacrine, donepezil, rivastigamine, metrifonate, epastigimine, nicotine, pyridostigimine, neostigimine, physostigimine, ambenomium chloride and Gingko biloba; substances that increase brain catecholamines and/or reduce oxidative damage to neurons, including selegiline and vitamin E; non-steroidal drugs having anti-inflammatory properties, including statins (the statins also have immunosuppressant properties), such as lovastatin, simvastatin, pravastatin, fluvastatin, atorvastatin, rosuvastatin, and cerivastatin; aminoarylcarboxylic acid derivatives, such as enfenamic acid, etofenamate, flufenamic acid, isonixin, meclofenamic acid and tolfenamic acid; arylacetic acid derivatives, such as aceclofenac, acemetacin, bromfenac, clopirac, etodolac, fentiazac, indomethacin, oxametacine, and tropesin; arylbutyric acid derivatives, such as bumadizon, butibufen, fenbufen and xenbucin; arylcarboxylic acid derivatives, such as clidanac, ketorolac, and tinoridine; arylpropionic acid derivatives, such as alminoprofen, carprofen, fenoprofen, ibuprofen, indoprofen, ketoprofen, naproxen, pirprofen and zaltoprofen; pyrazoles, such as difenamizole and epirizole; pyrazolones, such as apazone, benzpiperylon, feprazone, oxyphenbutazone, pipebuzone, ramifenazone, and thiazolinobutazone; salicylic acid derivatives, such as acetaminosalol, aspirin, balsalazide, diflunisal, gentisic acid, imidazole salicylate, olsalazine, parsalmide, salicylsulfuric acid, sodium salicylate and sulfasalzine; and thiazinecarboxyamides, such as ampiroxicam, droxicam, isoxicam, lornoxicam, piroxicam and tenoxicam; and cholinergic agonists, including xanomeline, milameline, AF 102B, or memric. Example 7 describes a murine model of Alzheimer's Disease and illustrates how treatment of humans suffering from Alzheimer's Disease treated with IFNτ alone or in combination with one or more second therapeutic agents can be evaluated to determine efficacy of treatment.

Therapeutic agents that may be combined with IFNτ treatment of liver fibrosis may be selected based on the cause of the fibrosis. For example, where liver fibrosis is caused by a hepatitis virus infection, such as hepatitis C infection, various anti-viral agents may be utilized, including monoclonal antibodies, such as palivizumab; peptidomimetics, such as amprenavir, indinavir, lopinavir, nelfinavir, ritonavir, and saquinavir; polynucleotides, such as ampligen and fomivirsen; purines/pyrimidones, such as abacavir, acyclovir, adefovir, cidofovir, cytarabine, didanosine, dideoxyadenosine, edoxudine, emtricitabine, famciclovir, floxuridine, ganciclovir, idoxuridine, inosine pranobex, lamivudine, MADU, penciclovir, sorivudine, stavudine, tenofovir, trifluridine, valacyclovir, valganciclovir, vidarabine, zalcitabine, and zidovudine; and sialic acid analogs, such as oseltamivir and zanamivir; and interferon-alpha, interferon-beta and interferon-gamma. Example 8 describes a model of liver fibrosis and illustrates how treatment of humans suffering from liver fibrosis treated with IFNτ alone or in combination with one or more second therapeutic agents can be evaluated to determine efficacy of treatment.

Where liver fibrosis is secondary to cancer, various anti-cellular proliferative agents may be utilized, including nitrogen mustards, ethyleneimines, methylmelamines, alkyl sulfonates, nitrosoureas, triazenes, folic acid analogs, pyrimidine analogs, purine analogs, vinca alkaloids, epipodophyllotoxins, antibiotics, enzymes, biological response modifiers (e.g., cytokines), platinum coordination complexes, anthracenedione, substituted ureas, methylhydrazine derivatives, adrenocortical suppressants, progestins, estrogens, antiestrogens, androgens, antiandrogens, and gonadotropin releasing hormone analogs. Representative drugs include, but are not limited to mechlorethamine, cyclophosphamide, ifosfamide, melphalan, chlorambucil, hexamethylmelamine, thiotepa, busulfan, carmustine, lomustine, semustine, streptozocin, dacarbazine, methotrexate, fluorouracil, floxuridine, cytarabine, mercaptopurine, thioguanine, pentostatin, vinblastine, vincristine, etoposide, teniposide, dactinomycin, daunorubicin, doxorubicin, bleomycin, plicamycin, mitomycin, asparaginase, interferon-alpha, cisplatin, carboplatin, mitoxantrone, hydroxyurea, procarbazine, mitotane, aminoglyethimide, prednisone, hydroxyprogesterone caproate, medroxyprogesterone acetate, megestrol acetate, diethylstilbestrol, ethinyl estradiol, tamoxifen, testosterone propionate, fluoxymesterone, flutamide, leuprolide, zidovudine (AZT), leucovorin, melphalan, dacarbazine, dipyridamole, and others. The skilled artisan can select other appropriate therapeutic agents where liver fibrosis is secondary to some other disease or condition.

Therapeutic agents that may be combined with IFNτ treatment of pulmonary fibrosis include immunosuppressants, such as alemtuzumab, azathioprine, basiliximab, brequinar, cyclophosphamide, corticosteroids (prednisone, prednisolone, triamcinolone, betamethasone, dexamethasone, etc.), cyclosporine, gusperimus, 6-mercaptopurine, mizoribine, pimecrolimus, rapamycin, mycophenolate mofetil, antithymocyte globulin, muromonab-CD3 monoclonal antibody, mercaptopurine, mitoxantrone, glatiramer acetate (Copaxone^{®}), interferon-gamma, interferon-beta (Avonex^{™}, Betaseron^{™}, Ribif^{™}), daclizumab, methotrexate, sirolimus, tacrolimus, and others. Galtiramer acetate is a synthetic basic random copolymer composed of tyrosine, glutamate, alanine and lysine and has, for example, anti-inflammatory properties. When administered with IFNτ, glatiramer acetate could induce higher levels of IL-10 and TGF-beta (Soos, J.M. et al., J. Immunol., 169:2231 (2002)). In certain cases where the underlying disease which caused the pulmonary fibrosis is known, such as rheumatoid arthritis, and tuberculosis, therapeutic agents known to treat such diseases or conditions may be utilized. For example, antibiotic treatment may be utilized in tuberculosis; anti-inflammatory agents may be used in rheumatoid arthritis, including the non-steroidal anti-inflammatory drugs previously discussed herein; Cox-2 inhibitors, including valdecoxib, lumiracoxib and celecoxib; as well as immunosuppressants previously described herein, including methotrexate to take advantage of its, for example, immunosuppressant properties. Example 9 describes a model of pulmonary fibrosis and illustrates how treatment of humans suffering from pulmonary fibrosis treated with IFNτ alone or in combination with one or more second therapeutic agents can be evaluated to determine efficacy of treatment.

Therapeutic agents that may be combined with IFNτ treatment of anti-phospholipid syndrome include anti-coagulants, such as unfractionated heparin, lovenox, acetylsalicylic acid, and coumadin; anti-malarials, including hydroxychloroquine, mefloquine, primaquine, proguanil, and doxycycline; corticosteroids, including prednisone, prednisolone, triamcinolone, betamethasone, and dexamethasone; and immunomodulatory agents, including intravenous immune globulins. Example 10 describes a model of anti-phospholipid syndrome and illustrates how treatment of humans suffering from anti-phospholipid syndrome treated with IFNτ alone or in combination with one or more second therapeutic agents can be evaluated to determine efficacy of treatment.

Therapeutic agents that may be combined with IFNτ treatment of stroke include cerebral vasodilators, such as bencyclane, ciclonicate, cinnarizine, cyclandelate, diisopropylamine dichloroacetate, eburnamonine, fasudil, fenoxedil, flunarizine, ibudilast, ifenprodil, lomerizine, nafronyl, nicametate, nicergoline, nimodipine, papaverine, pentifylline, vincamine, vinpocetine and viquidil; coronary vasodilators, such as amotriphene, benfurodil hemisuccinate, benziodarone, chloracizine, chromonar, clobenfurol, clonitrate, cloricromen, dilazep, etafenone, fendiline, hexobendine, mannitol hexanitrate, nitroglycerin, pentrinitrol, perhexiline, propatyl nitrate, trapidil, trimetazidine and visnadine; and tissue plasminogen activator. Other suitable agents include auto-antigens, such as myelin oligodendrocyte glycoprotein (MOG) or peptides thereof, statins, and glatiramer acetate. The MOG is preferably a mammalian MOG, such as a human MOG. The nucleotide and amino acid sequence (SEQ ID NO:5) of human MOG is known in the art and can be found, for example, in the NIH Genbank database as accession number BC035938. Suitable MOG peptides include those which range from about 10 to about 150 amino acids long, typically about 30 to about 100 amino acids long and may further be about 10 to 50 amino acids long, and are effective in treating stroke patients. As one example, a peptide from amino acid 35 to amino acid 55 of MOG may be used. As another example, a peptide of MOG from amino acid 37 to amino acid 46 may be used. It is realized that MOG and peptides thereof from other mammalian species, including, for example, ovine, bovine and porcine, that function in treating stroke may be used in the methods herein. Therefore, applicable MOGs include those that have amino acid sequences that have at least about 70% identity, further at least about 80% identity, and further at least about 90% identity to the amino acid sequence of human MOG. Percent identity may be determined, for example, by comparing sequence information using the advanced BLAST computer program, including version 2.2.9, available from the National Institutes of Health, or may be determined using other similar computer programs. The BLAST program is based on the alignment method of Karlin and Altschul. Proc. Natl. Acad. Sci. USA 87:2264-2268 (1990) and as discussed in Altschul, et al., J. Mol. Biol. 215:403-410 (1990); Karlin And Altschul, Proc. Natl. Acad. Sci. USA 90:5873-5877 (1993); and Altschul et al., Nucleic Acids Res. 25:3389-3402 (1997). Accordingly, administration of IFNτ in combination with one or more of the above-recited therapeutic agents is included in the scope of the methods of the present invention. Example 11 describes a stroke model and illustrates how treatment of humans who have suffered from a stroke and treated with IFNτ alone or in combination with one or more second therapeutic agents can be evaluated to determine efficacy of treatment.

Therapeutic agents that may be combined with IFNτ treatment of optic neuritis include statins, and the immunosuppressants previously described herein, including statins, corticosteroids and glatiramer acetate. Example 12 describes a model of optic neuritis and illustrates how treatment of humans suffering from optic neuritis treated with IFNτ alone or in combination with one or more second therapeutic agents can be evaluated to determine efficacy of treatment.

Therapeutic agents that may be combined with IFNτ treatment of chronic obstructive pulmonary disease include bronchodilators and corticosteroids. Suitable bronchodilators include, for example, ephedrine derivatives, such as albuterol, bambuterol, bitolterol, carbuterol, clenbuterol, dioxethedrine, ephedrine, fenoterol, isoetharine, mabuterol, pirbuterol, protokylol, rimiterol, salmeterol, soterenol, and tulobuterol; quaternary ammonium compounds, such as flutropium bromide, ipratropium bromide, oxitropium bromide and titropium bromide; and xanthine derivatives, such as acefylline, ambuphylline, aminophylline, bamifylline, doxofylline, etofylline, guathylline, theobromine and theophylline. Other suitable agents include statins, glatiramer acetate and corticosteroids previously described herein. Example 13 describes a model of chronic obstructive pulmonary disease and illustrates how treatment of humans suffering from chronic obstructive pulmonary disease treated with IFNτ alone or in combination with one or more second therapeutic agents can be evaluated to determine efficacy of treatment.

Therapeutic agents that may be combined with IFNτ treatment of autism include serotonin uptake inhibitors, including femoxetine, fluoxetine, fluvoxamine, indalpine, indeloxazine hydrochloride, milnacipran, paroxetine, sertraline and zimeldine; anti-psychotic drugs, including the benzamides, such as amisulpride, nemonapride, remoxipride, sulpiride, and sultopride; the benzisoxazoles, such as iloperidone and risperidone; the butyrophenones, such as benperidol, bromperidol, droperidol, fluanisone, haloperidol, melperone, moperone, pipamperon, spiperone, timiperone, and trifluperidol; the phenothiazines, such as acetophenazine, butaperazine, carphenazine, chlorpromazine, clospirazine, cyamemazine, dixyrazine, fluphenazine, mepazine, and mesoridazine; and the thioxanthenes, such as chlorprothixine, clopenthixol, flupentioxl and thiothixen; and drugs having anti-inflammatory properties, including statins, glatiramer acetate and corticosteroids previously described herein. Example 14 describes a model of autism and illustrates how treatment of humans suffering from autism treated with IFNτ alone or in combination with one or more second therapeutic agents can be evaluated to determine efficacy of treatment.

Therapeutic agents that may be combined with IFNτ treatment of diabetes include insulin, beta-cell associated auto-antigens, and heat shock proteins, including, for example, heat shock proteins 10, 22, 27, 60, 65, 70 and 90. The heat shock proteins are preferably mammalian heat shock proteins, such as human heat shock proteins, many of which may be purchased commercially or otherwise may be obtained by methods known to the skilled artisan.

Therapeutic agents that may be combined with IFNτ treatment of atherosclerosis include the immunosuppressant agents previously described herein heat shock proteins, including, for example, heat shock proteins 10, 22, 27, 60, 65, 70 and 90. The heat shock proteins are preferably mammalian heat shock proteins, such as human heat shock proteins. Other suitable therapeutic agents include, for example, anti-platelet agents, such as aspirin, clopidogrel, dypridamole, ticlopidine, and sulfinpoyrazone; bile acid sequestrants, including colestipol hydrochloride, and cholestryamine resin; fibrinates (fibric acid derivatives), including gemfibrozil, fenofibrate, and clofibrate; 3-hydroxy-3-methylglutaryl coenzyme A (HMG-CoA) reductase inhibitors, such as lovastatin (Mevacor^{®}), simvastatin (Zocor^{®}; velostatin), pravastatin (Pravachol^{®}), fluvastatin (Lescol^{®}), atorvastatin (Lipitor^{®}), rosuvastatin (Crestor^{®}), and cerivastatin (Baycol^{®}); an anti-hypertensive in cases of accompanying high blood pressure, including diuretics, such as organomercurials, including chlormerodrin, meralluride, mercaptomerin sodium and mersalyl; purines, including pamabrom, protheobromine, and theobromine; steroids, such as canrenone, oleandrin, and spironolactone, sulfonamide derivatives, such as acetazolamide, ambuside, azosemide, bumetanide, butazolamide, clofenamide, clopamide, disulfamide, furosemide, and torsemide; and thiazides, such as althiazide, benzthiazide, chlorothiazide, cyclopenthiazide, ethiazide, hydrochlorothiazide, indapamide, metolazone and teclothiazide; angiotensin converting enzyme inhibitors, such as alacepril, benazepril, captopril, ceronapril, cilazapril, delapril, enalapril, lisinopril, moveltipril, quinapril, ramipril and temocarpril; alpha-adrenergic agonists, such as adrafinil, adrenalone, amidephrine, apraclonidine, burdralazine, clonidine, cyclopentamine, ephedrine, fenoxazoline, mataraminol, methoxamine, midodrine, modafinil, octodrine, oxymetazline, pholedrine, rilmenidine and tyramine; beta-adrenergic blockers, such as acebutolol, alprenolol, amosulalol, arotinolol, atenolol, befunolol, betaxolol, bevantolol, bunitrolol, butidrine hydrochloride, carazolol, carteolol, dilevealol, indenolol, mepindolol, moprolol, nadoxolol, penbutolol, pindolol, propranolol, sulfinalol, tertatolol, and xibenolol; calcium channel blockers, including the arylalkylamines, such as bepridil, clentiazem, diltiazem, fendiline, gallopamil, mibefradil, prenylamine, semotiadil, terodiline, and verapamil; dihydropyridine derivatives, such as amlodipine, aranidipine, barnidipin, benidipin, manidipiine, nilvadipine and nitrendipine; and piperazine derivatives, such as cinnarizine, dotarizine, flunarizine, lidoflazine, and lomerizine; the immunosuppressants previously described herein and drugs having anti-inflammatory properties, including statins, glatiramer acetate and corticosteroids previously described herein. Example 15 describes a model of atherosclerosis and illustrates how treatment of humans suffering from atherosclerosis treated with IFNτ alone or in combination with one or more second therapeutic agents can be evaluated to determine efficacy of treatment.

Therapeutic agents that may be combined with IFNτ treatment of allergies include allergy-inducing agents, including pollen, ovalbumin, food ingredients, such as milk, wheat, animal meat, including bovine, porcine or ovine-derived meat; vegetables, including carrots, and cruciferous vegetables such as broccoli, cabbage, cauliflower, brussels sprouts, and turnips; nuts, including peanuts, pistachios, cashews; and mites; endoplasmic reticulum degrading agents, including Der1 (from, for example, Saccharomyces cerevisiae, the sequence of which has been cloned described in Knop, M., et al., Embo J. 15(4) 753-763 (1996); glatiramer acetate and the statins and corticosteroids previously described herein. Additionally, Der1 proteins known in the art from other species may be used, as well as Der1 like proteins known to the art.

Therapeutic agents that may be combined with IFNτ treatment of inflammatory bowel disease include infliximab (a monoclonal antibody against tumor necrosis-α factor, TNF-alpha factor) statins previously described herein, glatiramer acetate, anti-diarrheal agents, such as acetyltannic acid, alkofanone, bismuth subsalicylate, catechin, difenoxin, diphenoxylate, lidamidine, loperamide, racecadotril, trillium, uzarin and zaldaride; and antispasmodic agents, including belladonna, hyoscyamine, clidinium bromide, glycopyrrolate, dicyclomine hydrochloride, mebeverine, otilonium bromide, and cimetropium.

Therapeutic agents that may be combined with IFNτ treatment of arthritis include antigens, such as streptococcal cell wall; heat shock proteins, including those previously described herein such as heat shock protein 60; collagen, including type II collagen; non-steroidal anti-inflammatory agents previously described herein; monoclonal antibody against TNF-alpha factor, statins previously described herein, glatiramer acetate, methotrexate and COX-2 specific inhibitors previously described herein. Example 16 describes a model of arthritis and illustrates how treatment of humans suffering from arthritis treated with IFNτ alone or in combination with one or more second therapeutic agents can be evaluated to determine efficacy of treatment.

Therapeutic agents that may be combined with IFNτ treatment of multiple sclerosis include myelin basic protein and peptides thereof, myelin oligodendrocyte glycoprotein and peptides thereof previously described herein, a monoclonal antibody against TNF-alpha factor previously described herein, glatiramer acetate and the previously described corticosteroids and statins. The myelin basic protein is preferably a mammalian myelin basic protein, such as a human myelin basic protein. The nucleotide and amino acid sequence (SEQ ID NO:6) of human myelin basic protein may be found, for example, in the NIH Genbank database as accession number NM_002385. Suitable peptides of myelin basic protein include those from about 5 to about 30 amino acids long. Suitable peptides include the fragment from amino acid 1 to amino acid 11, which may include a modification at its 5' end, such as an acetyl group. It is realized that myelin basic protein and peptides thereof from other mammalian species, including, for example, ovine, bovine and porcine, that function in treating multiple sclerosis may be used in the methods herein. Therefore, applicable myelin basic proteins include those that have amino acid sequences that have at least about 70% identity, further at least about 80% identity, and further at least about 90% identity to the amino acid sequence of human myelin basic protein.

Therapeutic agents that may be combined with IFNτ treatment of uveitis include statins, glatiramer acetate, and other immunosuppressants previously described herein, including corticosteroids, and which may by administered systemically or in the form of drops for administration to the eye; uveitis inducing agents, including retinal S antigen (human retinal S antigen may be obtained as described in, for example, Beneski, D.A., et al., Inves. Opth. Vis. Sci. 25:686-690 (1984) and interstitial retinal binding protein (IRBP; the human sequence may be found in Liou, G.I., et al., J. Biol. Chem. 264 (14):8200-8206 (1989)); and human leukocyte antigen (HLA)-binding peptides, including those derived from HLA-A, HLA-B, HLA-C, HLA-E and HLA-G known to the art and discussed in, for example, PCT international application numbers PCT/US02/24311 and PCT/GB98/03686; Braud, V. M., et al., Nature 391:795-799 (1998).

Therapeutic agents that may be combined with IFNτ treatment of rejection from organ transplantation include immunosuppressants previously described herein, such as, for example, statins, glatiramer acetate, azathioprine, corticosteroids, or cyclophosphamide. Example 17 describes an organ transplant rejection model and illustrates how treatment of humans undergoing organ transplant and treated with IFNτ alone or in combination with one or more second therapeutic agents can be evaluated to determine efficacy of treatment.

It will be appreciated that in these combined treatment regimens, the second therapeutic agent can be administered prior to, concurrent with, or subsequent to orally administering IFN-τ. Selection of the timing of administration of IFN-τ and the second agent, as well as the suitable route of administration of the second agent, is readily done by those of skill in the art. The second agent can be administered by any suitable route, determined by the attending medical provider.

Another combination treatment regimen relates to administration of IFNτ in combination with an agent effective to protect and/or stabilize IFNτ after oral administration, and particularly during transit through the stomach and/or intestinal tract. The stabilizing agent serves to protect the IFNτ from loss of therapeutic activity through, i.e., denaturation in the stomach and/or intestines. Examples of stabilizers for use include buffers, antagonists to the secretion of stomach acids, amino acids such as glycine and lysine, carbohydrates such as dextrose, mannose, galactose, fructose, lactose, sucrose, maltose, sorbitol, mannitol, etc., proteolytic enzyme inhibitors, and the like.

In a preferred embodiment, the stabilizing agent is an antacid, such as an organic acid carboxylate salt, e.g., a salt of citric acid such as sodium citrate or potassium citrate, or an inorganic salt. Exemplary inorganic salts include, but are not limited to, aluminum hydroxide (Al(OH)₃) or phosphate, magnesium hydroxide (Mg(OH)₂), calcium carbonate (CaCO₃), sodium bicarbonate, magnesium oxide, magnesium trisilicate, magnesium carbonate, aluminum hydroxide gel, and combinations of these. The commercially available antacid Mylanta^{™} contains aluminum hydroxide and magnesium hydroxide, and is suitable as an orally administrable antacid. The antacid is typically insoluble in water and is given as an oral suspension.

The treatment regimen where an antacid is given in combination with IFNτ preferably involves administering the antacid orally prior to or concurrent with oral administration of IFNτ. For example, a patient would take the antacid 5-30 minutes before the IFNτ. The antacid protects the IFNτ from denaturation and/or degradation in the stomach, and more preferably in the intestinal tract, thus permitting a reduction in the dose of IFNτ required to achieve a therapeutic effect. As discussed above, a dose of IFNτ on the order of greater than 5 x 10⁸ Units is needed to achieve a measurable increase in a patient's blood IL-10 level. The same increase in IL-10 level can be achieved with a lower dose of IFNτ when the IFNτ is administered concurrent with or after administration of an antacid.

The class of compounds known as proton-pump inhibitors can also be administered as a stabilizing agent to protect and/or stabilize IFNτ after oral administration. Proton pump inhibitors prevent release of acid in the stomach and intestines and are often used to treat ulcers, acid reflux, or excess stomach acid. Generally, proton-pump inhibitors are substituted benzimidazoles and include rabeprazole (Aciphex^{®}), lansoprazole (Prevacid^{®}), omeprazole (Prilosec^{®}), and pantoprazole (Protonix^{®}). The proton pump inhibitor is administered prior to or concurrent with IFNτ and is effective to protect the IFNτ from denaturation and/or degradation in the stomach, and more preferably in the intestinal tract, thus permitting a reduction in the dose of IFNτ required to achieve a therapeutic effect. As discussed above, a dose of IFNτ on the order of greater than 5 x 10⁸ Units is needed, in the absence of a proton-pump inhibitor or an antacid, to achieve a measurable increase in a patient's blood IL-10 level. The same increase in IL-10 level can be achieved with a lower dose of IFNτ when the IFNτ is administered concurrent with or after administration of a proton-pump inhibitor.

Also contemplated is treatment of a patient by administering IFNτ orally in combination with both an antacid and a second therapeutic agent, such as those agents discussed above. In this embodiment, the dose of IFNτ required to achieve the desired clinical endpoint will be less that the dose required in the absence of the antacid. The dose of the second therapeutic agent can be adjusted if needed, based on the presence of the antacid and/or any synergistic effects with IFNτ.

Kits for treating patients having a viral infection, an autoimmune condition, or a condition characterized by cellular proliferation comprised of a therapeutically effective dose of IFNτ and of a second therapeutic agent having activity to treat, or at least partially alleviate the symptoms of, the condition, either in the same or separate packaging, and instructions for its use are also contemplated.

In one embodiment, a kit is comprised of (i) one or more unit dosages of IFNτ, the unit dosages providing a daily total dose of greater than 5 x 10⁸ Units, (ii) one or more unit dosages of a second treatment agent for multiple sclerosis, and (iii) instructions for use. The second treatment agent is, in preferred embodiment, natalizumab, a statin, or mycophenolate mofetil.

In another embodiment, a kit is comprised of (i) a unit dosage of IFNτ, the unit dosage being one-third to one-half of the recommended daily dosage of greater than 5 x 10⁸ Units and said unit dosage being in a form suitable for oral administration, (ii) a unit dosage of a second treatment agent for multiple sclerosis, the unit dosage being one-third to one-half of the physician prescribed daily dosage for the second treatment agent, and (iii) instructions for use. The attending medical caregiver prescribes that the patient administer the contents of two kits each day, if the kit contains unit dosages that are one-half the daily dosage, or three kits each day, if the kit contains unit dosages that are one-third the daily dosage. The second treatment agent is, in preferred embodiment, natalizumab, a statin, or mycophenolate mofetil.

In another embodiment, a kit is comprised of (i) an anti-acid; (ii) IFNτ in a form suitable for oral administration, (iii) a second treatment agent, and (iv) instructions for use. The anti-acid can be any one of the antacids described above and the second treatment agent is, in preferred embodiments, natalizumab, a statin, or mycophenolate mofetil.

A specific exemplary kit includes a therapeutic dose of a statin, a less than therapeutic dose of IFNτ, and an antacid, for treating a patient in need of multiple sclerosis treatment, and instructions for use. In another embodiment, a kit includes a therapeutic dose of a statin and a therapeutic dose of IFNτ for treating a patient in need of multiple sclerosis treatment, and instructions for use. In another embodiment, a kit includes therapeutic doses of natalizumab and IFNτ for treating a patient in need of multiple sclerosis treatment, and instructions for use. In another embodiment, a kit includes a therapeutic dose of natalizumab, a less than therapeutic dose of IFNτ, and an antacid, for treating a patient in need of multiple sclerosis treatment, and instructions for use, where the patient is instruction to take the antacid at least 5-30 minutes prior to oral administration of the IFNτ. Those of skill in the art can appreciate that these kits are merely exemplary of the various combinations and dosage regimens contemplated.

### E. Formulations and Dosages

Oral preparations containing IFNτ can be formulated according to known methods for preparing pharmaceutical compositions. In general, the IFNτ therapeutic compositions are formulated such that an effective amount of the IFNτ is combined with a suitable additive, carrier and/or excipient in order to facilitate effective oral administration of the composition. For example, tablets and capsules containing IFNτ may be prepared by combining IFNτ (*e.g.*, lyophilized IFNτ protein) with additives such as pharmaceutically acceptable carriers (*e.g.*, lactose, corn starch, microcrystalline cellulose, sucrose), binders (*e.g.*, alpha-form starch, methylcellulose, carboxymethylcellulose, hydroxypropylcellulose, hydroxypropylmethylcellulose, polyvinylpyrrolidone), disintegrating agents (*e.g.*, carboxymethylcellulose calcium, starch, low substituted hydroxy-propylcellulose), surfactants (*e.g.*, Tween 80, polyoxyethylene-polyoxypropylene copolymer), antioxidants (*e.g.*, L-cysteine, sodium sulfite, sodium ascorbate), lubricants (*e.g.*, magnesium stearate, talc), or the like.

Further, IFNτ polypeptides of the present invention can be mixed with a solid, pulverulent or other carrier, for example lactose, saccharose, sorbitol, mannitol, starch, such as potato starch, corn starch, millopectine, cellulose derivative or gelatine, and may also include lubricants, such as magnesium or calcium stearate, or polyethylene glycol waxes compressed to the formation of tablets. By using several layers of the carrier or diluent, tablets operating with slow release can be prepared.

Liquid preparations for oral administration can be made in the form of elixirs, syrups or suspensions, for example solutions containing from about 0.1 % to about 30% by weight of IFNτ, sugar and a mixture of ethanol, water, glycerol, propylene, glycol and possibly other additives of a conventional nature.

Another suitable formulation is a protective dosage form that protects the protein for survival in the stomach and intestines until absorbed by the intestinal mucosa. Protective dosage forms for proteins are known in the art, and include enteric coatings and/or mucoadhesive polymer coatings. Exemplary mucoadhesive polymer formulations include ethyl cellulose, hydroxypropylmethylcellulose, Eudragit^{®}, carboxyvinly polymer, carbomer, and the like. A dosage form designed for administration to the stomach via ingestion for delivery of IFNτ in an active form to the intestinal tract, and particularly to the small intestine, is contemplated. Alternatively, IFNτ can be co-administered with protease inhibitors, stabilized with polymeric materials, or encapsulated in a lipid or polymer particle to offer some protection from the stomach and/or intestinal environment.

An orally-active IFNτ pharmaceutical composition is administered in a therapeutically-effective amount to an individual in need of treatment. The dose may vary considerably and is dependent on factors such as the seriousness of the disorder, the age and the weight of the patient, other medications that the patient may be taking and the like. This amount or dosage is typically determined by the attending physician or other skilled person. The dosage will typically be between 1 x 10⁹ and 5 x 10¹² Units/day, more preferably between 1 x 10⁹ and 1 x 10¹² Units/day. In one specific embodiment, IFN-τ is administered orally at a dosage of greater than 1 x 10⁹ Units/day.

Disorders requiring a steady elevated level of IFNτ in plasma will benefit from administration as often as about every two to four hours, while other disorders, such as multiple sclerosis, may be effectively treated by administering a therapeutically-effective dose at less frequent intervals, *e.g.*, once a day or once every 48 hours. The rate of administration of individual doses is typically adjusted by an attending physician to enable administration of the lowest total dosage while alleviating the severity of the disease being treated. As discussed above, the method contemplates administering IFNτ orally at a first dose to a patient in need of treatment, and monitoring a biological marker to determine the individual patient response to the first dosage level. Monitoring can be readily done via a blood draw and analysis of a marker, such as IL-10 in the blood, using, for example, a ELISA or a radioimmunoassay kit Accordingly, in another aspect, a kit for using in treating a person suffering from a condition responsive to IFNτ therapy or to IL-10 therapy is contemplated. The kit includes a first part, comprised of a container containing one or more dosage form units designed for oral administration of IFNτ and a second part comprised of components required to monitor a biomarker of IFNτ, such as the components needed to analyze blood IL-10 levels.

Administration of IFNτ generally continues until a clinical endpoint is achieved. That clinical endpoint will vary according to the condition being treated, to the severity of the condition, and to the patient's individual characteristics (age, weight, health). Clinical endpoints are readily determined by an attending doctor or nurse and range from a temporary or permanent cessation of symptoms to resolution of the condition.

For example, in patients suffering from an autoimmune condition, such as psoriasis, treatment with IFNτ may continue until the psoriasis has cleared or a desired reduction in an assessment score has been achieved.

In multiple sclerosis patients, a suitable clinical endpoint would be a lessening of the severity of the symptoms.

In persons afflicted with an viral infection, a suitable clinical endpoint would be a reduction in viral titer or an attenuation of the symptoms associated with the viral infection (fever, rash, malaise, etc.).

In patients suffering from a condition characterized by cellular proliferation, a clinical endpoint at which to cease administration of IFNτ could be a regression in rate of cellular proliferation, as measured by regression of tumor size, or a slowing of cellular proliferation, as measured by a diminished rate of tumor growth.

For example, in patients suffering from Alzheimer's disease, a decrease in endogenous amyloid plaques and/or neurofibrillary tangles in the brain of such patients may be observed. Decreases in memory loss, language deterioration, confusion, restlessness and mood swings; and improved ability to mentally manipulate visual information as determined by standard methods may also be observed.

In individuals afflicted with pulmonary fibrosis, suitable clinical endpoints include an improvement in the ability to breath, a decrease in the progression of the disease as evidenced by a reduction in amount of fibrous tissue in the lungs, and/or a decrease in lung inflammation. The effect of IFNτ in ameliorating the decline in lung function, including the ability of the patient to breath after treatment compared to prior to treatment, may be measured by methods known to the skilled artisan, including measurement of the forced expiratory volume in one second (FEVI) as described, for example, in Pellegrino, R. et al., Eur. Respir. J., 10:543-549 (1997). A reduction in the increase in fibrous scar tissue in the lungs may be measured by methods known to the skilled artisan, including measurement of lung tissue hydroxyproline content, or other similar procedure known to the art, as described in Example 9. Decreases in lung inflammation may be observed by methods known to the art, including those discussed in Example 9.

In individuals with liver fibrosis, a suitable clinical endpoint includes a reduction in the increase in fibrous scar tissue in the liver as measured by methods known to the skilled artisan, including histological examination of liver tissue samples with scoring methods found in, for example, Desmet, V.J. et al., Hepatology., 19:1513-1520 (1994) or Chevallier, M. et al., Hepatology, 20:349-355 (1994). A decrease in serum hyaluronic acid levels and liver hydroxyproline content obtained by methods known to the art and described in Example 8 herein may also be observed. Additionally, a decrease in the serum level of various liver enzymes, including aspartate aminotransaminase (AST) and alanine aminotransaminase (ALT), measured by standard methods known to the art, may also be monitored.

In individuals who have experienced a stroke, a suitable clinical endpoint includes an increase in blood flow in the affected blood vessel as determined by computer tomographic methods as known in the art and as described, for example, in Nabavi, D.G. et al., Radiology, 213:141-149 (1999). A further clinical endpoint includes a decrease in numbness in the face, arm or leg; or a decrease in the intensity of a headache associated with the stroke. Yet another clinical endpoint includes a decrease in the cell, tissue or organ damage or death due to the stroke. Such decrease in cell or tissue damage may be assessed by brain imaging techniques, including computer assisted tomography (CAT) scanning, magnetic resonance imaging methods, or similar methods known to the art.

In individuals who have experienced optic neuritis, a suitable clinical endpoint includes an improvement in vision, a stabilization of vision (i.e., no further decline in vision) or a decrease in the rate of decline of vision. Such clinical endpoints may be determined by the skilled artisan as known in the art.

In individuals with chronic obstructive pulmonary disease, a suitable clinical endpoint includes an improvement in lung function or an otherwise decrease in the extent of the blockage. These clinical endpoints may be determined by, for example, a pulmonary function test. In one type of pulmonary function test, the patient breathes into a spirometer, which is a mechanical device that records changes in lung size as air is inhaled and exhaled by the patient as a function of time.

In individuals with autism, the clinical endpoint will depend on the particular symptoms associated with the patient. The symptoms include abnormalities in social skills, speech, communication and repeated behaviors and routines. For example, autistic individuals may exhibit no speech, non-speech vocalizations, echolalia, confusion between the pronouns "I" and "You", lack of eye contact, lack of response to people, and walking on tiptoes. Such abnormalities may be assessed by methods known to the skilled artisan.

In individuals with various autoimmune disorders, including diabetes, allergies, inflammatory bowel disease, psoriasis, arthritis, multiple sclerosis, uveitis, and anti-phospholipid syndrome, the clinical endpoint will depend on the particular disease or condition. For example, in individuals afflicted with anti-phospholipid syndrome, a suitable clinical endpoint includes reduction in the levels of antibodies directed against membrane anionic phospholipids, (e.g., anti-cardiolipin, anti-phosphatidylserine) or their associated plasma proteins (e.g., β-2-glycoprotein). Quantitation of such antibodies and or plasma proteins may be accomplished by routine methods known to the art, including by enzyme-linked inmmunosorbent assays (ELISA) as described, for example, in Pierangeli, S.S., et al., Thromb. Haemost. 74:1361-1367 (1995).

Yet another clinical endpoint in individuals with anti-phospholipid syndrome includes a decrease in vascular thrombosis, which may occur in a wide variety of tissue and/or organs. A decrease in vascular thrombosis may be observed by determining the blood flow in the affected tissue or organ by computer tomographic methods as described, for example, in Nabavi, D.G., et al., Radiology 213:141-149 (1999). A decrease in vascular thrombosis may also be histologically detected. Skin or other involved tissue may be analyzed. For example, biopsies from affected kidneys may show a reduction in glomerular and/or small arterial microthrombi. Additionally, the size of thrombi, as well as the rate of its disappearance, may be observed with fiber-optic devices used to transilluminate the vein and a trilocular stereoscopic operating microscope equipped with a closed-circuit video system, monitor and recorder as more fully described in Pierangeli, S.S., et al., Circulation 94:1746-1751 (1996) and Example 10 described herein.

A suitable clinical endpoint in diabetes includes the ability to control blood glucose levels either without insulin or with a decreased amount of insulin.

A suitable clinical endpoint for various allergies include a decrease in various symptoms of allergies, including a decrease in the amount of wheezing, sneezing, watery eyes, nausea, vomiting or diarrhea associated with the allergic condition and may be determined by common methods in the art.

A suitable clinical endpoint for inflammatory bowel disease, including Crohn's disease and ulcerative colitis, includes decreases in various symptoms of this disease, such as a decrease in the extent of diarrhea, a decrease in abdominal pain and/or cramping, decreased amount of blood in the stool, increased appetite, and decreased body temperature from fever associated with the disease.

A suitable clinical endpoint with respect to psoriasis includes a decrease in the amount of the characteristic dry, red patches of skin covered with silvery scales or a decrease in the swelling or stiffness of affected joints.

A suitable clinical endpoint in arthritis, such as found in rheumatoid arthritis, includes a decrease in the amount of pain and swelling in the affected joints, an increase in the range of motion of the patient, and an increase in the strength of the muscle attached to the affected joint.

A suitable clinical endpoint for multiple sclerosis includes a decrease in attacks and a decrease in number of brain lesions.

A suitable clinical endpoint for uveitis, including anterior uveitis, intermediate uveitis, posterior uveitis and diffuse uveitis, includes improvement in vision, including no further decline in vision, and a decrease in the amount of floaters. Decreased pain, redness and photophobia may be also be observed with anterior uveitis.

A suitable clinical endpoint for allergies includes a decrease in allergic reactions, such as swelling in mucosal tissues, a decrease in inflammation in affected tissues and a decrease in overall IgE levels.

Suitable clinical endpoints in organ transplantation include, for example, an increase in graft survival.

Suitable clinical endpoints in atherosclerosis will depend on the particular symptoms the patient exhibits. For example, the patient may exhibit decreased blood flow in one or more blood vessels. Accordingly, a suitable clinical endpoint includes increases in blood flow in the selected blood vessels. The rate of blood flow may be measured by methods known to the skilled artisan, including Laser Doppler Flowmetry (LDF), Magnetic Resonance Imaging (MRI), Positron Emission Tomography (PET), and Computed Tomography (CT) Imaging, including Single Photon Emission Computed Tomography (SPECT) (Leenders, K.L., et al. Brain 113:27-47 (1990); Sakai, F., et al. J. Cereb. Blood Flow Metab. 5:207-213 (1988); Rempp, K.A., et al, Radiology 193:637-641 (1994); Baird, A. E. and Warach, S., J. Cereb. Blood Flow Metab. 18:583-609 (1998); Danus, G., et al., Radiology 213:141-149 (1999); Calamante, F., et al., J. Cereb. Blood Flow Metab. 19:701-735 (1999); Ginsberg, M.D., et al., J. Cereb Blood Flow Metab. 2(1):89-98 (1982); Fukuda, O., Neurosurgery 36(2):358-364 (1995); Perez-Pinzon, et al., J. Neurolog. Sci. 153 (1):25-31 (1997); Borlongan, et al., Brain Res. 1010(1-2):108-116 (2004)). If the patient presents with angina pectoris derived from the atherosclerosis, a decrease in pain associated with this condition may be observed. If the patient presents with peripheral vascular disease derived from the atherosclerosis, decreased claudication and decreased impotence may be observed. Other suitable clinical endpoints are known to the skilled artisan.

Once the desired clinical endpoint is achieved, daily treatment with IFNτ can cease, however a maintenance dose can be administered if desired or as necessary. Subsequently, the dosage or the frequency of administration, or both, may be reduced, as a function of the symptoms, to a level at which the clinical endpoint is maintained or the improved condition is retained.

### IV. Examples

The following examples further illustrate the invention described herein and are in no way intended to limit the scope of the invention.

### Methods

### A. Production of IFNτ

In one embodiment, a synthetic IFNτ gene was generated using standard molecular methods (Ausubel, et al., *supra*, 1988) by ligating oligonucleotides containing contiguous portions of a DNA sequence encoding the IFNτ amino acid sequence. The DNA sequence used may be either SEQ ID NO:1 or SEQ ID NO:4 or the sequence as shown in Imakawa, K. et al, Nature, 330:377-379, (1987). The resulting IFNτ polynucleotide coding sequence may span position 16 through 531: a coding sequence of 172 amino acids.

In one embodiment, the full length synthetic gene Stul/SStl fragment (540 bp) may be cloned into a modified pIN III omp-A expression vector and transformed into a competent SB221 strain of E. coli. For expression of the IFNτ protein, cells carrying the expression vector were grown in L-broth containing ampicillin to an OD (550 nm) of 0.1 - 1, induced with IPTG (isopropyl-1-thio-b-D-galactoside) for 3 hours and harvested by centrifugation. Soluble recombinant IFN-τ may be liberated from the cells by sonication or osmotic fractionation.

For expression in yeast, the IFNτ gene may amplified using polymerase chain reaction (PCR; Mullis, K.B., U.S. Patent No. 4,683,202; Mullis, K.B. et al., U. S. Patent No. 4,683,195) with PCR primers containing Stul and Sacl restriction sites at the 5' and 3' ends, respectively. The amplified fragments were digested with Stul and SacII and ligated into the SacII and SmaI sites of pBLUESCRIPT+(KS), generating pBSY-IFNτ. Plasmid pBSY-IFNτ was digested with SacII and EcoRV and the fragment containing the synthetic IFNτ gene was isolated. The yeast expression vector pBS24Ub (Ecker, D.J., et al., J. Biol. Chem. 264:7715-7719 (1989)) was digested with SaII. Blunt ends were generated using T4 DNA polymerase. The vector DNA was extracted with phenol and ethanol precipitated (Sambrook, J., et al., in MOLECULAR CLONING: A LABORATORY MANUAL, Second Edition, Cold Spring Harbor Laboratory, Cold Spring Harbor, NY (1989)). The recovered plasmid was digested with SacII, purified by agarose gel electrophoresis, and ligated to the SacII-EcoRV fragment isolated from pBSY-IFNτ. The resulting recombinant plasmid was designated pBS24Ub-IFNτ.

The recombinant plasmid pBS24Ub-IFNτ was transformed into E. coli. Recombinant clones containing the IFNτ insert were isolated and identified by restriction enzyme analysis. IFNτ coding sequences were isolated from pBS24Ub-IFNτ and cloned into a *Pichia pastoris* vector containing the alcohol oxidase (AOX1) promoter (Invitrogen, San Diego, CA). The vector was then used to transform Pichia pastoris GS115 His⁻ host cells and protein was expressed following the manufacturer's instructions. The protein was secreted into the medium and purified by successive DEAE-cellulose and hydroxyapatite chromatography to electrophoretic homogeneity as determined by SDS-PAGE and silver staining.

### B. Antiviral Assay to Determine Specific Antiviral Activity

Antiviral activity was assessed using a standard cytopathic effect assay (Familletti, P.C., et al., Methods in Enzymology, 78:387-394 (1981); Rubinstein, S. et al., J. Virol., 37:755-758 (1981)). Briefly, dilutions of IFNτ were incubated with Madin-Darby bovine kidney (MDBK) cells for 16-18 hours at 37 °C. Following incubation, inhibition of viral replication was determined in a cytopathic effect assay using vesicular stomatitis virus as challenge. One antiviral unit (U) caused a 50% reduction in destruction of the monolayer. For the studies described herein, the IFNτ had a specific activity of about 1 x 10⁸ antiviral U/mg protein, unless otherwise specified.

### EXAMPLE 1

### Administration of IFNτ to Multiple Sclerosis Patients

Humans suffering from multiple sclerosis were enrolled in a trial for treatment with IFNτ. Fifteen patients were randomized into three treatment groups: Group I patients were given IFNτ orally at a dosage of 0.2 mg per day (2 x 10⁷ U/day) Group II patients were given IFNτ orally at a dosage of 0.8 mg per day (8 x 10⁷ U/day); and Group III patients were given IFNτ orally at a dosage of 1.8 mg per day (1.8 x 10⁸ U/day).

Prior to treatment with IFNτ, on screening Day and Day 1 (one), a blood sample was taken from each subject to determine a baseline serum cytokine concentration. Treatment was initiated by administering IFNτ orally to each patient following the blood draw on Day 1. Prior to administration, the vials of IFNτ (SEQ ID NO:3) and syringes were kept in a refrigerator maintained at 2 to 8 °C. Prior to self-administration of medication, the patient removed one vial and one syringe from the refrigerator. The cap was removed from the tip of the syringe and the tip of the syringe was placed into the bottle of medication to withdraw the appropriate volume into the syringe as instructed at the clinic on Day 1. The tip of the syringe was placed in the mouth and the syringe contents were emptied into the mouth by depressing the plunger. The patient then swallowed, and if desired, was allowed to drink a glass of water. The patient noted on his/her diary card the date and time the dose was administered.

Blood samples were taken from each patient on Days 1, 4, 8, 15, 29, and 57 of the study. The samples were analyzed for IL-10 concentrations and IFN-γ concentrations by using commercially available ELISA kits (Genzyme, Cambridge, Mass). The results are shown in Figs. 1A-1D (IL-10) and Figs. 2A-2D (IFN-γ) as well as Figs. 3A-3E (IL-10 and IFN-γ).

### A. Statistical Analysis of Results

Fifteen patients with Relapsing-Remitting Multiple Sclerosis were treated with oral IFN-tau at one of three doses (0.2 mg, 0.6 mg and 1.8mg) once per day for four weeks. Serum samples were obtained at screening and Days 1, 4, 8, 15, 29 and 57 and assessed for IL-10 and IFN-gamma levels (pg/ml). The results for the three groups were assessed over time using the Repeated Measures Analysis of Variance statistic. Of the 90 data points (Day 1 - Day 57), the values for nine missing data points were imputed by carrying the previous values forward.

**IL-10:** The analysis found no significant difference between the three dose groups (F=2.92, P=0.0927), no significant effect of time (F=0.70, P=0.6285), and no significant group-by-time interaction (F=0.74, P=0.6803). This suggests IL-10 levels were unchanged following the administration of IFNτ in all three groups across the 28-day dosing period and 28-day follow-up period. The average change from Day 1 to Day 29 of dosing for the lowest to highest dose groups was 7%, 3% and -25%, respectively. The average change to Day 57 for the three dose groups was 10%, -10% and -39%, respectfully. In all cases, the data in all three groups was highly variable.

**IFN-**γ: The analysis found no significant difference between the three dose groups (F=1.06, P>0.3769), no significant effect of time (F=1.86, P=0.1140), and no significant group-by-time interaction (F=1.45, P=0.1820). This suggests IFN-γ levels were unchanged following the administration of IFNτ in all three groups across the 24-day dosing period and 28-day follow-up period. The average change from Day 1 to Day 29 of dosing for the lowest to highest dose groups was - 63%, -14% and 35%, respectively. The average change to Day 57 for the three dose groups was -27%, -46% and 22%, respectfully. Similar to the IL-10 analysis, the data in all three groups was highly variable.

### EXAMPLE 2

### Administration of IFNτ Three Times Daily to Human Patients Infected with Hepatitis C

### A. IFNτ Preparation

On day one, one bottle of IFNτ (SEQ ID NO:3) was removed from the refrigerator and the patient self-administered the proper volume of test material according to Table 2. IFNτ (SEQ ID NO:2) may also be prepared and administered in the same manner.

**Table 2**

| **Recombinant Ov-IFNτ Patient Dose Administration** | | | | | |
|---|---|---|---|---|---|
| Dose Group | Number of Patients | IFNτ (mg/mL) | Volume (mL) per Dose (TID) | Total Daily Dose (mg) | Total Daily Dose (U) |
| I | 6 | 1.0 | 0.33 | 1.0 | 1 × 10⁸ |
| II | 6 | 1.0 | 1.0 | 3.0 | 3 × 10⁸ |
| III | 6 | 1.0 | 3.0 | 9.0 | 9 × 10⁸ |

### B. Patient Dosing Instructions

All vials of test material and syringes were kept in a refrigerator maintained at 2 to 8 °C. Prior to the self-administration of medication, the patient removed one vial and one syringe from the refrigerator. The cap was removed from the tip of the syringe and the tip of the syringe was placed into the bottle of medication to withdraw the appropriate volume into the syringe as instructed at the clinic on Day 1.

The tip of the syringe was placed in the mouth and the syringe contents were emptied into the mouth by depressing the plunger. The patient then swallowed the test material. If desired, the patient was allowed to drink a glass of water. The patient noted on his/her diary card the date and time the dose of test material was administered.

The above steps were repeated three times per day at approximately eight-hour intervals: once in the morning, once at midday, and once in the evening.

### C. Results

Blood samples were taken at defined intervals over a 169 day test period. The samples were analyzed for IL-10 levels and IFN-γ levels in the serum using ELISA kits (Genzyme, Cambridge, Mass) following the manufacturer's instructions. The viral titer of hepatitis C, using reverse-transcriptase polymerase chain reaction, blood levels of 2', 5'-oligoadenylate synthetase (OAS), and the serum concentration of alanine aminotransferase (ALT) were also determined and are not reported here.

The results for each subject are shown in Figs. 4A-4D (IL-10 levels) and Figs. 5A-5D (IFN-γ levels), and in Figs. 6A-6F (IL-10 and IFN-γ).

### D. Statistical Analysis of Results

The results for the three groups were assessed over time using the Repeated Measures Analysis of Variance statistic. The data for one patient in Group II was not used because of missing baseline serum samples. Of the 204 data points (Day 1 - Day 169), the values for seven missing data points for both measures were imputed by carrying the previous values forward.

IL-10: The analysis found a statistical significant difference between the three groups (F=12.08, P=0.0009), a significant effect of time (F=11.20, P=0.0001) and a significant group-by-time interaction (F=7.88, P=0.001). The latter finding is clearly seen by the difference in IL-10 response rates between the three dose groups over time. While the lowest dose group (Group I; 0.33 mg TID) produced a 22% increase in IL-10 levels from Day 1 to Day 43, Group II (1 mg TID) produced a peak response of 114% by Day 29. In contrast, Group III (3 mg TID) produced a 387% increase by Day 43 with a peak of 484% by Day 71.

The significant interaction term is also supported by the differential decline between dose groups in IL-10 levels once dosing was terminated at Day 84: Group I declined from its 11 % gain at Day 85 to 4% at Day 169, and Group II declined from 95% to 0.5% over the same time period. Therefore the two lowest dose groups returned to baseline six months following the termination of dosing. The highest dose group (Group III; 3 mg TID), however, declined from 453% to 194% by Day 169, thus still showing a substantial increase over baseline six months after dosing was stopped.

IFN-γ: The analysis found no significant difference between the three dose groups (F=1.13, P>0.3499), no significant effect of time (F=1.55, P=0.1187), and no significant group-by-time interaction (F=1.39, P=0.1275). This indicates IFN-γ levels were not significantly changed following the administration of IFNτ in all three groups across the 84-day dosing period and 84-day follow-up period. The average change from Day 1 to Day 85 of dosing for the lowest to highest dose groups was -6%, 8% and 7%, respectively. Interestingly, the average change to Day 169 for the three dose groups was 4%, 21 % and 31 %, respectfully, suggesting a dose response following the termination of dosing.

### EXAMPLE 3

### Administration of IFNτ Twice Daily to Patients Infected with Hepatatis C

Five patients infected with hepatitis C were recruited for a study. The patients were treated with IFNτ according to the method of Example 2, each patient received 7.5 mg twice daily, for a total daily dose of 15 mg (1.5 x 10⁹ U). The first dose was taken in the morning, before breakfast. The second dose was taken at least three hours after an evening meal.

Blood samples were taken at defined intervals over the 113 day test period. The samples were analyzed for IL-10, IL-12, and IFN-γ levels in the serum using commercially available ELISA kits (Genzyme, Cambridge, Mass). The results are shown in Fig. 7A (IL-10), Fig. 7B (IFN-γ), and in Figs. 8A-8D (IL-10, IL-12, and IFN-γ) for four of the patients.

### EXAMPLE 4

### Administration of IFNτ in for Treatment of Psoriasis

Forty-five patients with a clinical diagnosis of chronic plaque psoriasis (vulgaris psoriasis) covering at least 10% of the body surface, excluding the scalp, are randomized into three treatment groups. Group I is treated with 1 mg of IFNτ orally three times per day, for a daily dose of 3 mg IFNτ. Group II is treated with 3 mg of IFNτ orally three times per day, for a daily dose of 9 mg IFNτ. Group III is treated with a placebo. The antiviral activity of the IFNτ is determined to be about 5 x 10⁸ antiviral Units/mg protein; thus the 3 mg dose corresponds to 1.5 x 10⁹ Units/day and the 9 mg dose corresponds to 4.5 x 10⁹ Units/day. The IFNτ is provided as a liquid in a pharmaceutically-acceptable vehicle, which is immediately swallowed and ingested by each patient. Dosing continues for 84 days.

Beginning 14 days before treatment and on the first day of treatment, each patient is evaluated daily using the Physician's Static Global Assessment (PSGA). A PSGA score of 0, 1, 2, 3, 4, or 5 is assigned to each patient, based on the following criteria:
0 = clear, except for residual discoloration;
1 = majority of lesions have individual scores for induration, erythema, and scaling that averages 1;
2 = majority of lesions have individual scores for induration, erythema, and scaling that averages 2;
3 = majority of lesions have individual scores for induration, erythema, and scaling that averages 3;
4 = majority of lesions have individual scores for induration, erythema, and scaling that averages 4;
5 = majority of lesions have individual scores for induration, erythema, and scaling that averages 5.

An induration score is assigned a value of 0, 1, 2, 3, 4, or 5 according to the following criteria:
0 = no evidence of plaque elevation;
1 = minimal plaque elevation, - 0.5 mm;
2 = mild plaque elevation, - 1 mm;
3 = moderate plaque elevation, - 1.5 mm;
4 = marked plaque elevation, - 2 mm;
5 = severe plaque elevation, - 2.5 mm or more;

A scaling score is assigned a value of 0, 1, 2, 3, 4, or 5 according to the following criteria:
0 = no evidence of scaling;
1 = minimal; occasional fine scale over less than 5% of the lesion;
2 = mild; fine scale predominates;
3 = moderate; coarse scale predominates;
4 = marked; thick, non-tenacious scale predominates;
5 = severe; very thick tehacious scale predominates;

An erythema score is assigned a value of 0, 1, 2, 3, 4, or 5 according to the following criteria:
0 = no evidence of erythema, hyperpigmentation may be present;
1 = faint erythema;
2 = light red coloration;
3 = moderate red coloration;
4 = bright red coloration;
5 = dusky to deep red coloration;

Each patient is also given a Psoriasis Area and Severity Index (PASI) evaluation prior to treatment (Fredrikkson, T. et al., Dermatologica, 157:238 (1978)). A PASI score is calculated from an evaluation of the extent and severity of lesions on the head, trunk, upper extremities, and lower extremities of the patient, as described in Fredrikkson *et al.* (Id.).

During the 84 day dosing period, each patient is evaluated on days 1, 8, 15, 29, 43, 57, 71, and 85 and given a PASI score and a PSGA score. Post-study, each patient is again evaluated on days 113 and 169.

Patients achieve a PSGA of 0, 1, or 2 and/or a 75% improvement in PASI score.

### EXAMPLE 5

### Administration of IFNτ in Combination with a Second Agent

A patient suffering from multiple sclerosis is treated with IFN-τ, administered orally twice daily, for a total daily dose of 5.5 x 10⁸ U. Once every 28 days, the patient is treated with natalizumab at a dose of 3 mg/kg, given via intravenous infusion. The patient is treated for 6 months according to this regimen and then assessed for brain lesions using unenhanced proton-density T2-weighted MRI and gadolinium-enhanced T1-weighted MRI scans. During treatment, blood samples are taken for analysis of cytokine (IL-10, IL-12, and IFN-γ) levels.

### EXAMPLE 6

### Administration of IFNτ in Combination with a Second Agent

A heart transplant patient is treated with IFN-τ, administered orally twice daily, for a total daily dose of 5.5 x 10⁸ U, and with mycophenolate mofetil, administered orally at a dose of 500 mg/day. During treatment, periodic blood samples are taken for analysis of cytokine (IL-10, IL-12, and IFN-γ) levels. Treatment continues for the life of the patient.

### EXAMPLE 7

### Effect of IFNτ Treatment on Alzheimer's Disease

This example shows how IFNτ may be advantageous for treating Alzheimer's disease. A transgenic mouse model of Alzheimer's disease will be used. One group of the mice will receive IFNτ treatment and the control group will receive none. It is expected that treatment of the transgenic mice with IFNτ will exhibit a decreased plaque burden, a decrease in brain levels of amyloid beta peptide, and decreased microglial and astrocyte activation.

Ninety day old heterozygous male PDAPP transgenic mice with the APPV717F mutation (i.e., substitution of Phe for Val in the transmembrane domain of the amyloid precursor protein (APP) as discussed in Murrell, J. et al., Science 254(5028):97-99 (1991)) may be purchased from Taconic Farms (Germantown, NY) and kept under standard laboratory conditions in accordance with the NIH Guide for the Care and Use of Laboratory Animals. The mice may be maintained on a 12 hour light and 12 hour dark cycle. The mice may be allowed free access to animal chow and tap water *ad libitum*.

Immunohistochemical and histological staining of mouse brains to determine plaque burden can be accomplished as described in, for example, Weiner, H.L., et al., Ann. Neurol. 48:567-579 (2000). Briefly, formalin-fixed brain tissue can be washed in Tris-buffered saline, dehydrated, and embedded in paraffin. Sagittal sections of brain tissue (e.g., ten micrometer sections) can be air dried and baked at 58°C for one hour. Sections can be deparaffinized in Histoclear (National Diagnostics, Atlanta, GA) and rehydrated through graded ethanol to water.

Mice may be treated with various dosages of IFNτ (e.g., about 1 x 10⁵ U/day to about 5 x 10¹² U/day) by oral (intragastric) administration.

### EXAMPLE 8

### Effect of IFNτ Treatment on Liver Fibrosis

This example shows how IFNτ treatment may be advantageous for treating liver fibrosis. Liver fibrosis will be induced in experimental animals by carbon tetrachloride treatment. One group of the carbon-tetrachloride treated animals will receive IFNτ treatment and the control group will receive only vehicle. It is expected that the carbon-tetrachloride-treated rats will exhibit a decrease in the progression of the disease when treated with IFNτ.

Sprague-Dawley rats weighing between 200 and 250 grams can be obtained from Taconic Farms (Germantown, NY) and maintained as described in Example 7.

Liver fibrosis may be induced by intraperitoneal injections of carbon tetrachloride as described in Zhang, L.J., et al., World J. Gastroenterol. 10(1):77-81 (2004). Briefly, rats receive intraperitoneal injections of 50% carbon tetrachloride in saline at a dose of 2 ml/kg twice a week.

Liver tissues may be fixed in formal and embedded in paraffin (Zhang, L.J., et al., World J. Gastroenterol. 10(1):77-81 (2004)). Sections may be stained with hemotoxylin and eosin (HE) and examined under a light microscope. Stages of fibrosis may be assessed according to the criteria set forth in either Desmet, V.J.,. et al., Hepatology 19:1513-1520 (1994) or Chevallier, M., et al., Hepatology 20:349-355 (1994).

In order to determine the hydroxyproline content in liver, the liver may be first homogenized into power and hydrolyxed with 6M hydrochloric acid. (Weng, H. L., et al., World J. Gastroenterol. 7(1):42-48 (2001). The hydroxylproline content can be measured as described in Kivirikko, K.L., et al., Anal. Biochem 19:249-255 (1967).

Rats may be treated with IFNτ prior to, during or after administration of carbon tetrachloride. The rats can be treated with various dosages of IFNτ (e.g., about 1 x 10⁵ U/day to about 5 x 10¹² U/day) by oral (intragastric) administration. The control group will receive only vehicle.

### EXAMPLE 9

### Effect of IFNτ Treatment on Pulmonary Fibrosis

This example shows how IFNτ treatment may be advantageous for treating pulmonary fibrosis. Pulmonary fibrosis will be induced in experimental animals by bleomycin treatment. One group of the bleomycin-treated group will receive IFNτ treatment and the control group will receive only vehicle. Indicators of inflammation, such as myeloperoxidase activity of a bronchoalveolar lavage and serum TNF-alpha will be monitored. Tissue hydroxyproline content will be measured to monitor the extent of fibrosis. It is expected that treatment of bleomycin-treated mice treated with IFNτ will decrease the extent of fibrosis as determined by measuring the hydroxyproline content. It is also expected that IFNτ will decrease the markers of inflammation (myeloperoxidase activity and amount of TNF-alpha as determined by TNF-alpha mRNA) in treated versus control mice.

Eight-week old male C57BU6 mice may be purchased from Harlan (Indianapolis, IN) and maintained as described in Example 7.

In order to induce pulmonary fibrosis, mice can receive a single dose of intratracheal bleomycin (0.8 mg/kg) (Arai, T. et al., Am J. Physiol. Lung Cell. Mol. Physiol. 278:L914-L922 (2000)). Control mice are treated with vehicle. The mice may be sacrificed 7 days later (to examine inflammatory indicators) or 21 days later (to examine fibrotic indicators) by inhalation of sevoflurane. The lungs may then be excised, and blood may be obtained from the right ventricle. A bronchoalveolar lavage may be performed by instilling 1 ml of isotonic saline and withdrawn from the lungs with an intratracheal cannula.

The myeloperoxidase activity of the bronchoalveolar lavage may be determined as known in the art (Arai, T. et al., Am J. Physiol. Lung Cell. Mol. Physiol. 278:L914-L922 (2000)). Briefly, the bronchoalveolar lavage can be centrifuged at 400g for 5 minutes. The cell pellet can be resuspended in 0.1 M K₂HPO₄ buffer and sonicated for 90 seconds. The supernatant can be mixed, after centrifugation at 12,000 g for 10 minutes, with 0.3 ml of Hanks' BSS containing 0.25% bovine serum albumin, 0.25 ml of 0.1 M K₂HPO₄ (pH 7.0), 0.05 ml of 1.25 mg/ml o-dianisidine (Sigma, St. Louis, MO), and 0.05 ml of 0.05% H₂O₂ and incubated for 10 minutes at 25°C. The reaction can be terminated by adding 0.5 ml of 1 % NaN₃, and absorbance can be measured at 460 nm.

Bleomycin treated mice may be treated with IFNτ at the same time that bleomycin is introduced, or prior to or after administration. The mice may be treated with various dosages of IFNτ (e.g., about 1 x 10⁵ U/day to about 5 x 10¹² U/day) by oral (intragastric) administration. Bleomycin-treated mice in the control group will receive only vehicle.

### EXAMPLE 10

### Effect of IFNτ Treatment on Anti-Phospholipid Syndrome (APS)

This example shows how IFNτ may be advantageous for treating APS. APS will be induced in experimental animals by injection of human immunoglobulin G (IgG) anti-cardiolipin antibodies. One group of the antibody-treated group will receive IFNτ treatment and the control group will received only vehicle. One femoral vein will be subjected to an injury which induces a thrombus and the size of the thrombus that forms, as well as the time of formation and disappearance of the thrombus, in control and IFNτ treated animals will be used as an indicator of the effectiveness of treatment. It is expected that treatment of antibody-treated mice with IFNτ will decrease the size of the initial thrombus that forms, increase the time the thrombus takes to form and decreases the time it takes the thrombus to disappear.

Normal male CD-1^{®} Nude Mice (Crl:CD-1^{®}-*nu*BR;outbred), developed from the transfer of the nude gene to a CD-1 mouse through a series of crosses and backcrosses, which is T-cell deficient, may be purchased from Charles River Laboratories (Wilmington, MA) and maintained as described in Example 7.

IgG may be isolated from the serum of a patient with APS by protein G sepharose chromatography. (Pierangeli, S.S., et al., Circulation 94:1746-1751 (1996).) The purity of the IgG may be determined by observing a single band of 150 kD by sodium dodecyl sulfate-polyacrylamide gel electrophoresis (SDS-PAGE). Protein concentration may be determined by the Lowry method. After the protein concentration is adjusted with sterile saline solution, the solutions may be filter sterilized before injecting the animals.

Mice may be immunized on days 1, 7, 14 and 21 by subcutaneous injection with 150 ug of IgG-APS in adjuvant (Adju-Prime, Pierce Chemical Co.) (Pierangeli, S.S., et al., Circulation 94:1746-1751 (1996).). A specimen of blood can be drawn from each animal at weekly intervals to test for the presence of mouse anti-cardiolipin antibodies. Mouse anti-cardiolipin antibodies (IgG and IgM) may be determined by an enzyme-linked immunosorbent assay (ELISA) as known in the art and as described, for example, in Pierangeli, S.S., et al., Thromb. Haemost. 74:1361-1367 (1995). Alkaline phosphatase anti-mouse IgG and anti-mouse IgM may be used as secondary antibodies in the ELISA protocol. The color reaction may be stopped when a positive control (e.g., of about 100 G phospholipids units) reaches 1.0 OD units (typically in about 20 or 30 minutes).

After the mice have been found to produce relatively high levels of anti-cardiolipin antibodies (e.g., about 0.8 OD units; typically occurs after about two weeks after immunization), thrombi are induced by a pinch injury as more fully described in Pierangeli, S.S., et al., Circulation 94:1746-1751 (1996). Briefly, animals may be treated with anesthetic (sodium pentobarbital, 60 nmg/kg intraperitoneally) prior to making a longitudinal incision in the right groin of the mice, extending to the kneed. The right femoral vein is dissected free and a standardized thrombogenic injury may be produced in the vein by bringing together forceps until the flat circular opposing surfaces (may be about 0.1 mm in diameter) meet to produce the pinch injury.

A fiber-optic device may be used to transilluminate the vein and a trilocular stereoscopic operating microscope (ERNST, Leitz GMBH, Wetzlar) equipped with a closed-circuit video system (NEC-NC-A/CCD Camera, NEC, USA, Inc.), a Panasonic 12" color monitor and U-Matic V-5800 recorder (Sony Corp.) can be used to visualize and measure the size of the thrombus and its rate of appearance and disappearance Pierangeli, S.S., et al., Circulation 94:1746-1751 (1996).

Mice may be treated with IFNτ after injection with the antibodies from APS patients. The mice can be treated with various dosages of IFNτ (e.g., about 1 x 10⁵ U/day to about 5 x 10¹² U/day) by oral (intragastric) administration. The control group will receive only vehicle.

### EXAMPLE 11

### Effect of IFNτ Treatment on Stroke

This example shows how IFNτ may be advantageous for treating stroke. A stroke will be induced in experimental animals by occlusion of the middle cerebral artery with an intraluminal suture. The stroke-induced group will receive IFNτ treatment and the control group will receive only vehicle. The protective effects of IFNτ treatment can be assessed by determining brain infarct size, measurement of apoptotic cell nuclei in the brain and assessment of behavior using a scale known in the art. It is expected that mice subjected to stroke and treated with IFNτ will exhibit decreased brain infract size, decreased cerebral apoptotic nucleic and improved neurological function when compared to control mice.

Male Sprague Dawley neonatal rats (9 to 11 days of age) may be obtained from Taconic Farms (Germantown, NY). The mice may be maintained as described in Example 7.

Cerebral ischemia can be induced in the Sprague Dawley rats using an occluding intraluminal suture as described in Maier, C.M., et al., J. Neurosurg 94:90-96 (2001) and Bright, R., et al., J. Neurosci. 24(31):6880-6888 (2004). Briefly, after anesthetizing the animal with isoflurane, the tip of an uncoated 30 mm long segment of 3-0 nylon suture is rounded by flame. The suture is inserted into the stump of the external carotid artery and advanced into the internal carotid artery about 19 to about 20 mm from the bifuracation to occlude the ostium of the middle cerebral artery. After 2 hours of ischemia, the suture can be removed and the animal can be allowed to recover.

Behavior can be assessed at 24 hours after reperfusion using a scale of 1-4 described in Bright, R. et al., J. Neurosci., 24(31):6880-6888 (2004): grade 1, normal posture, spontaneous movement in any direction; grade 2, unilateral paw extension when lifted by the tail; grade 3, both paw extension and circuling pattern when spontaneously walking; grade 4, abnormal posture, paw extension, circuling pattern, can not walk spontaneously.

Apoptotic cell nucleic may be measured by terminal deoxynucleotidyl transferase-mediated biotinylated UTP nicke end labeling (TUNEL) as described in Bright, R., et al., J. Neurosci. 24(31):6880-6888 (2004). Briefly, animals subjected to a 2 hour middle cerebral artery occlusion can be sacrificed after 72 hours of reperfusion and transcardially perfused with normal saline followed by 4% paraformladehyde. Brains can be isolated and fixed overnight in 4% paraformaldehyde, immersed in 30% sucrose for about 2 to about 3 days, and snap frozen in OCT cyroprotectant (Tissue-Tek, Miles, Eklkart, IN).

Coronal slices (16 µm) can be taken from equivalent midbrain regions in each brain, with each slice separated by at least 48 µm. Slides can be stained and images can be taken from four defined regions in the ipsilateral cortex of each slice.

Rats may be treated with IFNτ prior to, during or after induction of ischemia. The rats can be treated with various dosages of IFNτ (e.g., about 1 x 10⁵ U/day to about 5 x 10¹² U/day) by oral (intragastric) administration. The control group will receive only vehicle.

### EXAMPLE 12

### Effect of IFNτ treatment on Optic Neuritis

This example shows how IFNτ may be advantageous for treating optic neuritis. Experimental allergic encephalitis (EAE), an animal model of optic neuritis, will be induced in test animals. The EAE-induced group will receive IFNτ treatment and the control group will receive only vehicle. The protective effects of IFNτ treatment can be assessed by determining the extent of axonal demyelination. It is expected that EAE-induced mice treated with IFNτ will exhibit decreased axonal demyelination when compared to control mice.

SJL/J mice may be purchased from the Jackson Laboratory (Bar Harbor, Maine) and maintained as described in Example 7.

EAE can be induced in test mice by sensitization with homologous spinal cord emulsion in complete Freund;s adjuvant that can be injected subdermally into the nuchal area as described, for example, in Guy, J., et al., Proc. Natl. Acad. Sci. U.S.A. 95:13847-13852 (1998).

The extent of demyleination may be quantitated by threshold measurements of the myelin sheaths derived from axonal transmission electron micrographs for each optic nerve as described in Guy, J., et al., Proc. Natl. Acad. Sci. U.S.A. 95:13847-13852 (1998).

Briefly, mice may be sacrificed and perfused by cardiac puncture with fixative consisting of 4% paraformaldehyde in 0.1 M PBS buffer (pH 7.4) The eyes with attached optic nerves can be dissected out and tissue specimens will be postfixed in 5% acrolein, 0.1 M sodium cacodylate-HCL buffer (pH 7.4) and 7% sucrose and dehydrated through an ethanol series and embedded in LR White that can be polymerized at 50°C overnight. Ultrathin sections (e.g., about 90 mm) can be placed on nickel grids for immunocytochemistry. Nonspecific binding of antibodies can be blocked by floating the grids on 2% teleost gelatin and 2% nonfat dry milk in 0.01 M TBS (pH 7.2) with Tween 20 for 30 minutes. The grids can then be reacted with rabbit anti-albumin antibodies, washed and then reacted with secondary goat anti-rabbit igG antibodies conjugated to 10 nm gold for about 1 hour at room temperature. Grids can then be rinsed in deionized water. Immunolabeled specimens can be photographed by transmission electron microscopy without poststaining.

Mice may be treated with IFNτ prior to injection with the spinal chord emulsion, after injection with spinal chord emulsion or after optic neuritis is induced. The mice can be treated with various dosages of IFNτ (e.g., about 1 x 10⁵ U/day to about 5 x 10¹² U/day) by oral (intragastric) administration. The control group will receive only vehicle.

### EXAMPLE 13

### Effect of IFNτ treatment on Chronic Obstructive Pulmonary Disease

This example shows how IFNτ may be advantageous for treating chronic obstructive pulmonary disease. Patients diagnosed with a chronic obstructive pulmonary disease, including chronic bronchitis and emphysema, will be treated with IFNτ. The protective effects of IFNτ treatment can be assessed by determining improvements in lung function. It is expected that patients treated with IFNτ will exhibit improved lung function.

Human subjects who have been diagnosed with a chronic obstructive pulmonary disease can be treated with more than 0.5 x 10⁹ antiviral units of IFNτ daily. Improvements in lung function can be assessed with a spirometer as known in the art.

### EXAMPLE 14

### Effect of IFNτ treatment on Autism

This example shows how IFNτ may be advantageous for treating autism. Patients diagnosed with autism will be treated with IFNτ. The advantageous effects of IFNτ treatment can be assessed by determining alterations in the patient's pre-existing behavior. It is expected that patients treated with IFNτ will exhibit positive changes in behavior.

Human subjects who have been diagnosed with autism can be treated with more than 0.5 x 10⁹ antiviral units of IFNτ. Alterations in behavior, including improvements in social skills, speech, communication and/or repeated behaviors and routines may be assessed by methods known to the skilled artisan.

### EXAMPLE 15

### Effect of IFNτ treatment on atherosclerosis

This example shows how IFNτ may be advantageous for treating atherosclerosis. Atherosclerosis will be induced in low density receptor (LDL)-deficient mice by feeding them a high cholesterol diet. One group of mice will receive IFNτ and the control group will receive only vehicle. The protective effects of IFNτ treatment can be assessed by determining the extent of atherosclerotic plaques that develop in the mice. It is expected that mice treated with IFNτ will exhibit decreased atherosclerotic plaques when compared to control mice.

LDL receptor deficient mice may be purchased from Jackson Laboratories and maintained as described in Example 7.

Atherosclerosis may be induced in the mice by feeding them a diet high in fat and cholesterol as described in Lichtman, A.H., et al., Arterioscler. Thromb. Vasc. Biol. 19:1938-1934 (1999).

The surface area of the aorta occupied by atherosclerotic lesions may be quantified by en face oil red O staining as more fully described in Lichtman, A.H., et al., Arterioscler. Thromb. Vasc. Biol. 19:1938-1934 (1999). Briefly, mice may be sacrificed after 12 weeks on the particular diet by ether inhalation. After perfusion of the left ventricle and arterial tree with PBS, the entire aorta attached to the heart can be dissected and placed in formaldehyde overnight. The aorta can then be stained with oil red O, the adventitial fat is removed and the aorta can then be opened longitudinally, pinned en face on a black silicone-covered dish and photographed while immersed in PBS. Slides can then be scanned into a computer and the percent surface area occupied by oil red O-stained lesions can be determined by using image analysis software (NIH Image).

Mice may be treated with IFNτ when they are on cholesterol-rich diets. The mice may be treated with various dosages of IFNτ (e.g., about 1 x 10⁵ U/day to about 5 x 10¹² U/day) by oral (intragastric) administration. Mice in the control group will receive only vehicle.

### EXAMPLE 16

### Effect of IFNτ treatment on an autoimmune disorder

This example shows how IFNτ may be advantageous for treating and/or preventing collagen-induced arthritis (CIA) in mice. CIA will be induced in Balb C mice by injection of type II collagen. Prior to induction, mice will receive IFNτ treatment. The advantageous effects of IFNτ treatment can be assessed by determining the extent of development of CIA in the mice. It is expected that mice treated with IFNτ will exhibit decrease disease incidence.

Inhibition of development of CIA in Balb C mice may be performed by injecting Balb C mice with a single dose of IFNτ on the day of immunization with chicken type II collagen for induction of CIA. Collagen can be emulsified in complete Freund's adjuvant with H37Ra and may be injected on either side of the base of the tail. On the day of immunization and 48 hours later, Pertussis toxin may also be injected.

Recombinant ovine IFNτ may be expressed in Pichia pastoris using a synthetic gene construct. The protein can be secreted into the medium and purified by successive DEAE-cellulose and hydroxyapatite chromatography to electrophoretic homogeneity as determined by SDS-PAGE and silver staining.

Mice can be examined daily for signs of CIA. The severity of the disease may be graded as an arthritic index on the following scale: 1, redness of one toe; 2, redness and swelling of one toe; 3, deformation of one toe; 4 for each additional toe, the index number is added to the index.

Mice may be treated with IFNτ after the transplantation procedure. The mice may be treated with various dosages of IFNτ (e.g., about 1 x 10⁵ U/day to about 5 x 10¹² U/day) by oral (intragastric) administration. Mice in the control group will receive only vehicle.

### EXAMPLE 17

### Effect of IFNτ treatment on organ transplant rejection

This example shows how IFNτ may be advantageous for preventing or otherwise ameliorating organ transplant rejection. Kidney transplantation in a rat model will be performed. One group of rats will be treated with IFNτ and the control group will receive only vehicle. The effect of IFNτ in allograft rejection, as measured by renal allograft survival and the presence of selected immune system cells, will be examined. Treatment with IFNτ is expected to increase renal allograft survival and decrease the presence of selected immune systems cells in the graft.

Inbred Fisher 344 (F344) and Lewis rats may be purchased from Charles River Italia (Calco, Italy). Lewis rats can be the recipients and the Fisher rats can be the donors. The animals may be maintained as described in Example 7.

Kidney transplantation may be accomplished as described in Noris, M. et al., J. Am. Soc. Nephrol., 12:1937-1946 (2001). Briefly, the left donor kidney may be removed and positioned orthotopically into the recipient whose renal vessels have been isolated and clamped and whose left native kidney has been removed. End-to-end anastomosis of the renal artery, vein and ureter can be performed using 10-O Prolen sutures. The right native kidney can be removed on the 11^{th} postoperative day. Complete allograft failure can be defined as death of the animal because the animals are dependent on the transplanted kidney function.

Immunohistochemical analysis for selected immune system cells may be performed as described in Noris, M. et al., J. Am. Soc. Nephrol., 12:1937-1946 (2001). Briefly, mouse monoclonal antibodies can be used for the detection of the following antigens: 1) ED1 antigen; 2) a rat MHC class II antigen monomorphic determinant; 3) CD4 cell surface glycoprotein; 4) rat CD8 cell surface glycoprotein; 5) rat dendritic cells-restricted antigen. All antigens can be analyzed by an indirect immunofluorescence technique.

Rats may be treated with IFNτ after the transplantation procedure. The rats may be treated with various dosages of IFNτ (e.g., about 1 x 10⁵ U/day to about 5 x 10¹² U/day) by oral (intragastric) administration. Rats in the control group will receive only vehicle.

Although the invention has been described with respect to particular embodiments, it will be apparent to those skilled in the art that various changes and modifications can be made without departing from the invention.

## Claims

1. A composition for use in the preparation of a medicament for treating an autoimmune condition responsive to an increase in blood interleukin-10 (IL-10) level, said composition being comprised of an orally-administrable formulation of interferon-tau (IFN-τ) at a dosage of greater than 1 x 10⁹ Units.

2. The composition of claim 1, wherein said IFN-τ Is selected from ovine IFN-τ and bovine IFN-τ.

3. The composition of claim 1 or claim 2, wherein ovine IFN-τ has a sequence identified as SEQ ID NO:2 or SEQ ID NO:3.

4. The composition of any one of claims 1 - 3, wherein said orally-administrable formulation achieves administration to the intestinal tract.

5. The composition of claim 1, wherein the autoimmune condition is multiple sclerosis.

6. The composition according to any preceding claim, wherein the composition is provided in combination with a second therapeutic agent.

7. The composition of claim 6, wherein said second therapeutic agent is selected from the group consisting of anti-viral agents, anti-cancer agents, anti-inflammatory agents, and agents suitable for treatment of autoimmune disorders.

8. The composition of claim 6, wherein said second therapeutic agent is administered prior to, concurrent with, or subsequent to said orally administering IFN-τ.

9. The composition of claim 6, wherein said administering a second therapeutic agent comprises administering a second therapeutic agent selected from the group consisting of natalizumab, statins, mycophenolate mofetil, and copaxone.

10. The composition of claim 6, for treating or preventing organ transplant rejection, wherein the second therapeutic agent is mycophenolate mofetil.

11. The composition of claim 6, for treating an inflammatory condition in a subject, wherein the second therapeutic agent is a statin.

12. The composition of claim 6, for treating psoriasis in a subject, wherein the second therapeutic agent is selected from collagen, a retinoid, anthralin, calpotriene, coal tar, salicylic acid, and an immunosuppressant.

13. The composition according to any one of claims 1 - 4, for use in slowing progression of multiple sclerosis in a subject or for reducing the risk of relapse in a subject suffering from multiple sclerosis.

14. The composition according to any one of claims 1 - 4, for use in treating a subject with psoriasis.

15. The composition according to any one of claims 1 - 4, for use in preventing an increase in the blood level of IFN-γ in a subject at risk of an elevated IFN-γ due to (i) administration of a therapeutic agent or (ii) a disease condition.

16. The composition of claim 15, wherein said subject has an elevated IFN-γ level due to treatment with IFN-α or IFN-β, and said administering continues during the period of the subject's symptoms.

17. The composition of claim 15, wherein said subject is suffering from multiple sclerosis and is being treated with IFN-β.

18. The composition of claim 15, wherein said subject is suffering from a viral infection and is being treated with IFN-α.

19. The composition according to any one of claims 1 - 4, for use in decreasing the blood level of interleukin-12 in a subject.

## Patentansprüche

1. Zusammensetzung zur Verwendung bei der Herstellung eines Medikaments zur Behandlung eines Autoimmunleidens, das auf einen Anstieg des Interleukin-10 (IL-10) -Spiegels im Blut reagiert, wobei die Zusammensetzung eine oral verabreichbare Formulierung von Interferon-tau (IFN-τ) in einer Dosierung umfasst, die höher als 1 x 10⁹ Einheiten ist.

2. Zusammensetzung nach Anspruch 1, wobei das IFN-τ ausgewählt ist aus IFN-τ vom Schaf und IFN-τ vom Rind.

3. Zusammensetzung nach Anspruch 1 oder 2, wobei das IFN-τ vom Schaf eine als SEQ ID NO:2 oder SEQ ID NO:3 identifizierte Sequenz aufweist.

4. Zusammensetzung nach einem der Ansprüche 1 - 3, wobei die oral verabreichbare Formulierung eine Verabreichung in den Magen-Darm-Trakt bewirkt.

5. Zusammensetzung nach Anspruch 1, wobei das Autoimmunleiden Multiple Sklerose ist.

6. Zusammensetzung nach einem der vorstehenden Ansprüche, wobei die Zusammensetzung in Kombination mit einem zweiten Therapeutikum bereitgestellt wird.

7. Zusammensetzung nach Anspruch 6, wobei das zweite Therapeutikum ausgewählt ist aus der Gruppe bestehend aus anti-viralen Therapeutika, Anti-Krebs-Therapeutika, entzündungshemmenden Therapeutika und Therapeutika, die für die Behandlung von Autoimmunerkrankungen geeignet sind.

8. Zusammensetzung nach Anspruch 6, wobei das zweite Therapeutikum vor, gleichzeitig mit oder im Anschluss an die orale Verabreichung von IFN-τ verabreicht wird.

9. Zusammensetzung nach Anspruch 6, wobei die Verabreichung eines zweiten therapeutischen Wirkstoffes die Verabreichung eines zweiten Therapeutikums umfasst, das ausgewählt ist aus der Gruppe bestehend aus Natalizumab, Statinen, Mycophenolat-Mofetil und Copaxon.

10. Zusammensetzung nach Anspruch 6 zur Behandlung oder Prävention der Abstoßung eines Organtransplantats, wobei das zweite Therapeutikum Mycophenolat-Mofetil ist.

11. Zusammensetzung nach Anspruch 6 zur Behandlung eines entzündlichen Zustandes bei einem Individuum, wobei das zweite Therapeutikum ein Statin ist.

12. Zusammensetzung nach Anspruch 6 zur Behandlung von Psoriasis bei einem Individuum, wobei das zweite Therapeutikum ausgewählt ist aus Kollagen, einem Retinoid, Anthralin, Calpotriene, Steinkohlenteer, Salicylsäure und einem Immunsuppressivum.

13. Zusammensetzung nach einem der Ansprüche 1 - 4 zur Verwendung, um das Fortschreiten der Multiplen Skerose bei einem Individuum zu verlangsamen oder das Rückfallrisiko bei einem Individuum, das an Multipler Sklerose leidet, zu verringern.

14. Zusammensetzung nach einem der Ansprüche 1 - 4 zur Verwendung bei der Behandlung eines Individuums mit Psoriasis.

15. Zusammensetzung nach einem der Ansprüche 1 - 4 zur Verwendung, um einen Anstieg des IFN-γ-Spiegels im Blut bei einem Individuum zu verhindern, das auf Grund (i) der Verabreichung eines Therapeutikums oder (ii) einer Erkrankung ein Risiko für erhöhtes IFN-γ hat.

16. Zusammensetzung nach Anspruch 15, wobei das Individuum auf Grund einer Behandlung mit IFN-α oder IFN-β einen erhöhten IFN-γ-Spiegel hat, und die Verabreichung während der symptomatischen Phase des Individuums fortgesetzt wird.

17. Zusammensetzung nach Anspruch 15, wobei das Individuum an Multipler Sklerose leidet und mit IFN-β behandelt wird.

18. Zusammensetzung nach Anspruch 15, wobei das Individuum an einer Virusinfektion leidet und mit IFN-α behandelt wird.

19. Zusammensetzung nach einem der Ansprüche 1 - 4 zur Verwendung, um den Interleukin-12-Spiegel im Blut eines Individuums zu senken.

## Revendications

1. Composition pour utilisation dans la préparation d'un médicament pour le traitement d'une maladie auto-immune en réponse à une augmentation du taux sanguin d'interleukine-10 (IL-10), ladite composition comprenant une formulation d'interférontau (IFN-τ) administrable oralement à un dosage supérieur à 1 × 10⁹ unités.

2. Composition selon la revendication 1, dans laquelle ledit IFN-τ est sélectionné parmi l'IFN-τ ovin et l'IFN-τ bovin.

3. Composition selon la revendication 1 ou 2, dans laquelle l'IFN-τ ovin a une séquence identifiée comme SEQ ID NO:2 ou SEQ ID NO:3.

4. Composition selon l'une des revendications 1 - 3, dans laquelle ladite formulation administrable oralement réalise l'administration dans le tractus intestinal.

5. Composition selon la revendication 1, dans laquelle la maladie auto-immune est la sclérose en plaques.

6. Composition selon l'une quelconque des revendications précédentes, dans laquelle la composition est donnée en combinaison avec un second agent thérapeutique.

7. Composition selon la revendication 6, dans laquelle ledit second agent thérapeutique est sélectionné dans le groupe constitué d'agents anti-viraux, d'agents anti-cancéreux, d'agents anti-inflammatoires, et d'agents adaptés au traitement des maladies auto-immunes.

8. Composition selon la revendication 6, dans laquelle le second agent thérapeutique est administré avant, en simultanée avec, ou après ladite administration orale d'IFN-τ.

9. Composition selon la revendication 6, dans laquelle ladite administration d'un second agent thérapeutique comprend l'administration d'un second agent thérapeutique sélectionné dans le groupe constitué du natalizumab, des statines, du mycophénolate mofétile, et de la copaxone.

10. Composition selon la revendication 6, pour traiter ou prévenir le rejet de transplants d'organes, dans laquelle le second agent thérapeutique est le mycophénolate mofétile.

11. Composition selon la revendication 6, pour traiter une maladie inflammatoire chez un sujet, dans laquelle le second agent thérapeutique est une statine.

12. Composition selon la revendication 6, pour traiter le psoriasis chez un sujet, dans laquelle le second agent thérapeutique est sélectionné parmi le collagène, un rétinoide, l'anthraline, le calpotriène, le goudron de houille, l'acide salicylique, et un immunosuppressant.

13. Composition selon l'une des revendications 1 - 4, pour une utilisation destinée au ralentissement de la progression de la sclérose en plaques chez un sujet ou pour réduire le risque de rechute chez un sujet atteint de sclérose en plaques.

14. Composition selon l'une des revendications 1 - 4, pour une utilisation dans le traitement d'un sujet atteint de psoriasis.

15. Composition selon l'une des revendications 1 - 4, pour une utilisation dans la prévention d'une augmentation du taux sanguin d'IFN-γ chez un sujet à risque d'IFN-γ élevé du à (i) l'administration d'un agent thérapeutique ou (ii) à une condition pathologique.

16. Composition selon la revendication 15, dans laquelle ledit sujet a un taux élevé d'IFN-γ du à un traitement avec l'IFN-α ou l'IFN-β, et ladite administration continue pendant la durée des symptômes chez le sujet.

17. Composition selon la revendication 15, dans laquelle ledit sujet souffre de sclérose en plaques et est traité avec l'IFN-β.

18. Composition selon la revendication 15, dans laquelle ledit sujet souffre d'une infection virale et est traité avec l'IFN-α.

19. Composition selon l'une des revendications 1 - 4, pour une utilisation pour diminuer le taux sanguin d'interleukine-12 chez un sujet.
